# EUROPEAN PATENT SPECIFICATION

(11) **EP 3 697 934 B1**
(45) Date of publication and mention of the grant of the patent: **27.03.2024**
(21) Application number: 18868173.8
(22) Date of filing: 15.10.2018
(51) Int. Cl.: C12Q 1/6897, C12N 15/12, C12N 15/85, C12N 5/10, C12Q 1/02, G01N 33/566

(54) **SYSTEMS AND METHODS FOR THE ASSESSMENT OF G-PROTEIN ACTIVATION**
SYSTEME UND VERFAHREN ZUR BEWERTUNG DER G-PROTEIN-AKTIVIERUNG
SYSTÈMES ET PROCÉDÉS POUR L'ÉVALUATION D'UNE ACTIVATION DES PROTÉINES G

(30) Priority: 18.10.2017 US 201762573853 P
(43) Date of publication of application: 26.08.2020
(73) Proprietor: Université de Montréal, Montreal, QC H3T 1J4 (CA); Domain Therapeutics, 67400 Illkirch Graffenstaden (FR)
(72) Inventor: LE GOUILL, Christian, Montréal, Québec J1E 3P4 (CA); BOUVIER, Michel, Montréal, Québec H3T 1B7 (CA); LUKASHEVA, Viktoriya, Saint-Laurent, Québec H4L 5A6 (CA); HOGUE, Mireille, Laval, Québec H7C 1E5 (CA); BRETON, Billy, Montréal, Québec H2G 2G7 (CA)
(74) Representative: Ernest Gutmann - Yves Plasseraud S.A.S.
(86) International application number: PCT/CA2018/051294
(87) International publication number: WO 2019/075556

(56) References cited:
- WO-A1-2005/121755
- WO-A1-2006/086883
- WO-A1-2016/041093
- WO-A1-2016/058094
- WO-A1-2016/058094
- MASUHO IKUO ET AL: "Distinct profiles of functional discrimination among G proteins determine the actions of G protein-coupled receptors", SCIENCE SIGNALING, vol. 8, no. 405, 1 December 2015 (2015-12-01), pages ra123-ra123, XP055804785, US ISSN: 1945-0877, DOI: 10.1126/scisignal.aab4068 Retrieved from the Internet: URL:https://stke.sciencemag.org/content/si gtrans/8/405/ra123.full.pdf>
- ONER SUKRU SADIK ET AL: "Receptor-regulated Interaction of Activator of G-protein Signaling-4 and G[alpha]i*", JOURNAL OF BIOLOGICAL CHEMISTRY, vol. 285, no. 27, 1 July 2010 (2010-07-01) , pages 20588-20594, XP055804892, US ISSN: 0021-9258, DOI: 10.1074/jbc.C109.088070
- BRETON BILLY ET AL: "Combining resonance energy transfer methods reveals a complex between the [alpha] 2A -adrenergic receptor, G[alpha] i1 [beta] 1 [gamma] 2 , and GRK2", THE FASEB JOURNAL, vol. 24, no. 12, 1 December 2010 (2010-12-01), pages 4733-4743, XP055804916, & EXPERIMENTAL BIOLOGY MEETING; SAN DIEGO, CA, USA; APRIL 21 -25, 2018 ISSN: 0892-6638, DOI: 10.1096/fj.10-164061
- "Deutsche Gesellschaft für Experimentelle und Klinische Pharmakologie und Toxikologie e.V", NAUNYN-SCHMIEDEBERG'S ARCHIVES OF PHARMACOLOGY, SPRINGER, DE, vol. 385, no. Suppl 1, 22 February 2012 (2012-02-22), pages 1-116, XP037076577, ISSN: 0028-1298, DOI: 10.1007/S00210-012-0736-0 [retrieved on 2012-02-22]
- CHANG, M. et al.: "Dissecting G Protein-coupled Receptor Signaling Pathways with Membrane-permeable Blocking Peptides", The Journal of Biological Chemistry, vol. 275, no. 10, 10 March 2000 (2000-03-10), pages 7021-7029, XP009084317, ISSN: 0021-9258, DOI: doi:10.1074/jbc.275.10.7021
- HAMM, H: "The Many Faces of G Protein Signaling", The Journal of Biological Chemistry, vol. 273, no. 2, 9 January 1998 (1998-01-09), pages 669-672, XP055008220, ISSN: 0021-9258, DOI: doi:10.1074/jbc.273.2.669
- SYROVATKINA, V. et al.: "Regulation, Signaling and Physiological Functions of G-protein s", Journal of Molecular Biology, vol. 428, no. 19, 25 September 2016 (2016-09-25), pages 3850-3868, XP029721377, ISSN: 0022-2836, DOI: doi:10.1016/j.jmb.2016.08.002

## Description

### TECHNICAL FIELD

The present invention generally relates to the monitoring of G-protein activation in a Gα protein subunit family-selective manner, in a system comprising a cell.

### BACKGROUND ART

Heterotrimeric G proteins are the canonical signaling partners of G protein-coupled receptors (GPCRs), and also known to be involved in the signaling of other types of receptors, notably receptor tyrosine kinases (RTKs) through the Go-interacting Vesicle-associated protein (GIV; also known as Girdin) (Ghosh P, 2016, Pharmacol Res. 105:99-107). Receptor activation triggers the exchange of a Gα-bound GDP for GTP, resulting in a conformational rearrangement of the heterotrimeric G protein that promotes dissociation of Gα and Gβ/γ subunits. GTP-bound Gα and free Gβ/γ subunits are then available to engage specific effectors.

G proteins are grouped into families based on the signaling outcomes following activation of the Gα subunit. The Gs family (Gαs, Gαolf) bolsters the production of cAMP through direct activation of adenylyl cyclases (AC). Conversely, the Gi family (Gαi1, Gαi2, Gαi3, GαoA, GαoB and Gαz) reduces cAMP levels by inhibiting specific ACs. The Gq family (Gαq, Gα11, Gα14 and Gα15) activates Phospholipases Cβ (PLCβs) to produce the second messengers diacylglycerol (DAG) and inositol triphosphate (IP₃), which subsequently promote the activation of PKCs and Ca²⁺ release from the endoplasmic reticulum, respectively. Finally, G12/13 family (Gα12 and Gα13) is known to control Rho-GEFs such as LARG, p115 and TRIO and thus influence processes linked to cytoskeletal remodeling (e.g., chemotaxis). In view of the distinct outcomes of signaling following activation of G proteins belonging to different families, systems and assays that permit to monitor G protein activation in a G protein family-selective manner are needed, for example to better characterize cell surface receptor activation by various ligands (*e.g.,* signaling pathways activated by a given ligand) and identity more specific modulators of G proteins.

Although cell surface receptor activation occurs primarily at the plasma membrane (PM) in response to ligand binding, signaling of cell surface receptors (*e.g.,* GPCRs, RTKs) has been shown to occur in other cellular compartments, including the endosomes and the Golgi. Upon activation, many cell surface receptors enter the endosomes, and trafficking of a ligand-receptor complex within the endosomes provides a mechanism to either terminate signaling through degradation of the receptor (in lysosomes and proteasomes), or to sustain signaling through recycling of the receptor back to the cell surface. However, it has been demonstrated that receptor signaling can also be initiated, sustained, and terminated in the endosomes (Murphy et al., 2009, PNAS, vol. 106 no. 42, 17615-17622; Tsvetanova et al., 2015, J. Biol. Chem. 290(11), pp. 6689-6696; Vilardaga et al., 2014, Nature Chemical Biology 10, 700-706). Similarly, some G proteins have been found associated with, and activated at, the Golgi (Lo et al., 2015, Dev. Cell. 33: 189-203) and the endoplasmic reticulum (ER)-Golgi interface (Bastin et al., Front Bioeng Biotechnol. 2015 Sep 1;3: 128). Different signals can arise from receptors at the PM and other compartments such as the endosomes and the Golgi, resulting in distinct physiological responses, and different mechanisms regulate signaling of receptors at these compartments. These distinct mechanisms of signaling and regulation raise the possibility of novel therapies based on targeting signaling at other cellular compartments (rather than PM), and thus systems and assays to monitor G protein activation at different cellular compartments are needed.

U.S. Patent No. 9,029,097 and WO/2016/058094 disclose BRET-based biosensors for monitoring G protein activation. However, these biosensors require the tagging of one or more of the G protein subunits (Gα, Gβ, and/or Gγ), and/or of the GPCR, which may influence the activity of these proteins. Also, these biosensors detect global G protein activation in the cells, but do not allow the monitoring of G protein activation at different cellular compartments and in a G protein family-selective manner.

### SUMMARY OF THE INVENTION

The invention is as defined in the claims and relates to a system for measuring modulation of G protein activation in a Gα protein subunit family-selective manner, said system comprising a cell expressing:
(i) a first component comprising a Gα subunit interacting polypeptide (GASIP) tagged with a bioluminescent donor molecule or a fluorescent acceptor molecule;
   wherein said GASIP comprises the G protein-binding domain of: Rap1GAP, a Regulator of G-protein signaling (RGS) protein, P63RhoGEF, PDZRhoGEF, or P115RhoGEF;
(ii) a second component comprising a plasma membrane (PM)-targeting moiety comprising (a) a palmitoylation, myristoylation, and/or prenylation signal sequence and/or (b) a polybasic sequence, an endosomal-targeting moiety or a Golgi-targeting moiety tagged with a bioluminescent donor molecule or a fluorescent acceptor molecule;
   wherein if said GASIP is tagged with said fluorescent acceptor molecule, said PM-targeting moiety, endosomal-targeting moiety or Golgi-targeting moiety is tagged with said bioluminescent donor molecule, and if said GASIP is tagged with said bioluminescent donor molecule, said PM-targeting moiety, endosomal-targeting moiety or Golgi-targeting moiety is tagged with said fluorescent acceptor molecule;
(iii) a third component that is a cell surface receptor that signals through said G protein, wherein said receptor is a GPCR, an RTK or an integrin receptor;
and wherein there is no direct protein-protein interaction between (i) the PM-targeting moiety, endosomal-targeting moiety or Golgi-targeting moiety and (ii) the GASIP, as define in claim 1.

The invention also relates to a host cell expressing the components of the system as defined in the claims, as set in claim 19.

As set in claim 20, the invention also relates to a method for determining whether an agent modulates the activation of a G protein of interest, said method comprising:
(a) contacting the system as claimed with a substrate for said bioluminescent donor molecule; and
(b) measuring the BRET signal in the system in the presence and absence of said agent; wherein a difference in said BRET signal in the presence of said agent relative to the absence thereof is indicative that said agent modulates the activation of said G protein of interest.

As set in claim 21, the invention also relates to a method for determining whether an agent modulates non-receptor guanine nucleotide exchange factor (GEF)-mediated G protein activation, said method comprising
(a) contacting the system as defined in the claims with a substrate for said bioluminescent donor molecule; and
(b) measuring the BRET signal in the system in the presence and absence of said agent; wherein a difference in said BRET signal in the presence of said agent relative to the absence thereof is indicative that said agent modulates non-receptor GEF-mediated G protein activation.

Other objects, advantages and features of the present invention will become more apparent upon reading of the following non-restrictive description of specific embodiments thereof, with reference to the accompanying drawings.

### BRIEF DESCRIPTION OF DRAWINGS

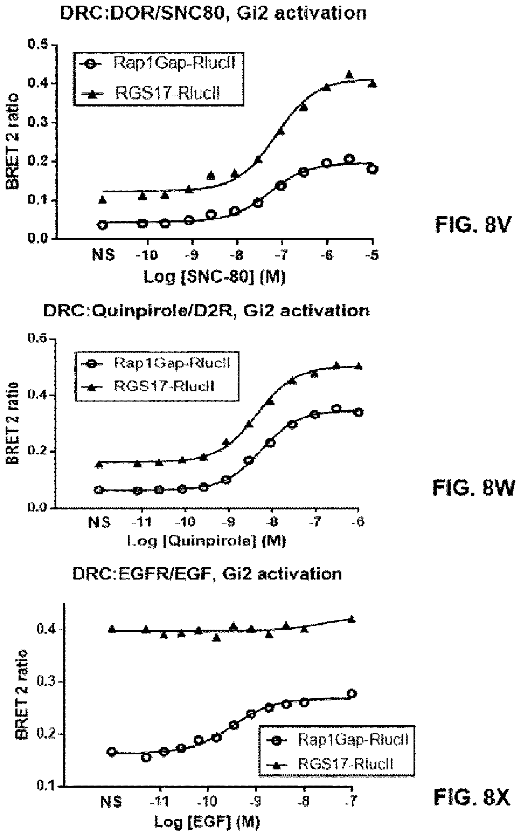

In the appended drawings:

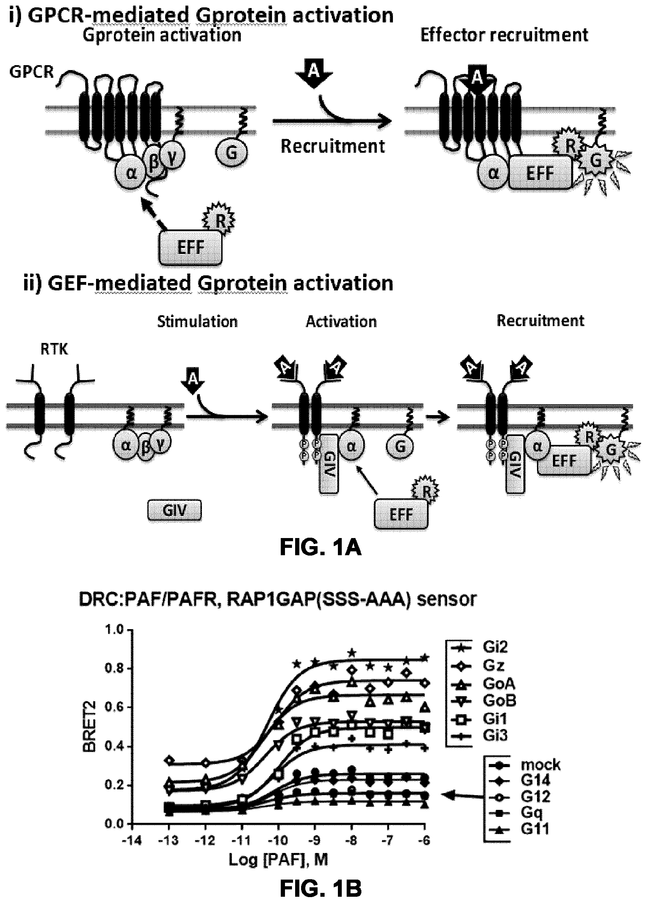

FIG. 1A depicts the principle of an effector-based sensor to monitor GPCR-mediated direct G protein activation (upper panel **i**) or guanine-nucleotide exchange factor (GEF)-mediated G protein activation (lower panel **ii**). Cells expressing a receptor, a cellular compartment (or subcellular localization) marker, such as a plasma-membrane (PM) or early endosomes (EE) marker, tagged with a suitable Bioluminescent Resonance Energy Transfer (BRET) donor or acceptor (*e*.*g*., rGFP), the Gα-interaction domain of a specific G protein effector tagged with suitable BRET donor or acceptor (*e.g.,* Rlucll) are exposed to an agonist to activate the coexpressed G protein. In panel **i)**, the agonist-induced GPCR stimulation activates directly G proteins, which recruits a tagged effector from the cytoplasm to the labeled membrane. In panel **ii)** G protein activation is mediated by the recruitment of a GEF such as GIV/Girdin following the activation of an RTK (*e*.*g*., EGFR) or an integrin α-β complex.

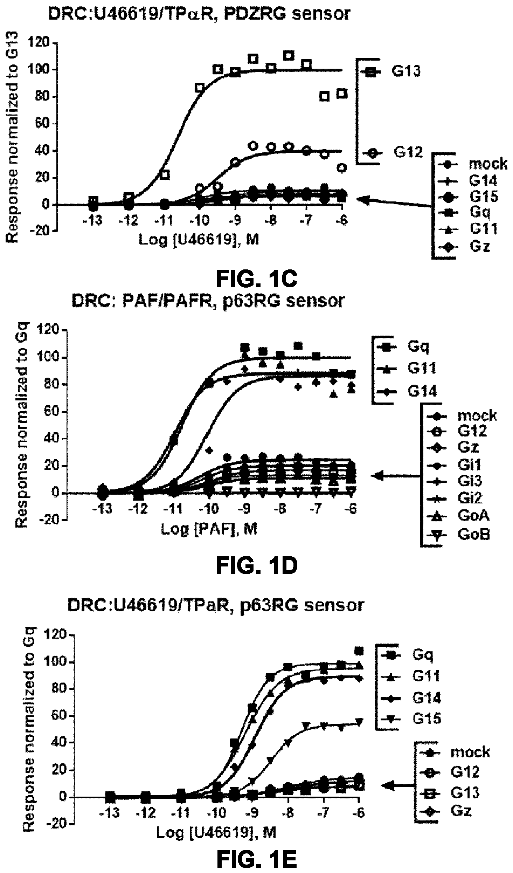

**FIGs 1B-1E** show examples of specificity obtained with three different G protein effectors. Receptors coupled to members of Gq, Gi and G12/13 families were co-expressed with these G proteins, with an rGFP fused to a PM marker (rGFP-CAAX, GKKKKKKSKTKCVIM, SEQ ID NO: 1) and a G protein effector tagged with Rlucll. **FIG. 1B** shows dose response curves (DRCs) obtained with platelet-activating factor (PAF)/PAFR-mediated G protein (Gq, G11, G14, G12, GoA, GoB, Gz, Gi1, Gi2, Gi3) activation, using Rap1GAP (SSS-AAA)-Rlucll and rGFP-CAAX (PM marker). Mock response represent activation of endogenously expressed Gi1, i2 and i3 proteins. **FIG. 1C** shows DRCs obtained with U46619/TPαR-mediated G protein (Gq, G11, G14, G15, G12, G13, Gz) activation, using PDZRG-Rlucll and rGFP-CAAX. Mock response represent activation of endogenously expressed G12 and G13 proteins. **FIGs. 1D and 1E** show DRCs obtained with PAF/PAFR-mediated G protein (Gq, G11, G14, G12, GoA, GoB, Gz, Gi1, Gi2, Gi3) activation (**FIG. 1D**) and U46619/TPαR-mediated G protein (Gq, G11, G14, G15, G12, G13, Gz) activation (**FIG. 1E**), using P63RG-Rlucll and rGFP-CAAX. Mock response represent activation of endogenously expressed Gq and G11 proteins.

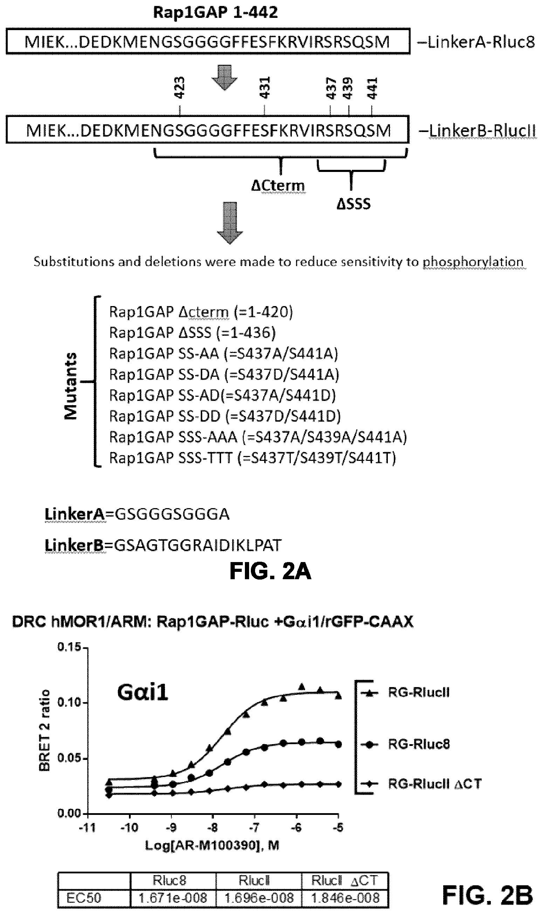

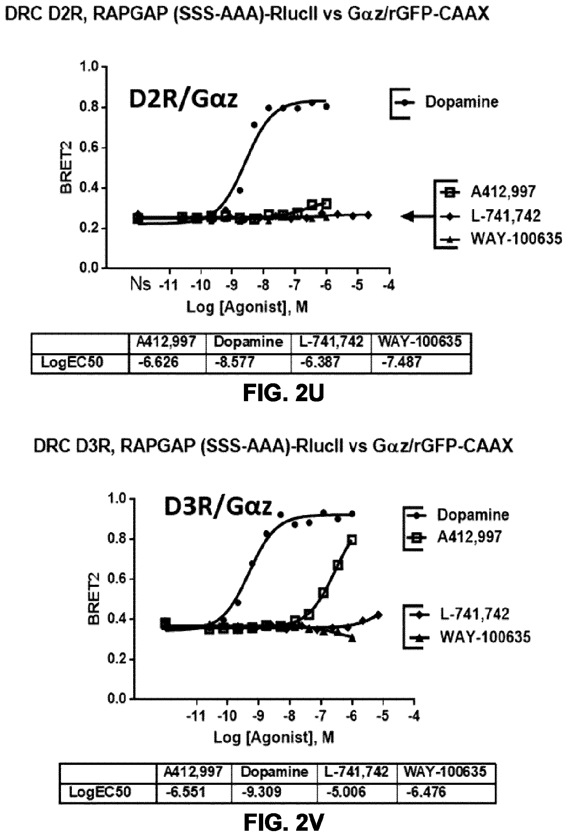

**FIG. 2A** depicts various constructs tested of a Rap1GAP-based system for monitoring activation of G proteins of the Gi family. The first construct consists of a portion (residues 1-442) of Rap1GAP tagged in C-terminal with the BRET donor, Rluc8. From this construct, a second construct was derived using a different linker and using Rlucll instead of Rluc8. The results of the experiments are depicted in **FIGs. 2B-2Z****.** LinkerA = GSGGGSGGGA (SEQ ID NO: 6) and LinkerB = GSAGTGGRAIDIKLPAT (SEQ ID NO: 7). Rap1GAP 1-442 = SEQ ID NO: 8; Rap1GAP Δcterm (1-420) = SEQ ID NO: 10; Rap1GAP ΔSSS = SEQ ID NO: 9; Rap1GAP SS-AA = SEQ ID NO: 13; Rap1GAP SS-DA = SEQ ID NO: 14; Rap1GAP SS-AD = SEQ ID NO: 15; Rap1GAP SS-DD = SEQ ID NO: 16; Rap1GAP SSS-AAA = SEQ ID NO: 11; Rap1GAP SSS-TTT = SEQ ID NO: 12.

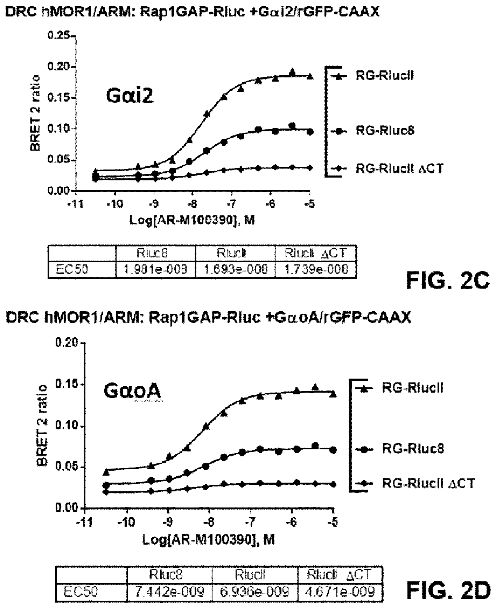

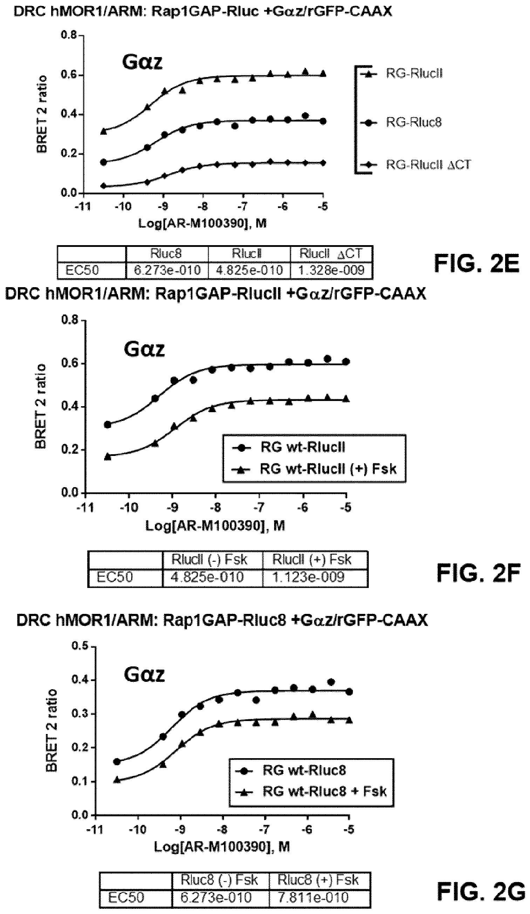

**FIGs. 2B-2E** show DRCs using the human Mu opioid receptor (hMOR1)-mediated activation of Gi1 (**FIG. 2B**), Gi2 (**FIG. 2C**), GoA (**FIG. 2D**) and Gz (**FIG. 2E**) upon AR-M100390 (ARM) stimulation using three different Rap1GAP-Rluc constructs: Rap1GAP (1-442)-Rluc8 (circles), Rap1GAP (1-442)-Rlucll (triangles) and Rap1GAP (ΔCT)-RlucII (diamonds).

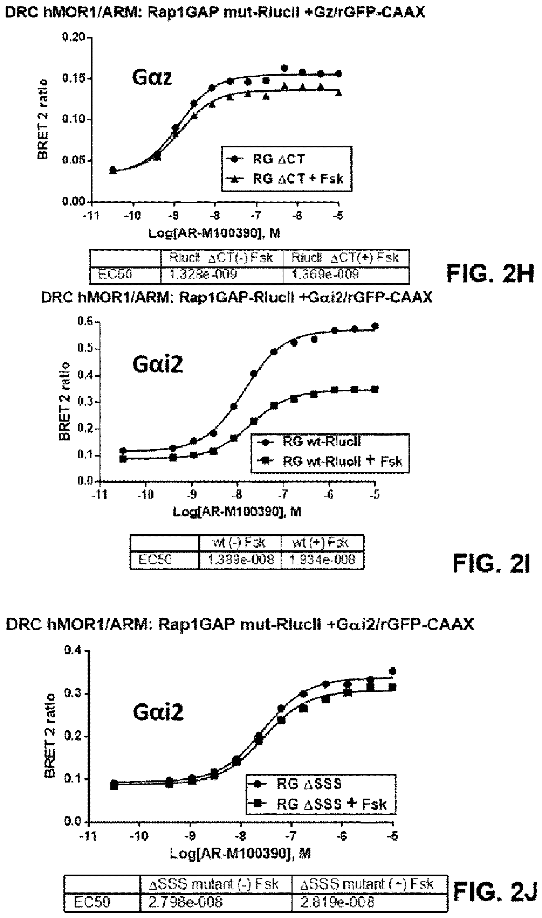

**FIGs. 2F-2H** show DRCs using hMOR1/ARM-promoted activation of Gz at the PM in the presence of forskolin (triangles) or vehicle (DMSO/Tyrode, circles), which promotes an increase cAMP production and activation of protein kinase A leading to phosphorylation of different proteins, using Rap1GAP (1-442)-Rluc8 (**FIG. 2F**), Rap1GAP (1-442)-Rlucll (**FIG. 2G**) or RAP1GAP (ΔCT)-RlucII (**FIG. 2H**).

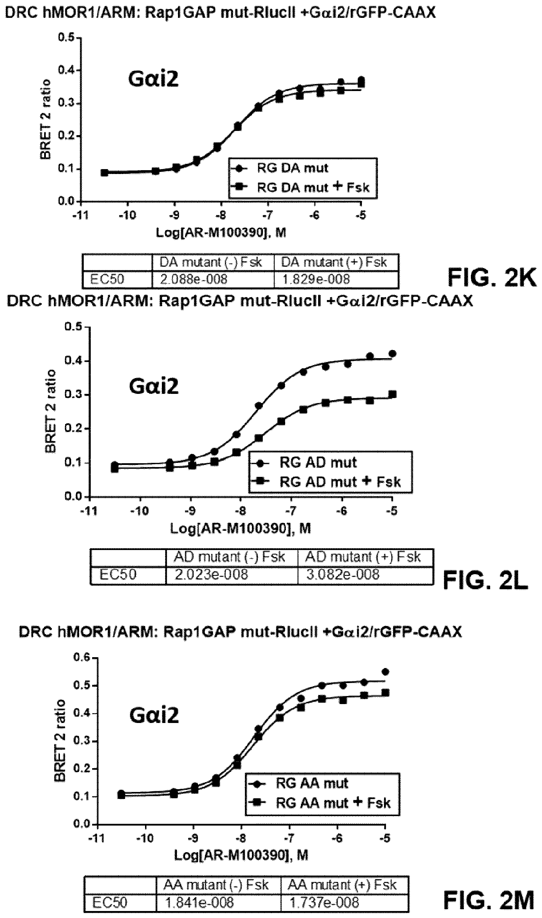

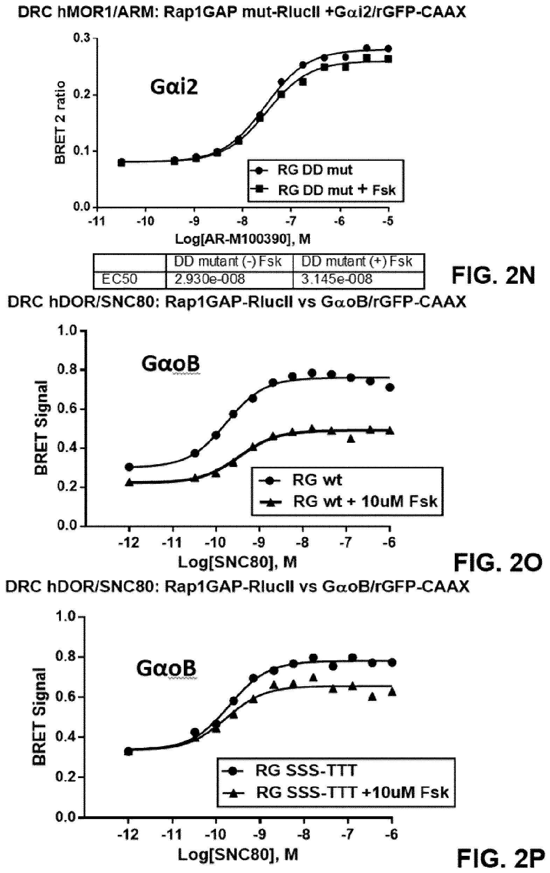

**FIGs. 2I-2N** show DRCs using hMOR1/ARM-promoted activation of Gi2 at the PM in the presence of forskolin (squares) or vehicle (circles) using Rap1GAP (1-442)-Rlucll (**FIG. 2I**), the truncated (residues 1-436) version Rap1GAP (ΔSSS)-RlucII (**FIG. 2J**), Rap1GAP (SS-AD)-Rlucll (**FIG. 2K**), Rap1GAP (SS-DA)-Rlucll (**FIG. 2L**), Rap1GAP (SS-AA)-Rlucll (**FIG. 2M**), or Rap1GAP (SS-DD)-Rlucll (**FIG. 2N**).

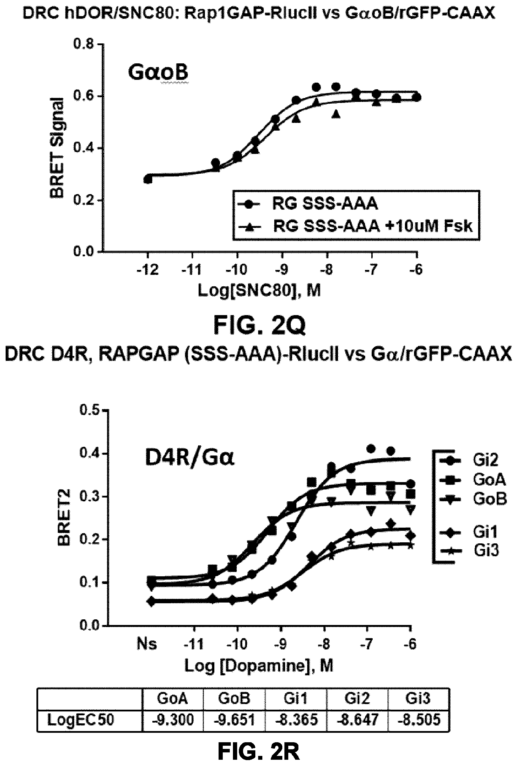

**FIGs. 2O-2Q** show DRCs using hMOR1/ARM-promoted activation of GoB at the PM in the presence of forskolin (triangles) or vehicle (circles) using Rap1GAP (1-442)-Rlucll (**FIG. 2O**), Rap1GAP (SSS-TTT)-Rlucll (**FIG. 2P**) and Rap1GAP (SSS-AAA)-Rlucll (**FIG. 2Q**).
**FIG. 2R** shows DRCs of G protein activation following dopamine-promoted stimulation of the Dopamine D4 receptor (D4R) using Rap1GAP (SSS-AAA)-Rlucll translocation to the plasma membrane, with co-expression of Gi1, Gi2, Gi3, GoA or GoB.

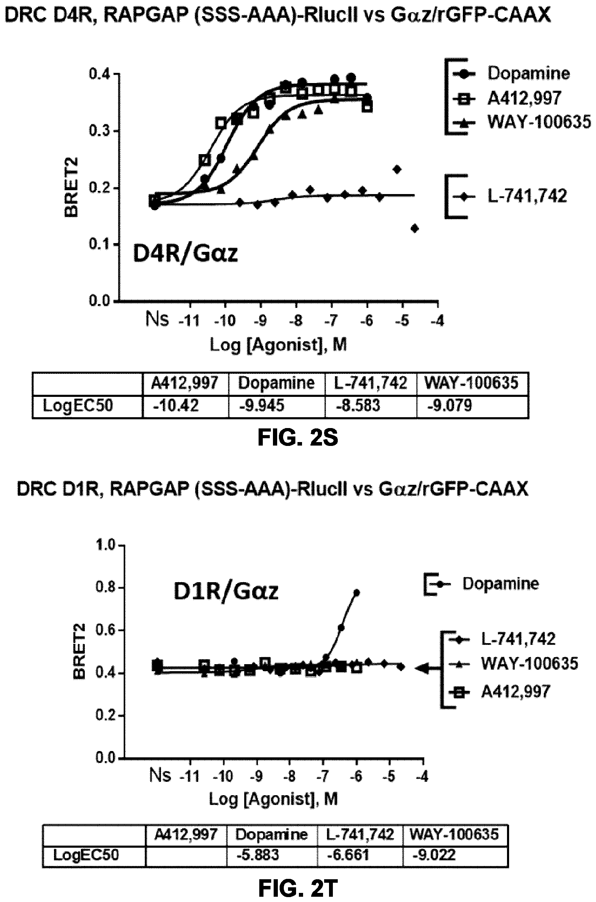

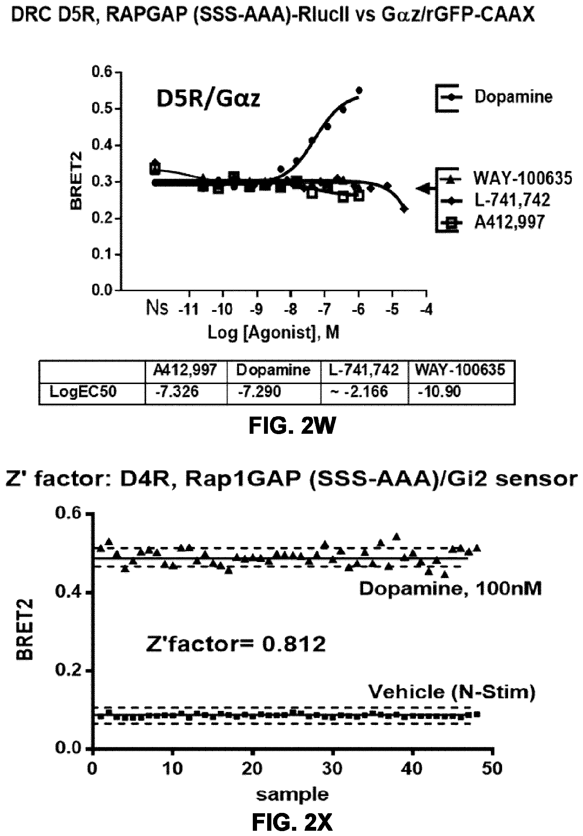

**FIGs. 2S-2W** show DRCs of Gz activation following stimulation of the dopamine receptors D4R (**FIG. 2S**), D1R (**FIG. 2T**), D2R (**FIG. 2U**), D3R (**FIG. 2U**) or D1R (**FIG. 2W**) with the ligands A412,997, Dopamine, L741,742 and Way-100635.

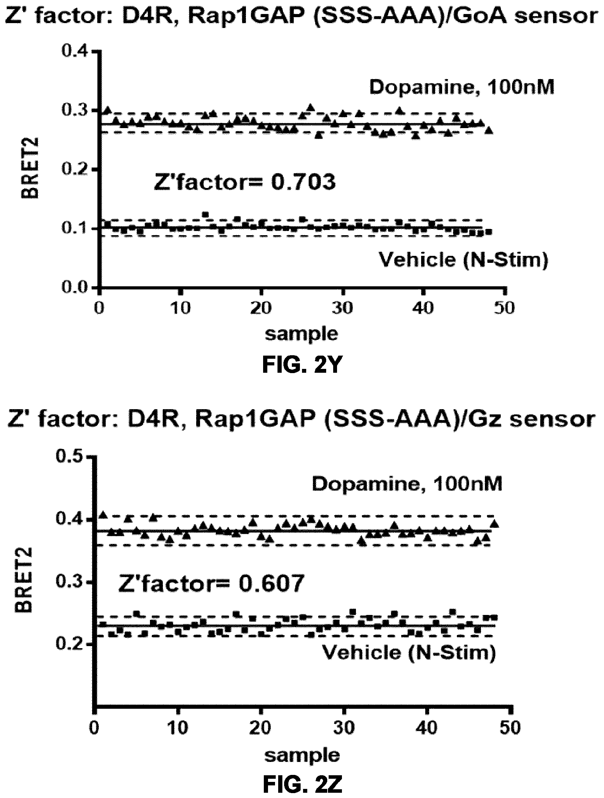

**FIGs. 2X-2Z** are graphs depicting the Z' factors for the assays, which is an indication of the robustness of the assays. HEK293 were co-transfected with D4R, Rap1GAP (SSS-AAA)-RlucII, rGFP-CAAX, together with WT Gi2 (**FIG. 2X**), with WT GoA (**FIG. 2Y**), or WT Gz (**FIG. 2Z**), plated in a 96-well plate and stimulated with 100nM dopamine (triangles) or vehicle (DMSO/Tyrode; squares) at 37°C, for 6-8 min. Recruitment of Rap1GAP (SSS-AAA)-Rlucll to the PM was evaluated in BRET2. BRET values are expressed per well in the presented graphs.

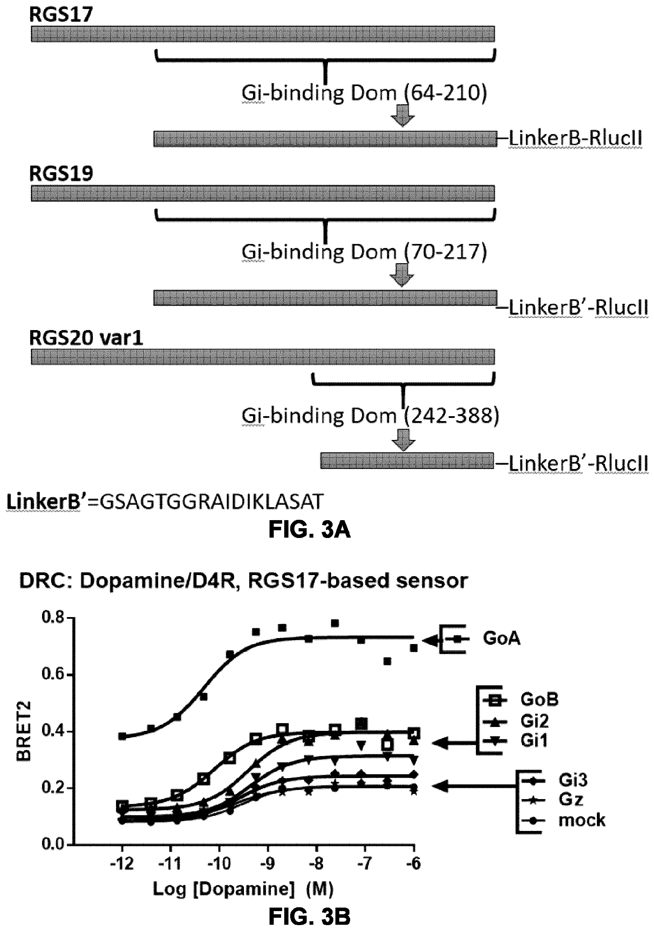

**FIG. 3A** depicts other constructs for monitoring activation of G proteins of the Gi family based on the RGS domain of members of the Regulator of G-protein signaling (RGS) proteins used in the studies described herein. A fragment comprising the RGS (Gi-binding) domain of RGS17 (residues 64-210 of SEQ ID NO: 17), RGS19 (residues 70-217 of SEQ ID NO: 18) and RGS20 (residues 242-388 of SEQ ID NO: 19) is tagged in C-terminal with the BRET donor Rlucll. LinkerB' = GSAGTGGRAIDIKLASAT (SEQ ID NO: 20).

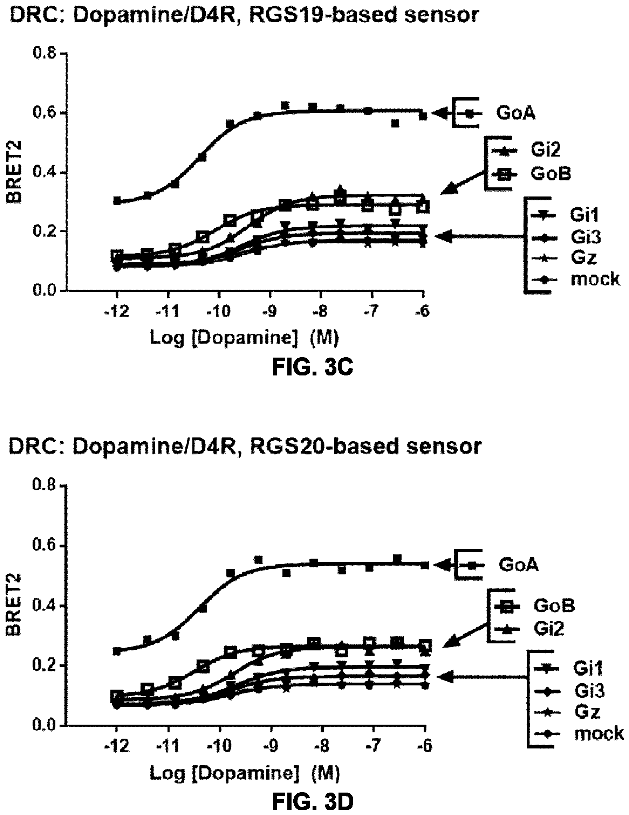

**FIGs. 3B-3D** show DRCs of G protein activation following dopamine-promoted stimulation of D4) using RGS(RGS17)-Rlucll (**FIG. 3B**), RGS(RGS19)-Rlucll (**FIG. 3C**) or RGS(RGS20)-Rlucll (**FIG. 3D**) translocation to the PM, with co-expression of GoA, GoB, Gz, Gi1, Gi2 or Gi3.

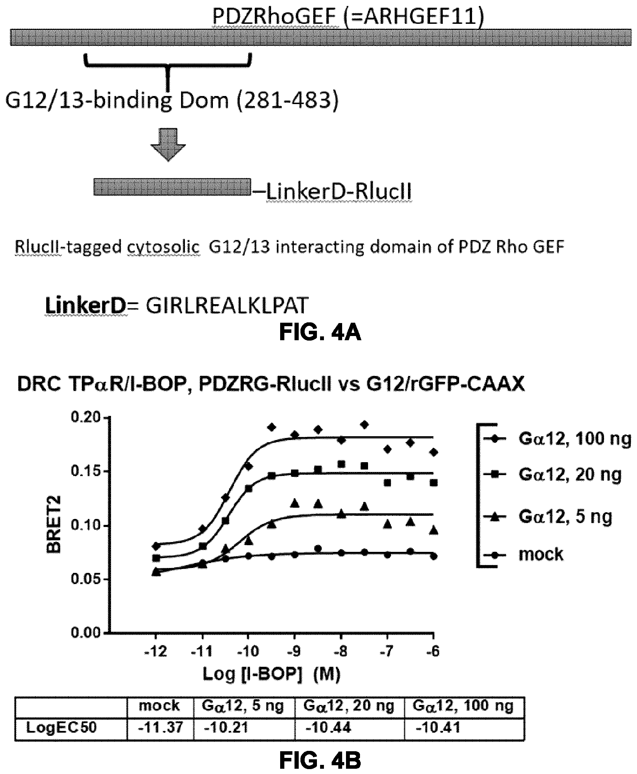

**FIG. 4A** depicts the PDZRhoGEF (PDZRG)-Rlucll construct for monitoring activation of G protein of the G12/13 family used in the studies described herein. A fragment comprising the G12/13 binding domain of PDZRhoGEF (residues 281-483 of SEQ ID NO: 21) is tagged in C-terminal with the BRET donor Rlucll. LinkerD = GIRLREALKLPAT (SEQ ID NO: 22).

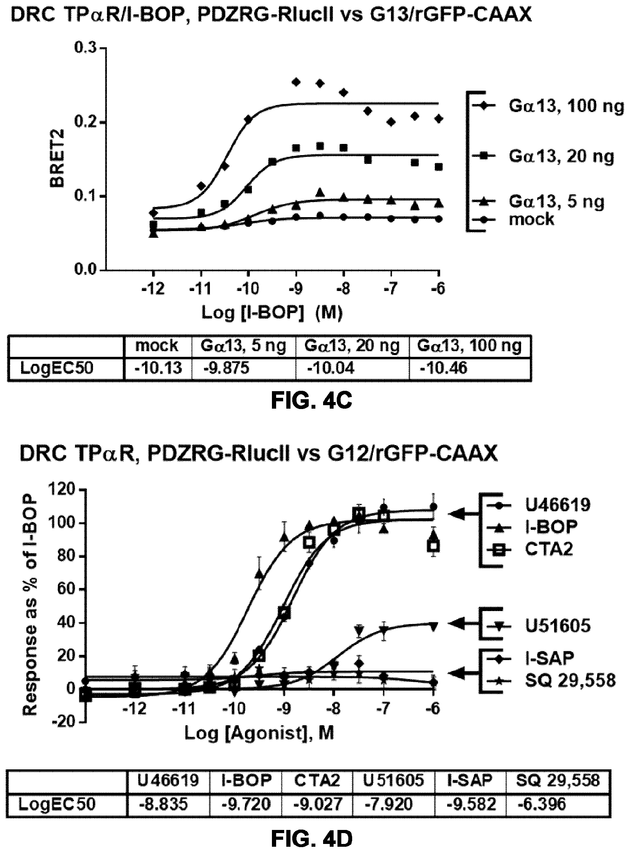

**FIGs. 4B** and **4C** show DRCs of G12 (**FIG. 4B**) and G13 (**FIG. 4C**) activation in HEK293 cells cotransfected with thromboxane receptor (TPαR), PDZRG-Rlucll, rGFP-CAAX and with either no Gα (Mock), 5 ng of Gα, 20 ng of Gα or 100 ng of Gα, following stimulation with the TPαR agonist I-BOP (CAS Number: 128719-90-4).

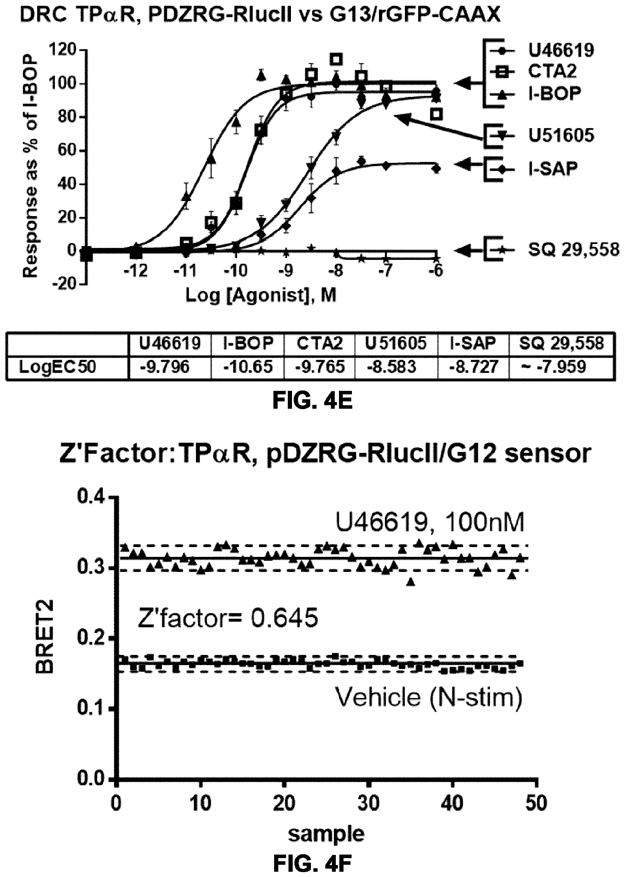

**FIGs. 4D** and **4E** show DRCs of TPαR ligands on G12 (**FIG. 4D**) and 13 (**FIG. 4E**) activation at the PM using PDZRG-Rlucll. HEK293 cells cotransfected with thromboxane receptor (TPαR), PDZRG-Rlucll, rGFP-CAAX were stimulated with known full agonists (U46619, I-BOP, CTA2), with a partial agonist (U51605) and the antagonists I-SAP and SQ 29,558. Results were normalized and presented as % of I-BOP response (n=4) +/- SEM.

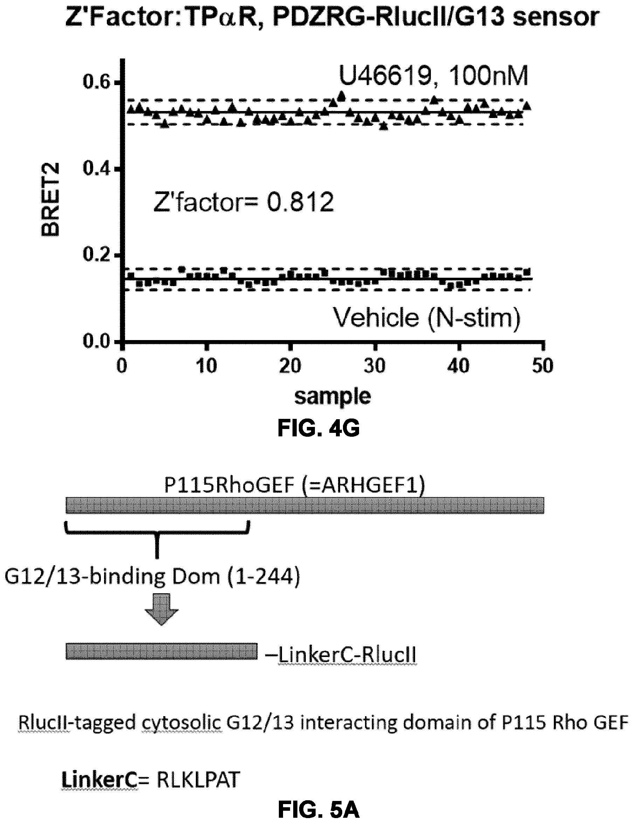

**FIGs. 4F** **and** **4G** are graphs depicting the Z' factors for the assays using the PDZRG-Rlucll construct. HEK293 were co-transfected with TPαR, PDZRG-Rlucll, rGFP-CAAX and with WT G12 (**FIG. 4F**) or WT G13 (**FIG. 4G**), plated in a 96-well plate and stimulated with 100 nM of the TPαR agonist U46619 (triangles) or vehicle (methyl acetate/Tyrode; squares) at 37°C, for 6-8 min. Recruitment of PDZRG-Rlucll to the PM was evaluated in BRET2. BRET values are expressed per well in the presented graphs.
**FIG. 5A** depicts the P115RhoGEF(P115RG)-Rlucll construct for monitoring activation of G protein of the G12/13 family used in the studies described herein. A fragment comprising the G12/13 binding domain of P115RhoGEF (residues 1-244 of SEQ ID NO:23) is tagged in C-terminal with the BRET donor Rlucll. LinkerC = RLKLPAT (SEQ ID NO: 24).

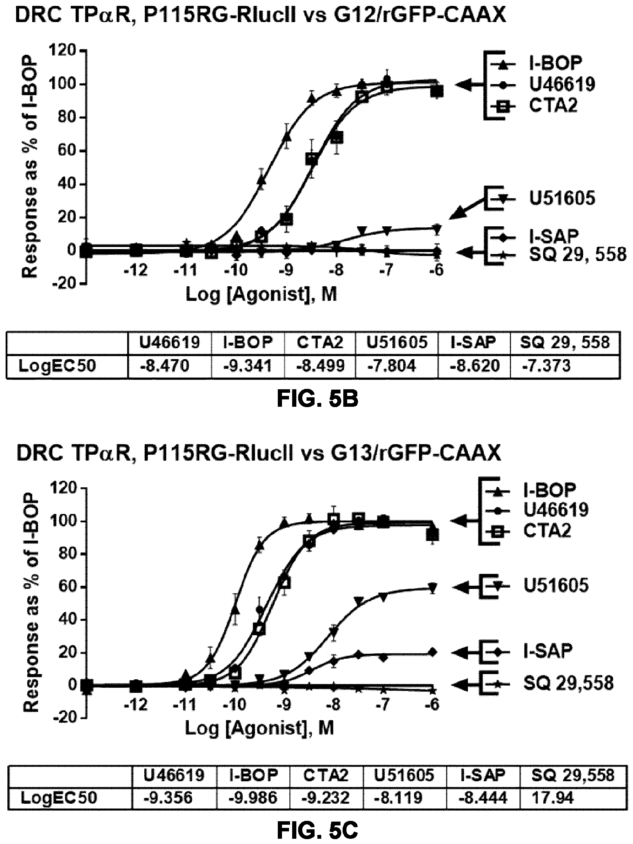

**FIGs. 5B** and **5C** show DRCs of TPαR ligands on G12 (**FIG. 5B**) and G13 (**FIG. 5C**) activation at the PM using P115RG-Rlucll. HEK293 cells cotransfected with thromboxane receptor (TPαR), P115RG-Rlucll, rGFP-CAAX were stimulated with U46619, I-BOP, CTA2, U51605, I-SAP and SQ 29,558. Results were normalized and presented as % of I-BOP response (n=4) +/- SEM.

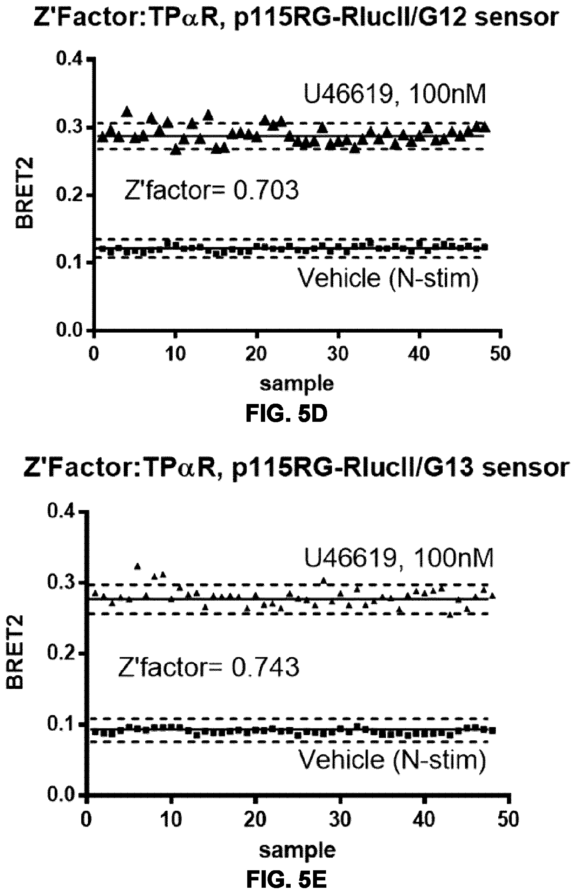

**FIGs. 5D and 5E** are graphs depicting the Z' factors for the assays using the PDZRG-Rlucll construct. HEK293 were co-transfected with TPαR, P115RG-Rlucll, rGFP-CAAX and with WT G12 (**FIG. 5D**) or WT G13 (**FIG. 5E**), plated in a 96-well plate and stimulated with 100 nM of the TPαR agonist U46619 (triangles) or vehicle (methyl acetate/Tyrode; squares) at 37°C, for 6-8 min. Recruitment of P115RG-Rlucll to the PM was evaluated in BRET2. BRET values are expressed per well in the presented graphs.

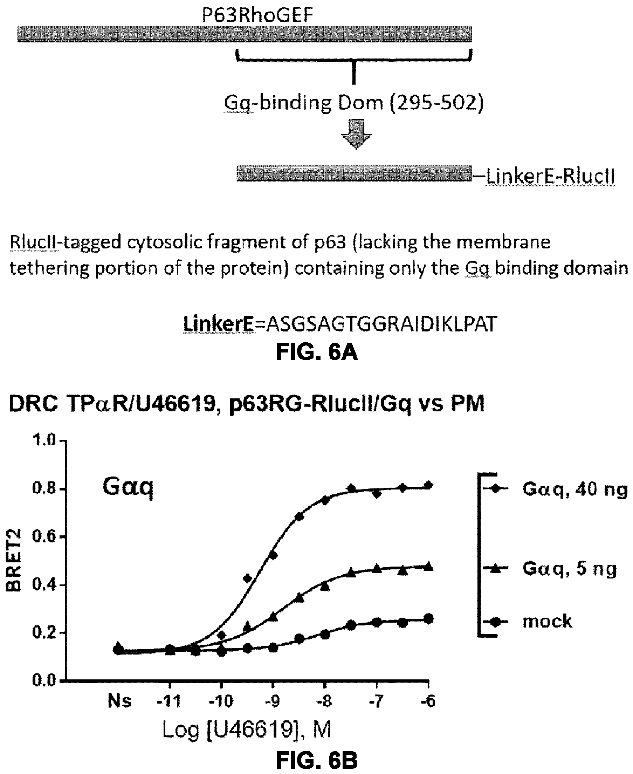

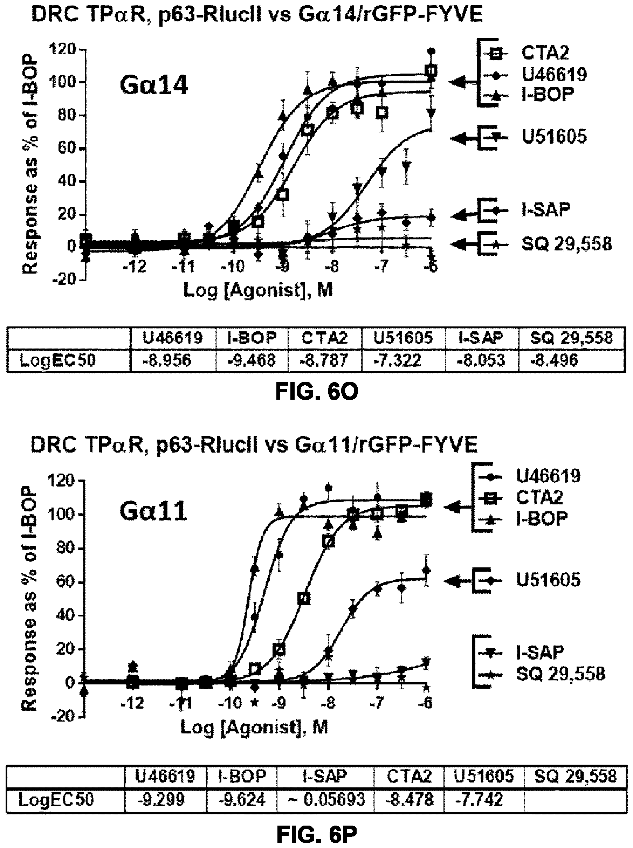

**FIG. 6A** depicts the P63RhoGEF (P63RG) construct for monitoring activation of G proteins of the Gq family (Gq, G11, G14 & G15) used in the studies described herein. A fragment comprising the Gq binding domain of P63RhoGEF (residues 295-502 of SEQ ID NO: 25) is tagged in C-terminal with the BRET donor Rlucll. LinkerE = ASGSAGTGGRAIDIKLPAT (SEQ ID NO: 26).

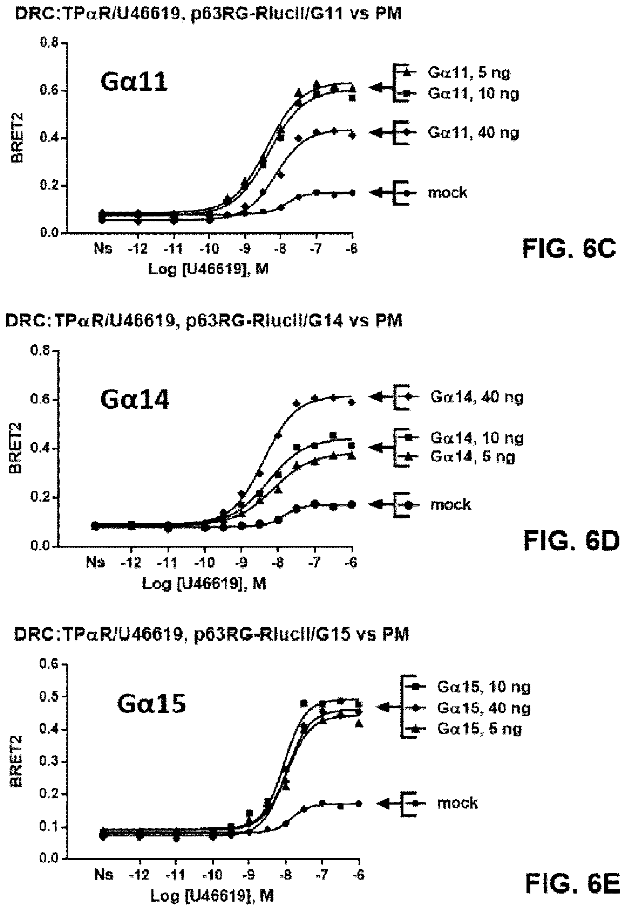

**FIGs. 6B-6E** show DRCs of Gq (**FIG. 6B**), G11 (**FIG. 6C**)**,** G14 (**FIG. 6D**) and G15 (**FIG. 6E**) activation at the PM in HEK293 cells cotransfected with TPαR, P63RG-Rlucll, rGFP-CAAX, and either no Gα (Mock, responses obtained from endogenous G proteins) or different quantities of Gα subunit, following stimulation with the TPαR agonist U46619.

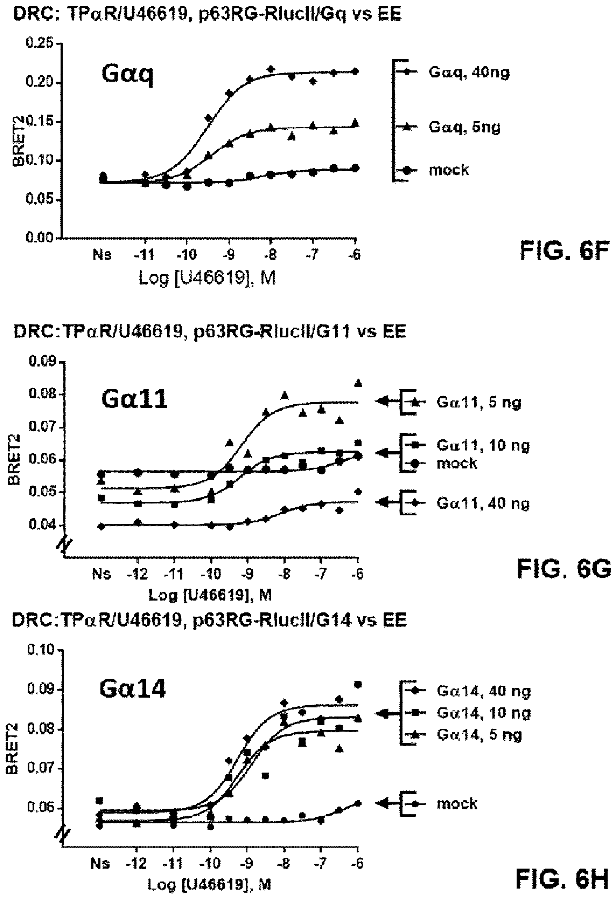

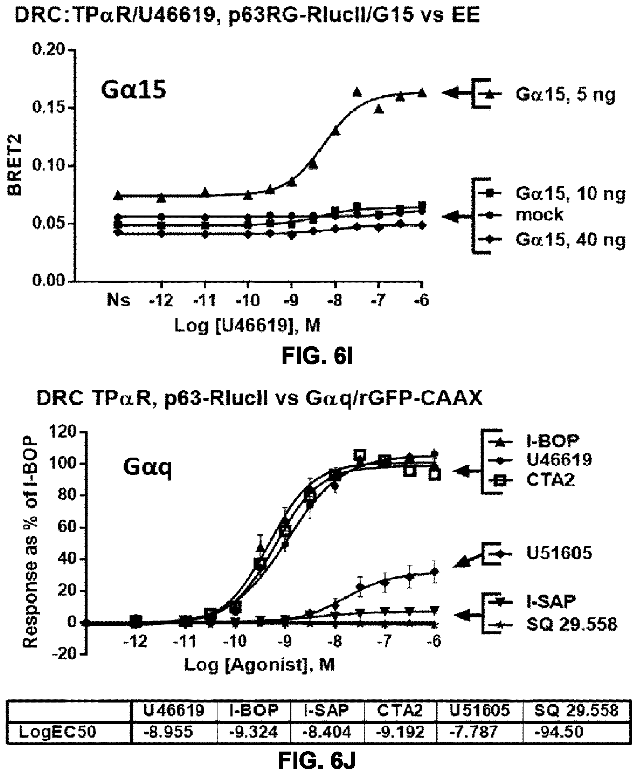

**FIGs. 6F-6I** show DRCs of Gq (**FIG. 6F**), G11 (**FIG. 6G**), G14 (**FIG. 6H**) and G15 (**FIG. 6I**) activation at the early endosomes (EE) in HEK293 cells cotransfected with TPαR, P63RG-Rlucll, rGFP-FYVE, and either no Gα (Mock, responses obtained from endogenous G proteins) or different quantities of Gα subunit, following stimulation with the TPαR agonist U46619.

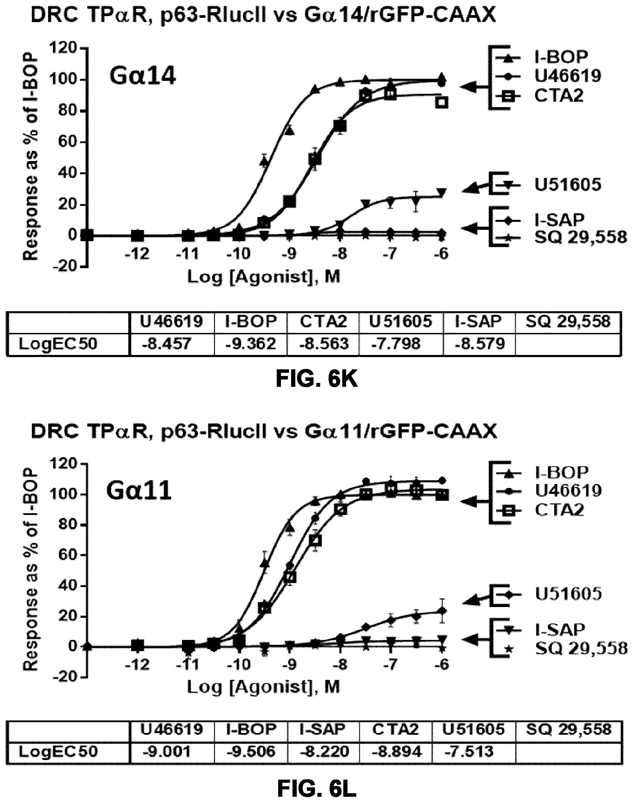

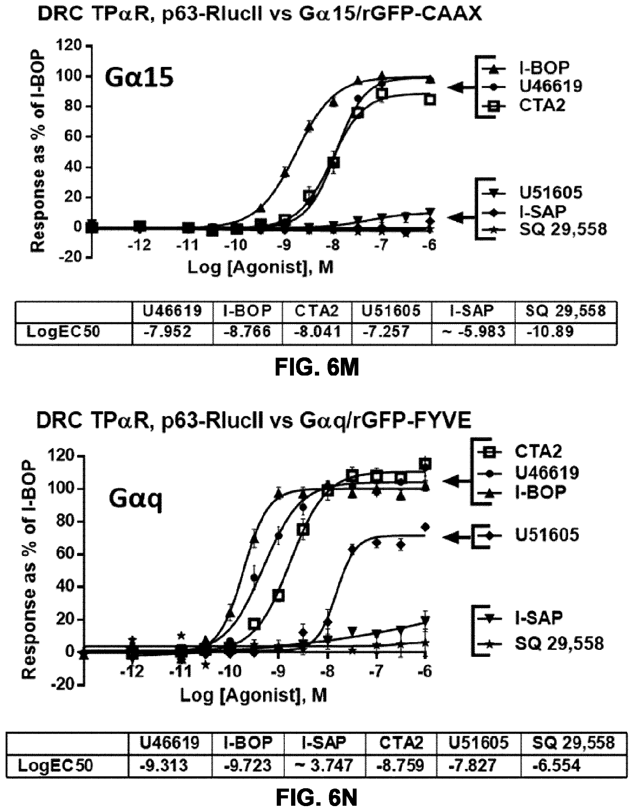

**FIGs. 6J-6M** show DRCs of TPαR ligands on Gq (**FIG. 6J**), G11 (**FIG. 6K**), G14 (**FIG. 6L**) and G15 (**FIG. 6M**) activation at the PM using P63RG-Rlucll under the optimal conditions determined in **FIGs. 6B-6E****.** HEK293 cells cotransfected with thromboxane receptor (TPαR), P63RG-Rlucll, rGFP-CAAX were stimulated with U46619, I-BOP, CTA2, U51605, I-SAP and SQ 29,558. Results were normalized and presented as % of I-BOP response (between n=3 and n=5) +/- SEM.

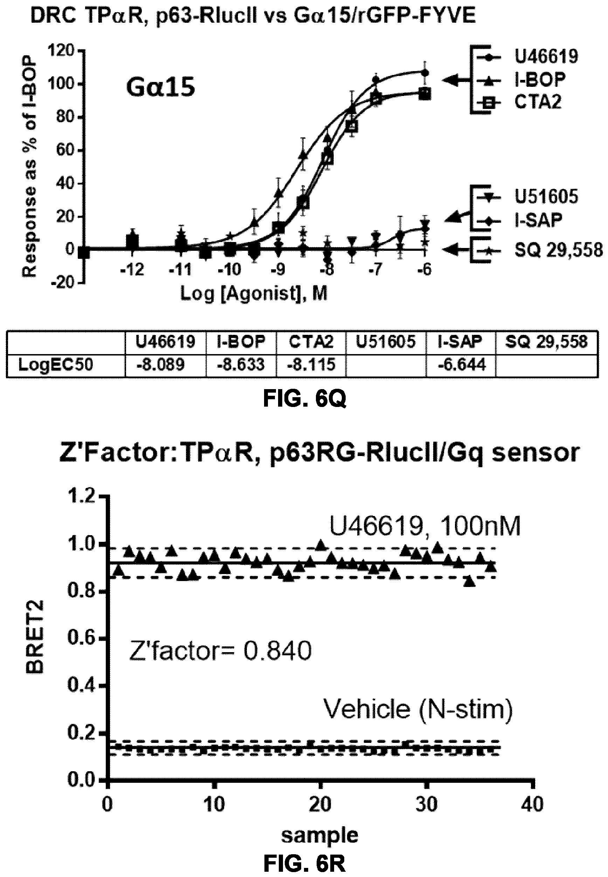

**FIGs. 6N-6Q** show DRCs of TPαR ligands on Gq (**FIG. 6N**), G11 (**FIG. 6O**), G14 (**FIG**. **6P**) and G15 (**FIG. 6Q**) activation at the EE using P63RG-Rlucll under the optimal conditions determined in FIGs. 6F-6I. HEK293 cells cotransfected with thromboxane receptor (TPαR), P63RG-Rlucll, rGFP-FYVE were stimulated with U46619, I-BOP, CTA2, U51605, I-SAP and SQ 29,558. Results were normalized and presented as % of I-BOP response (between n=3 and n=5) +/- SEM.

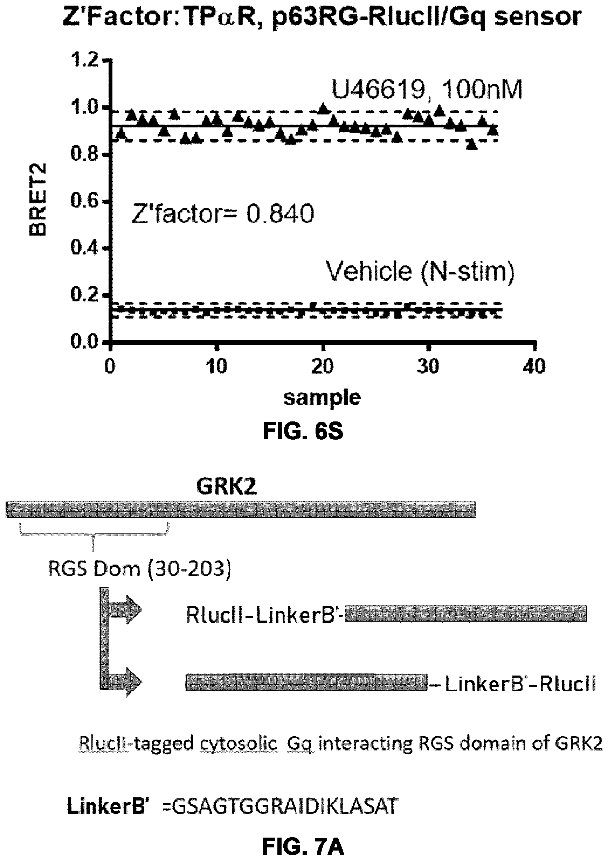

**FIGs. 6R** and **6S** are graphs depicting the Z' factors for the assays using the P63RG-Rlucll construct. HEK293 were co-transfected with TPαR, P63RG-Rlucll, rGFP-CAAX and with WT Gq (**FIG. 6R**) or WT G11 (**FIG. 6S**), plated in a 96-well plate and stimulated with 100 nM of the TPαR agonist U46619 (triangles) or vehicle (methyl acetate/Tyrode; squares) at 37°C, for 6-8 min. Recruitment of P63RG-Rlucll to the PM was evaluated in BRET2. BRET values are expressed per well in the presented graphs.
**FIG. 7A** depicts two RGS(GRK2) constructs for monitoring activation of G proteins of the Gq family (Gq, G11, G14 & G15) used in the studies described herein. A fragment comprising the Gq binding domain (RGS domain) of GRK2 (residues 30-203 of SEQ ID NO: 27) is tagged at the N-terminal Rlucll-RGS(GRK2) or C-terminal (RGS(GRK2)-Rlucll) with the BRET donor Rlucll. LinkerB' = GSAGTGGRAIDIKLASAT (SEQ ID NO: 20).

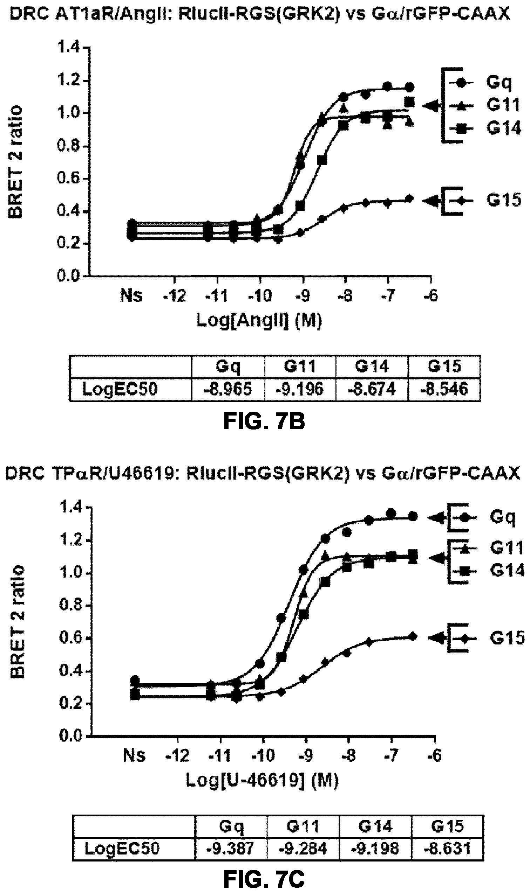

**FIGs. 7B** and **7C** show DRCs of the activation of Gq, G11, G14 and G15 at the PM in two ligand/receptor systems, namely At1AR/ANGII (**FIG. 7B**) and TPαR/U46619 (**FIG. 7C**), using RlucII-RGS(GRK2).

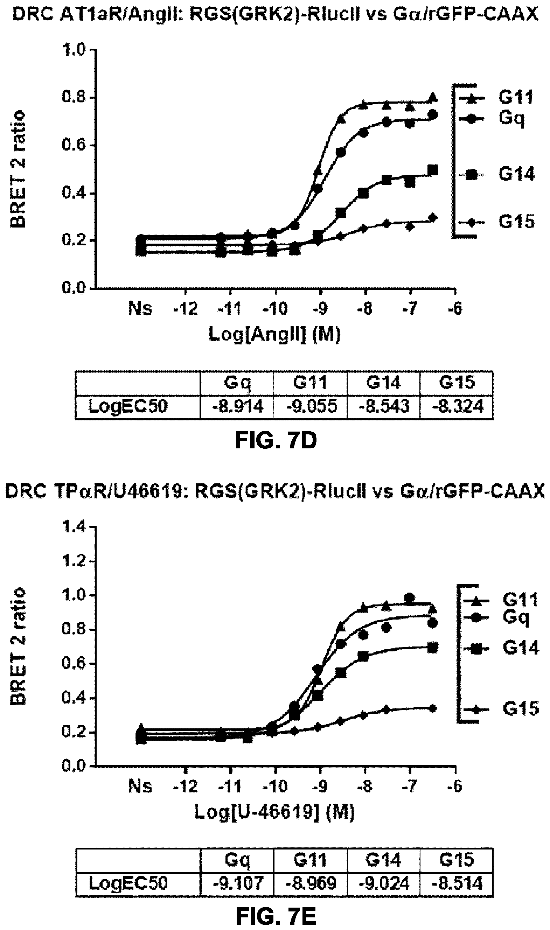

**FIGs. 7D** and **7E** show DRCs of the activation of Gq, G11, G14 and G15 at the PM in two ligand/receptor systems, namely At1AR/ANGII (**FIG. 7D**) and TPαR/U46619 (**FIG. 7E**), using RGS(GRK2)-Rlucll.

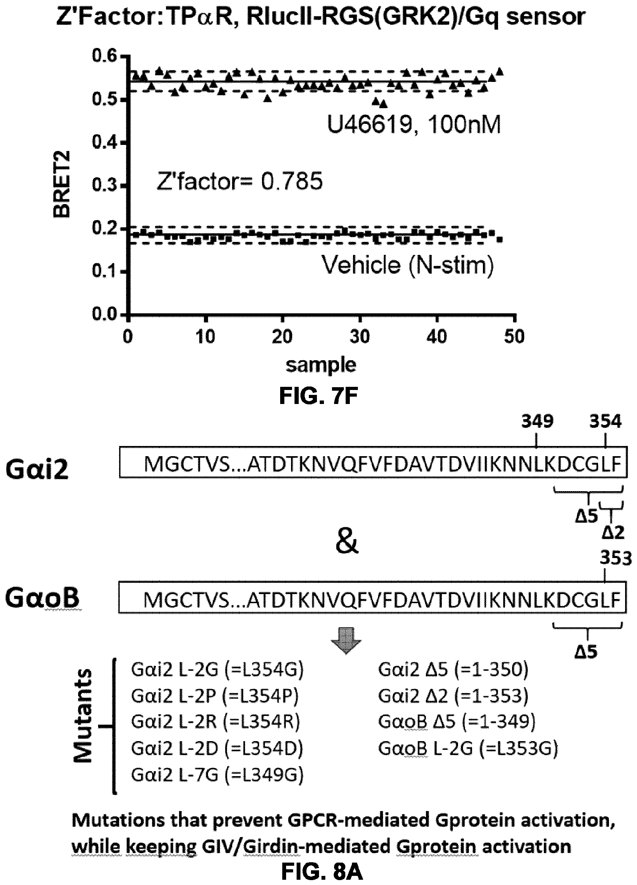

**FIG. 7F** is a graph depicting the Z' factors for the assays using the Rlucll-RGS(GRK2) construct. HEK293 were co-transfected with TPαR, Rlucll-RGS(GRK2), rGFP-CAAX and with WT Gq, plated in a 96-well plate and stimulated with 100 nM of the TPαR agonist U46619 (triangles) or vehicle (methyl acetate/Tyrode; squares) at 37°C, for 6-8 min. Recruitment of Rlucll-RGS(GRK2) to the PM was evaluated in BRET2. BRET values are expressed per well in the presented graphs.
**FIG. 8A** depicts mutated Gi2 and GoB proteins tested in the studies described herein for their ability to monitor GEF-mediated activation of G proteins, i.e. G protein activation not mediated by GPCRs. Gαi2 Δ5 = SEQ ID NO: 28; Gαi2 Δ2 = SEQ ID NO: 29; Gαi2 L-2G = SEQ ID NO: 31; Gαi2 L-2P = SEQ ID NO: 33; Gαi2 L-2R = SEQ ID NO: 34; Gαi2 L-2D = SEQ ID NO: 32; Gαi2 L-7G = SEQ ID NO: 30; GαoB Δ5 = SEQ ID NO: 36; GαoB L-2G = SEQ ID NO: 35.

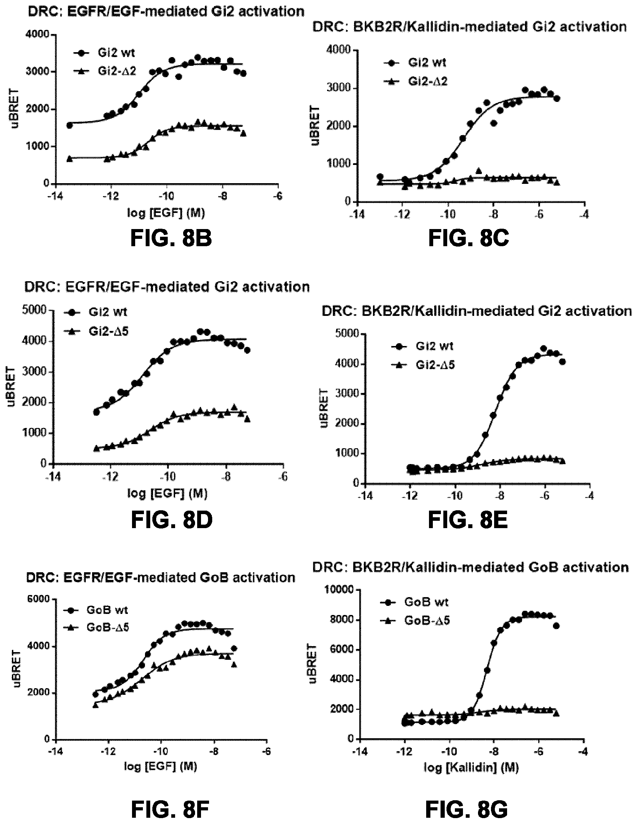

**FIGs. 8B-8G** show DRCs of the activation of deletion mutants of Gi2 and GoB, namely Gi2 Δ2 (**FIGs. 8B** and **8C**), Gi2 Δ5 (**FIGs. 8D** and **8E**) and GoB Δ5 (**FIGs. 8F** and **8G**). HEK293 were co-transfected with constructs encoding either the EGF receptor (EGFR, **FIGs. 8B, 8D, 8F**) or the bradykinin receptor (BKB2R, **FIGs. 8C, 8E, 8G**), Rap1GAP (SSS-AAA)-Rlucll, rGFP-CAAX and the WT or mutated Gi2/GoB subunits.

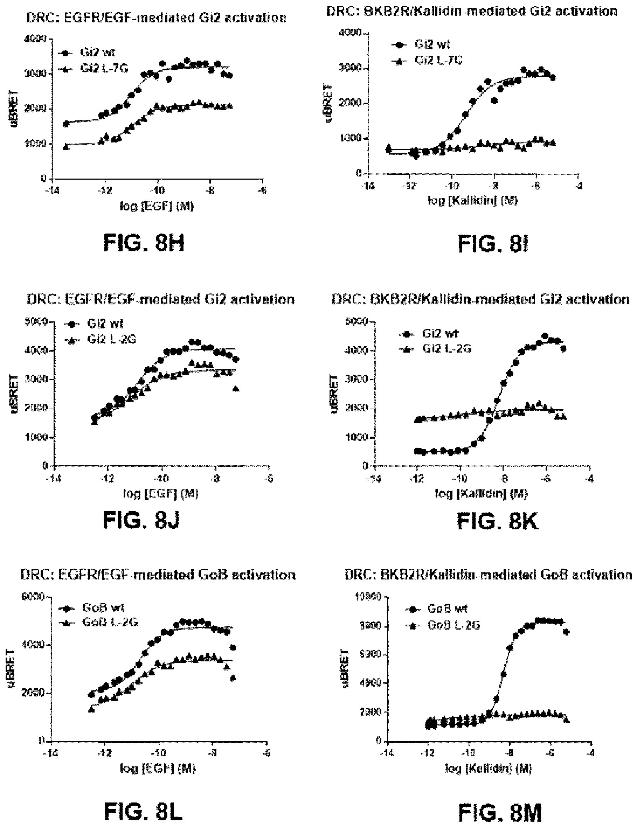

**FIGs. 8H-8M** show DRCs of the activation of Leu to Gly mutants of Gi2 and GoB, namely Gi2 L-7G (**FIGs. 8H** and **8I**), Gi2 L-2G (**FIGs. 8J** and **8K**) and GoB L-2G (**FIGs. 8L** and **8M**). HEK293 were co-transfected with constructs encoding either EGFR (**FIGs. 8H, 8J, 8L**) or BKB2R (**FIGs. 8I, 8K, 8M**), Rap1GAP (SSS-AAA)-Rlucll, rGFP-CAAX and the WT or mutated Gi2/GoB subunits.

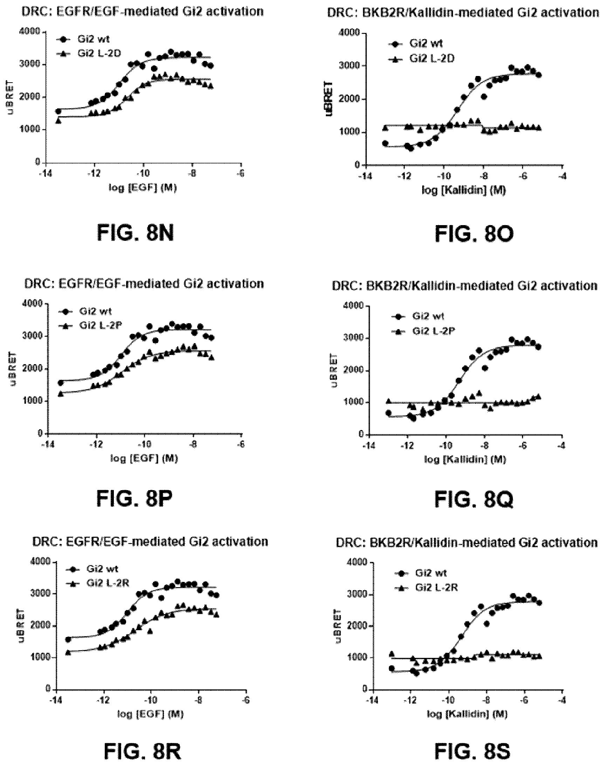

**FIGs. 8N-8S** show DRCs of the activation of position -2 mutants of Gi2, namely Gi2 L-2D (**FIGs**. **8N** and 8O), Gi2 L-2P (**FIGs**. **8P** and **8Q**) and Gi2 L-2R (**FIGs. 8R** and **8S**). HEK293 were co-transfected with constructs encoding either EGFR (**FIGs. 8N, 8P, 8R**) or BKB2R (**FIGs. 8O**, **8Q**, **8S**), Rap1GAP (SSS-AAA)-Rlucll, rGFP-CAAX and the WT or mutated Gi2 subunits.

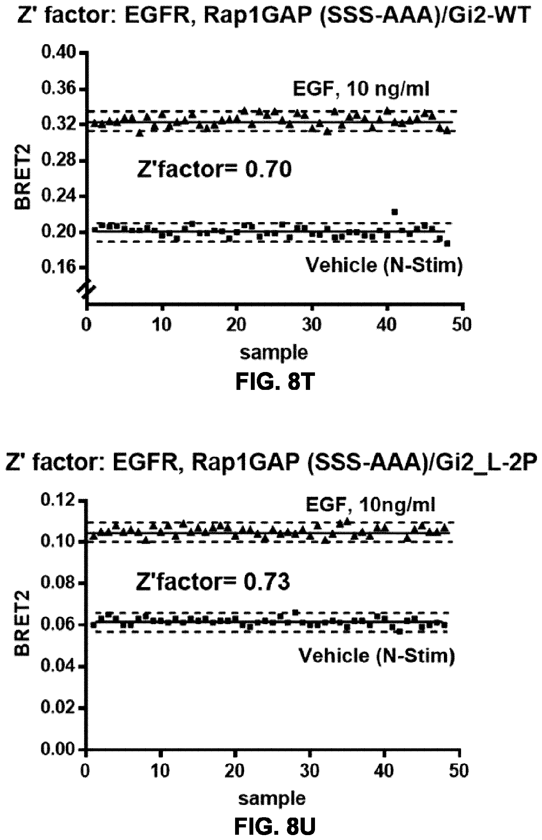

**FIGs. 8T-8U** are graphs depicting the Z' factors for the assay monitoring GEF-mediated activation of G proteins using the Rap1GAP (SSS-AAA)-Rlucll construct. HEK293 were co-transfected with EGFR, Rap1GAP (SSS-AAA)-Rlucll, rGFP-CAAX and WT Gi2 (**FIG. 8T**) or mutant Gi2 L-2P (**FIG. 8U**), plated in a 96-well plate and stimulated with 10 ng/ml of EGF (triangles) or vehicle (Tyrode; squares) at RT, for 2 min. Recruitment of Rap1GAP (SSS-AAA)-Rlucll to the PM was evaluated in BRET2. BRET values are expressed per well in the presented graphs.
**FIGs**. **8V-8X** show DRCs of Gi2 activation by two GPCRs, delta-opioid receptor (DOR) in **FIG. 8V** and D2R in **FIG. 8W**, and an RTK (EGFR; **FIG. 8X**), monitored using Rap1GAP (SSS-AAA)-Rlucll construct (circles) and RGS(RGS17)-Rlucll (triangles). HEK293 were co-transfected with constructs encoding a receptor (DOR, D2R or EGFR), Rap1GAP (SSS-AAA)-Rlucll or RGS(RGS17)-Rlucll, rGFP-CAAX and WT Gi2, plated in a 96-well plate and stimulated with the indicated doses at RT, for 8 min with a GPCR agonist (**FIGs. 8V, 8W**) or 7 min with EGF (**FIG. 8X**). Recruitment of Rap1GAP (SSS-AAA)-Rlucll and RGS(RGS17)-Rlucll to the PM was evaluated in BRET2. DRCs presented are representative of three independent experiments.

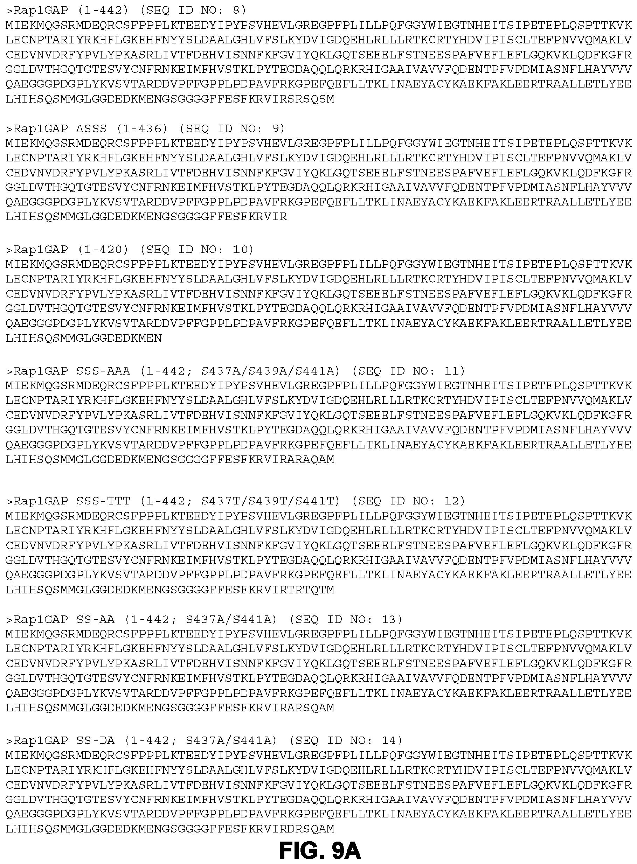

**FIGs. 9A-9D** show the amino acid sequences of polypeptides used in the studies described herein.

### DISCLOSURE OF INVENTION

Terms and symbols of genetics, molecular biology, biochemistry and nucleic acid used herein follow those of standard treatises and texts in the field, *e.g.* Kornberg and Baker, DNA Replication, Second Edition (W University Science Books, 2005); Lehninger, Biochemistry, 6th Edition (W H Freeman & Co (Sd), New York, 2012); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); and the like. All terms are to be understood with their typical meanings established in the relevant art.

The articles "a" and "an" are used herein to refer to one or to more than one (i.e. to at least one) of the grammatical object of the article. By way of example, "an element" means one element or more than one element. Throughout this specification, unless the context requires otherwise, the words "comprise," "comprises" and "comprising" will be understood to imply the inclusion of a stated step or element or group of steps or elements but not the exclusion of any other step or element or group of steps or elements.

The description relies on information, including concerning nucleotide and amino acid sequences, corresponding to the Genbank, RefSeq, UniProt, NCBI and/or Ensembl accession numbers (or any other database).

All methods described herein can be performed in any suitable order unless otherwise indicated herein or otherwise clearly contradicted by context.

The use of any and all examples, or exemplary language (*"e.g.",* "such as") provided herein, is intended merely to better illustrate the disclosure.

Herein, the term "about" has its ordinary meaning. The term "about" is used to indicate that a value includes an inherent variation of error for the device or the method being employed to determine the value, or encompass values close to the recited values, for example within 10% or 5% of the recited values (or range of values).

Any and all combinations and subcombinations of the embodiments and features disclosed herein are encompassed by the present disclosure.

The present disclosure relates to systems and assays allowing the monitoring of G protein activation in a G protein family-selective manner, and at different cellular compartments. These systems and assays are based on the use of specific effectors of G proteins and cellular compartment markers, tagged with suitable energy (i.e. BRET) donors and acceptors. These systems and assays advantageously do not, and according to the invention do not, require the modification (*e.g.,* tagging/fusion with a BRET donor or acceptor or other detectable labels) of any of the G protein subunits (Gα, Gβ or Gγ) as G protein activation at a particular cellular compartment is indirectly detected in a G protein family-selective manner by assessing the translocation of specific G protein effectors, thus minimizing the risk that the function/activity of the G protein be altered. The dynamic window obtained with the system described herein is higher than that obtained with other systems for assessing G protein activation (*e.g.,* U.S. Patent No. 9,029,097 and WO/2016/058094), which may be important for high throughout screening (HTS) applications (including the identification of partial agonists), and also permits the detection of the signal mediated by endogenous G proteins (at the G protein family level). Specificity is achieved by using G protein-binding domains of G protein effectors specific to the G protein subunit(s) of interest. Examples of such G protein-binding domains are the G protein-binding domain of Rap1GAP for the Gi family (Gi1, Gi2, Gi3, GoA, GoB, Gz), the G protein-binding domain of P63RhoGEF (P63RG) for the Gq family (Gq, G11, G14 & G15) and the G protein-binding domain of PDZRhoGEF or P115RhoGEF for the G12/13 family. When needed, specificity at the G protein subunit level may be achieved by co-expressing the G protein subunit(s) of interest with the studied receptor.

Accordingly, the present disclosure describes a system for measuring modulation of G protein activation in a Gα protein subunit family-selective manner, said system, although not claimed, comprising:
a cell expressing:
(i) a first component comprising a Gα subunit interacting polypeptide (GASIP) tagged with a bioluminescent donor molecule or a fluorescent acceptor molecule;
   wherein:
   if said Gα protein subunit family is Gi, said GASIP comprises a domain of a protein that binds to Gi;
   if said Gα protein subunit family is Gq, said GASIP comprises a domain of a protein that binds to Gq;
   if said Gα protein subunit family is G12/13, said GASIP comprises a domain of a protein that binds to G12/13; and
   if said Gα protein subunit family is Gs, said GASIP comprises a domain of a protein that binds to Gs; and
(ii) a second component comprising a plasma membrane (PM)-targeting moiety, an endosomal-targeting moiety or a Golgi-targeting moiety tagged with a bioluminescent donor molecule or a fluorescent acceptor molecule;
wherein if said GASIP is tagged with said fluorescent acceptor molecule, said PM-targeting moiety, endosomal-targeting moiety or Golgi-targeting moiety is tagged with said bioluminescent donor molecule, and if said GASIP is tagged with said bioluminescent donor molecule, said PM-targeting moiety, endosomal-targeting moiety or Golgi-targeting moiety is tagged with said fluorescent acceptor molecule.

In another aspect, the present disclosure describes a system for measuring modulation of G protein activation in a Gα protein subunit family-selective manner, said system, although not claimed, comprising:
a cell expressing:
(i) a first component comprising a Gα subunit interacting polypeptide (GASIP) tagged with a bioluminescent donor molecule or a fluorescent acceptor molecule;
   wherein:
   if said Gα protein subunit family is Gi, said GASIP comprises a G protein-binding domain of: Rap1GAP, a Regulator of G-protein signaling (RGS) protein or TNFAIP8, preferably a G protein-binding domain of Rap1GAP or an RGS protein;
   if said Gα protein subunit family is Gq, said GASIP comprises a G protein-binding domain of: a RhoGEF protein (*e.g.,* P63RhoGEF or TRIO), GRK2, GRK3, TPR1 (tetratricopeptide repeat domain 1) or an RGS protein (*e.g.,* RGS2), preferably a G protein-binding domain of P63RhoGEF or GRK2/GRK3;
   if said Gα protein subunit family is G12/13, said GASIP comprises a G protein-binding domain of a RhoGEF protein (PDZRhoGEF, P115RhoGEF or LARG), JNK-Interacting Leucine Zipper Protein (JLP), cadherin, Axin1, PP2A, SNAP-alpha, polycistin1, RGS16, AKAP110 or HAX1 (HS1-associated protein X1), preferably a G protein-binding domain of a PDZRhoGEF or P115RhoGEF;
   if said Gα protein subunit family is Gs (Gαₛ, XLGαₛ, Gαolf), said GASIP comprises a G protein-binding domain of SNX13 (RGS-PX1), AXIN1 or TPR1 (tetratricopeptide repeat domain 1);
(ii) a second component comprising a plasma membrane (PM)-targeting moiety, an endosomal-targeting moiety or a Golgi-targeting moiety tagged with a bioluminescent donor molecule or a fluorescent acceptor molecule;
wherein if said GASIP is tagged with said fluorescent acceptor molecule, said PM-targeting moiety, endosomal-targeting moiety or Golgi-targeting moiety is tagged with said bioluminescent donor molecule, and if said GASIP is tagged with said bioluminescent donor molecule, said cellular M-targeting moiety, endosomal-targeting moiety or Golgi-targeting moiety is tagged with said fluorescent acceptor molecule.

As defined in claim 1, the invention is directed to a system for measuring modulation of G protein activation in a Gα protein subunit family-selective manner, said system comprising a cell expressing:
(i) a first component comprising a Gα subunit interacting polypeptide (GASIP) tagged with a bioluminescent donor molecule or a fluorescent acceptor molecule;
   wherein said GASIP comprises the G protein-binding domain of:
   Rap1GAP, a Regulator of G-protein signaling (RGS) protein, P63RhoGEF, PDZRhoGEF, or P115RhoGEF;
(ii) a second component comprising a plasma membrane (PM)-targeting moiety comprising (a) a palmitoylation, myristoylation, and/or prenylation signal sequence and/or (b) a polybasic sequence, an endosomal-targeting moiety or a Golgi-targeting moiety tagged with a bioluminescent donor molecule or a fluorescent acceptor molecule;
   wherein if said GASIP is tagged with said fluorescent acceptor molecule, said PM-targeting moiety, endosomal-targeting moiety or Golgi-targeting moiety is tagged with said bioluminescent donor molecule, and if said GASIP is tagged with said bioluminescent donor molecule, said PM-targeting moiety, endosomal-targeting moiety or Golgi-targeting moiety is tagged with said fluorescent acceptor molecule;
(iii) a third component that is a cell surface receptor that signals through said G protein, wherein said receptor is a GPCR, an RTK or an integrin receptor;
and wherein there is no direct protein-protein interaction between (i) the PM-targeting moiety, endosomal-targeting moiety or Golgi-targeting moiety and (ii) the GASIP.

The bioluminescent donor molecule (or BRET donor) and the fluorescent acceptor molecule (or BRET acceptor) are selected so that the emission spectrum of the bioluminescent donor molecule overlaps with the absorbance spectrum of the fluorescent acceptor molecule. Under such conditions, the light energy delivered by the bioluminescent donor molecule is at a wavelength that is able to excite the fluorescent acceptor molecule, i.e. bioluminescence resonance energy transfer (BRET). Resonance energy transfer (abbreviated RET) is a mechanism describing energy transfer between two chromophores, having overlapping emission/absorption spectra. When the two chromophores (the "donor" and the "acceptor"), are within a short distance (*e.g.,* 10-100 Angstroms) of one another and their transition dipoles are appropriately oriented, the donor chromophore is able to transfer its excited-state energy to the acceptor chromophore through non-radiative dipole-dipole coupling. Bioluminescence Resonance Energy Transfer (BRET) is based on the non-radiative transfer of energy between a donor bioluminescent molecule (bioluminescent enzyme such as *renilla* luciferase) and an acceptor fluorescent molecule (*e.g., renilla* GFP).

As used herein, the term bioluminescent donor molecule refers to any molecule able to generate luminescence following either action on a suitable substrate, or its own excitation by an external source. There are a number of different bioluminescent donor molecules that can be employed in the present disclosure. Light-emitting systems have been known and isolated from many luminescent organisms including bacteria, protozoa, coelenterates, molluscs, fish, millipedes, flies, fungi, worms, crustaceans, and beetles, particularly click beetles of genus *Pyrophorus* and the fireflies of the genera *Photinus*, *Photuris*, and *Luciola.* Additional organisms displaying bioluminescence are listed in PCT publications No. WO 00/024878 and WO 99/049019. The bioluminescent donor molecule can be a luciferase. Examples of bioluminescent proteins with luciferase activity are disclosed in US Patents Nos. 5,229,285, 5,219,737, 5,843,746, 5,196,524, and 5,670,356. Two of the most widely used luciferases are: (i) *Renilla* luciferases and (ii) *Firefly* luciferases.

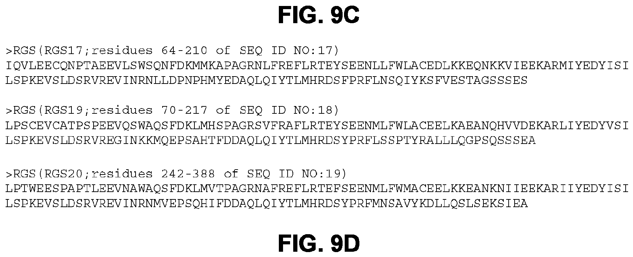

In an embodiment, in particular referred to in claim 18, the bioluminescent donor molecule is a *Renilla* luciferase (*r*Luc). The term *Renilla luciferase* as used herein refers to an oxidative enzyme used in bioluminescence and that is derived from an organism of the genus *Renilla,* such as *Renilla reniformis* or *Renilla mulleri.* It includes the native luciferase from a *Renilla* organism, or variants thereof, for example the native form (in terms of amino acid sequence) of *Renilla reniformis* luciferase (Rluc) or variants thereof such as Rlucll, Rluc3, Green Renilla Luciferase or Rluc8. The term "Rlucll" refers to a mutant form of *Renilla reniformis* luciferase that comprises the following amino acid substitutions: A55T, C124A and M185V relative to a native *Renilla* luciferase. In an embodiment, the Rlucll comprises the sequence depicted in **FIG. 9C****.** The term "Rluc8" refers to a mutant form of *Renilla reniformis* luciferase that comprises the following amino acid substitutions: A55T, C124A, S130A, K136R, A143M, M185V, M253L, and S287L relative to a native *Renilla reniformis* luciferase. The amino acid sequence of native *Renilla mulleri* luciferase is disclosed in GenBank accession No. AAG54094.1.

Natural and synthetic luminescent substrates for these enzymes are well known in the art and are commercially available. Examples of luciferase substrates include luciferin (*e.g.,* D-luciferin and salts thereof, Latia luciferin, bacterial luciferin, Dinoflagellate luciferin, etc.), coelenterazine, coelenterazine h, coelenterazine e, coelenterazine f, coelenterazine fcp, coelenterazine cp, coelenterazine hcp, coelenterazine i, coelenterazine ip, coelenterazine n, coelenterazine 400a (DeepBlueC^{™}), methoxy e-Coelenterazine (Prolume^{®} Purple I from NanoLight Technology@), Methoxy-Coelenterazine-Methoxy (Prolume^{®} Purple II from NanoLight Technology@), Methoxy-Coelenterazine-F (Prolume^{®} Purple III from NanoLight Technology@), Methoxy-Coelenterazine-lodine (Prolume^{®} Purple IV from NanoLight Technology@), Methoxy-v-Coelenterazine- Methoxy (Prolume^{®} Purple V from NanoLight Technology^{®}), ViviRen^{™} (from Promega^{®}). The suitable substrate of the bioluminescent donor molecule may be selected by the skilled person based on the desired wavelength and/or intensity of the light emitted by the bioluminescent protein. The luciferase substrate can be coelenterazine-h, methoxy e-Coelenterazine or coelenterazine 400A.

As used herein, the term fluorescent acceptor molecule refers to any compound which can accept energy emitted as a result of the activity of a bioluminescent donor molecule, and re-emit it as light energy. Representative fluorescent acceptor proteins can include, but are not limited to, green fluorescent protein (GFP), variant of green fluorescent protein (such as GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPI, pocilloporin, *Renilla* GFP (*r*GFP), Monster GFP, paGFP, Kaede protein or a Phycobiliprotein, or a biologically active variant or fragment of any one thereof. The most frequently used bioluminescent or fluorophore is the GFP from the jellyfish *Aequorea victoria* and numerous other variants (GFPs) obtained for example mutagenesis and chimeric protein technologies. GFPs are classified based on the distinctive component of their chromophores, each class having distinct excitation and emission wavelengths: class 1, wild-type mixture of neutral phenol and anionic phenolate: class 2, phenolate anion: class 3, neutral phenol: class 4, phenolate anion with stacked s-electron system: class 5, indole: class 6, imidazole: and class 7, phenyl.

Examples of non-proteinaceous fluorescent acceptor molecules are Alexa^{™} (Molecular Probes), fluor dye, Bodipy dye^{™} (Life technologies), Cy dye^{™}(Life technologies), fluorescein, dansyl, umbelliferone (7-hydroxycoumarin), fluorescent microsphere, luminescent nanocrystal, Marina blue^{™}(Life technologies), Cascade blue^{™}(Life technologies), Cascade yellow^{™}(Life technologies), Pacific blue^{™}(Life technologies), Oregon green^{™}(Life technologies), Tetramethylrhodamine, Rhodamine, Texas red^{™}(Life technologies), rare earth element chelates, or any combination or derivatives thereof.

Other representative fluorescent acceptor molecules can include, but are not limited to sgGFP, sgBFP, BFP blue-shifted GFP (Y66H), Cyan GFP, DsRed, monomeric RFP, EBFP, ECFP, GFP (S65T), GFP red-shifted (rsGFP), non-UV excitation (wtGFP), UV excitation (wtGFP), GFPuv, HcRed, rsGFP, Sapphire GFP, sgBFP^{™}, sgBFP^{™} (super glow BFP), sgGFP^{™}, sgGFP^{™} (super glow GFP), Yellow GFP, semiconductor nanoparticles (e.g., raman nanoparticles), 1,5 IAEDANS; 1,8-ANS; 4-Methylumbelliferone; 5-carboxy-2,7-dichlorofluorescein; 5-Carboxyfluorescein (5-FAM); 5-Carboxynapthofluorescein; 5 -Carboxy tetramethylrhodamine (5-TAMRA); 5-FAM (5-Carboxyfluorescein); 5-HAT (Hydroxy Tryptamine); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITS A; Aequorin (Photoprotein); AFPs (AutoFluorescent Protein; Quantum Biotechnologies); Alexa Fluor 350^{™}; Alexa Fluor 430^{™}; Alexa Fluor 488^{™}; Alexa Fluor 532^{™}; Alexa Fluor 546^{™}; Alexa Fluor 568^{™}; Alexa Fluor 594^{™}; Alexa Fluor 633^{™}; Alexa Fluor 647^{™}; Alexa Fluor 660^{™}; Alexa Fluor 680^{™}; Alizarin Complexon; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG^{™} CBQCA; ATTO-TAG^{™} FQ; Auramine; Aurophosphine G; Aurophosphine; BAO 9 (Bisaminophenyloxadiazole); BCECF (high pH); BCECF (low pH); Berberine Sulphate; Beta Lactamase; Bimane; Bisbenzamide; Bisbenzimide (Hoechst); bis-BTC; Blancophor FFG; BlancophorSV; BOBO^{™}-I ; BOBO^{™}-3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy FI; Bodipy FL ATP; Bodipy Fl-Ceramide; Bodipy R6G SE; Bodipy TMR; Bodipy TMR-X conjugate; Bodipy TMR-X, SE; Bodipy TR; Bodipy TR ATP; Bodipy TR-X SE; B0-PR0^{™}-1 ; BO-PRO^{™}-3; Brilliant Sulphoflavin FF; BTC; BTC-5N; Calcein; Calcein Blue; Calcium Crimson^{™}; Calcium Green; Calcium Green-1 Ca²⁺ Dye; Calcium Green-2 Ca²⁺; Calcium Green-5N Ca²⁺; Calcium Green-C18 Ca²⁺; Calcium Orange; Calcofluor White; Carboxy-X-rhodamine (5-ROX); Cascade Blue^{™}; Cascade Yellow; Catecholamine; CCF2 (GeneBlazer); CFDA; Chlorophyll; Chromomycin A; Chromomycin A; CL-NERF; CMFDA; Coumarin Phalloidin; C-phycocyanine; CPM Methylcoumarin; CTC; CTC Formazan; Cy2^{™}; Cy3.18; Cy3.5^{™}; Cy3^{™}; Cy5.18; Cy5.5^{™}; Cy5^{™}; Cy7^{™}; cyclic AMP Fluorosensor (FiCRhR); Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3'DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); Dichlorodihydrofluorescein Diacetate (DCFH); DiD-Lipophilic Tracer; DiD (DiIC18(5)); DIDS; Dihydorhodamine 123 (DHR); Dil (DiIC18(3)); Dinitrophenol; DiO (DiOC18(3)); DiR; DiR (DiIC18(7)); DM-NERF (high pH); DNP; Dopamine; DTAF; DY-630-NHS; DY-635-NHS; ELF 97; Eosin; Erythrosin; Erythrosin ITC; Ethidium Bromide; Ethidium homodimer-1 (EthD-1); Euchrysin; EukoLight; Europium (III) chloride; EYFP; Fast Blue; FDA; Feulgen (Pararosaniline); FIF (Formaldehyd Induced Fluorescence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; FluorX; FM 1-43^{™}; FM 4-46; Fura Red^{™} (high pH); Fura Red^{™}/Fluo-3; Fura-2; Fura-2/BCECF; Genacryl Brilliant Red B; Genacryl Brilliant Yellow 10GF; Genacryl Pink 3G; Genacryl Yellow 5GF; GeneBlazer (CCF2); Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1 ; JO-JO-1 ; JO-PRO-1 ; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensor Yellow/Blue; Mag Green; Magdala Red (Phloxin B); Mag-Fura Red; Mag-Fura-2; Mag-Fura-5; Mag-Indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant lavin E8G; Oregon Green; Oregon Green 488-X; Oregon Green^{™}; Oregon Green^{™} 488; Oregon Green^{™} 500; Oregon Green^{™} 514; Pacific Blue; Pararosaniline (Feulgen); PBFI; PE-Cy5; PE-Cy7; PerCP; PerCP-Cy5.5; PE-TexasRed [Red 613] ; Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 (Sigma); PKH67; PMIA; Pontochrome Blue Black; POPO-1; POPO-3; PO-PRO-1 ; PO-PRO-3; Primuline; Procion Yellow; Propidium lodid (PI); PyMPO; Pyrene; Pyronine; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); S65A; S65C; S65L; S65T; SBFI; Serotonin; Sevron Brilliant Red 2B; Sevron Brilliant Red 4G; Sevron Brilliant Red B; Sevron Orange; Sevron Yellow L; SITS; SITS (Primuline); SITS (Stilbene Isothiosulphonic Acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11 ; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41 ; SYTO 42; SYTO 43; SYTO 44; SYTO 45; SYTO 59; SYTO 60; SYTO 61 ; SYTO 62; SYTO 63; SYTO 64; SYTO 80; SYTO 81; SYTO 82; SYTO 83; SYTO 84; SYTO 85; SYTOX Blue; SYTOX Green; SYTOX Orange; Tetracycline; Tetramethylrhodamine (TRITC); Texas Red^{™}; Texas Red-X^{™} conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1 ; TO-PRO-3; TO-PRO-5; TOTO-1; TOTO-3; TriColor (PE-Cy5); TRITC TetramethylRodaminelsoThioCyanate; True Blue; TruRed; Ultralite; Uranine B; Uvitex SFC; WW 781; X-Rhodamine; XRITC; Xylene Orange; Y66F; Y66H; Y66W; YO-PRO-1 ; YO-PRO-3; YOYO-1; YOYO-3, SYBR Green, and Thiazole orange (interchelating dyes).

In an embodiment, in particular referred to in claim 18, the fluorescent acceptor molecule is a *Renilla* GFP (*r*GFP). The term *"Renilla* GFP" refers to a green fluorescent protein that is derived from organisms of the genus *Renilla,* such as *Renilla reniformis* or *Renilla mulleri.* It includes the native GFP from a *Renilla* organism, or variants thereof. In an embodiment, the *Renilla* GFP is a *Renilla reniformis* GFP, in a further embodiment, the native form (in terms of amino acid sequence) of *Renilla reniformis* GFP. In an embodiment, the rGFP comprises the sequence depicted in **FIG. 9D****.** The amino acid sequence of native *Renilla mulleri* GFP is disclosed in GenBank accession No. AAG54098.1. The nucleic acid sequence of the *Renilla* luciferase and/or *Renilla* GFP may be codon-optimized for expression in human cells (i.e. "humanized", see, *e.g.,* WO 2002/057451 for a humanized version of *Renilla mulleri* GFP).

Representative combinations of bioluminescent donor and fluorescent acceptor molecule suitable for BRET (referred to as BRET pairs) include luciferase (Luc)/GFP, Luc/Venus, Luc/Topaz, Luc/GFP-10, Luc/GFP-2, Luc/YFP, Luc/rGFP, and the like. One of the following BRET configurations can be used in the biosensors and methods described herein: Rlucll/coel-400a/enhanced blue (EB) FP2, Rlucll/coel-400a/super cyan fluorescent protein (SCFP3A), Rlucll/coel-400a/mAmetrine, Rlucll/coel-400a/rGFP, Rlucll/coel-400a/mAmetrine.

In the embodiment of claim 18, the bioluminescent donor molecule is a *Renilla* luciferase and the fluorescent acceptor molecule is *Renilla* GFP.

According to the invention, the second component comprises a PM-targeting moiety. According to the present disclosure, the term "plasma membrane (PM) targeting moiety" as used herein refers to any moiety capable of recruiting or sequestering the bioluminescent donor or fluorescent acceptor molecule (*e.g., Renilla* GFP or *Renilla* Luc) to the PM. The bioluminescent donor or fluorescent acceptor molecule may thus be fused to any protein found at the plasma membrane (*e.g.,* receptors or any other protein found at the PM), or fragments thereof. An example of such proteins is Caveolin-1, which the main component of the caveolae (a type of lipid raft that correspond to small (50-100 nm) invaginations of the plasma membrane) found in many cell types. Two isoforms of Caveolin-1, generated by alternative splicing of the CAV1 gene, have been identified: Caveolin-1α (comprising residues 2-178) and Caveolin-1β (corresponding to the 32-178 sequence). Other examples of such moiety include peptides/polypeptides comprising a signal sequence for protein lipidation/fatty acid acylation, such as myristoylation, palmitoylation and prenylation, as well as polybasic domains. Several proteins are known to be myristoylated, palmitoylated and/or prenylated (*e.g*., protein kinases and phosphatases such as Yes, Fyn, Lyn, Lck, Hck, Fgr, G_{α} proteins, nitric oxide synthase, ADP-ribosylation factors (ARFs), calcium binding proteins and membrane or cytoskeleton-associated structural proteins such as MARCKS (see, *e.g.,* Wright et al., J Chem Biol. Mar 2010; 3(1): 19-35; Alcart-Ramos et al., Biochimica et Biophysica Acta (BBA) - Biomembranes, Volume 1808, Issue 12, December 2011, Pages 2981-2994), and thus the myristoylation, palmitoylation and prenylation (*e.g.,* geranylgeranylation) signal sequences from any of these proteins may be used in the biosensor. In an aspect the myristoylation and/or palmitoylation sequence is from the Lyn kinase.

The PM membrane targeting moiety of present disclosure can comprise a CAAX motif (C is cysteine residue, AA are two aliphatic residues, and X represents any amino acid. CAAX motifs are found in "CAAX proteins" that are defined as a group of proteins with a specific amino acid sequence at C-terminal that directs their post translational modification. CAAX proteins encompass a wide variety of molecules that include nuclear lamins (intermediate filaments) such as prelamin A, lamin B1 and lamin B2, Ras and a multitude of GTP-binding proteins (G proteins) such as Ras, Rho, Rac, and Cdc42, several protein kinases and phosphatases, etc. (see, *e.g.,* Gao et al., Am J Transl Res. 2009; 1(3): 312-325). The proteins that have a CAAX motif or box at the end of the C-terminus typically need a prenylation process before the proteins migrate to the plasma membrane or nuclear membrane and exert different functions. The CAAX box can be derived from a human RAS family protein, for example HRAS, NRAS, Ral-A, KRAS4A or KRAS4b. The last C-terminal residues of RAS, NRAS, KRAS4A or KRAS4b (referred to as the hypervariable region or HVR) are depicted below, with the putative minimal plasma membrane targeting region in italics and the CAAX box underlined (see, *e.g.,* Ahearn et al., Nature Reviews Molecular Cell Biology 13: 39-51, January 2012): HRAS: KLNPPDESGPGCMSCK*CVLS* (SEQ ID NO: 41); NRAS: KLNSSDDGTQ*GCMGLPCVVM* (SEQ ID NO: 42); KRAS4A: KISKEEKTPG*CVKIKKCIIM* (SEQ ID NO: 43); KRAS4B: KMSKDG*KKKKKKSKTKCVIM* (SEQ ID NO: 44); Ral-A/Ral1: KNGKKKRKSLAKRIRERCCIL (SEQ ID NO: 45). As defined in claim 8, the PM targeting moiety can comprise the amino acid sequence GCMSCKCVLS (SEQ ID NO:60), GCMGLPCVVM (SEQ ID NO:61), CVKIKKCIIM (SEQ ID NO:62), KKKKKKSKTKCVIM (SEQ ID NO:63), or KNGKKKRKSLAKRIRERCCIL (SEQ ID NO: 45). Although not specifically claimed but encompassed within the scope of claim 1, the PM targeting moiety can comprise the sequence GKKKKKKSKTKCVIM (SEQ ID NO:1) from KRAS4B. In another embodiment of claim 8, the PM targeting moiety comprises the the plasma-membrane targeting palmitoylation sequence from HRAS and prenylation signal sequence from Ral-A/Ral1 (sequence: CMSCKCCIL, SEQ ID NO: 4).

Several proteins also contain a non-lipid, polybasic domain that targets the PM such as Ras small GTPases, phosphatase PTEN, nonreceptor tyrosine kinase Src, actin regulators WASP and MARCKS, and G protein-coupled receptor kinases (GRKs) such as GRK5. In an embodiment of claim 8, the polybasic domain is from GRK5, and comprises the sequence SPKKGLLQRLFKRQHQNNSKS (SEQ ID NO: 5).

According to the invention, the second component comprises an endosomal targeting moiety. According to the present disclosure, the term "endosomal targeting moiety" refers to any moiety capable of recruiting or sequestering the bioluminescent donor or fluorescent acceptor molecule to the endosomes, *e.g.,* the early endosomes. Several endosomal targeting moieties/markers are known in the art and include the Rab family of proteins (RAB4, RAB5, RAB7, RAB9 and RAB11), mannose 6-phosphate receptor (M6PR), caveolin-1 and -2, transferrin and its receptor, clathrin, as well as proteins comprising a FYVE domain such as early endosome autoantigen 1 (EEA1), Rabenosyn-5, Smad anchor for receptor activation (SARA), Vps27p and Endofin. Some markers are more specific to early endosomes (*e.g.,* RAB4, Transferrin and its receptor, and proteins comprising a FYVE domain), others are more specific to late endosomes (*e.g.,* RAB7, RAB9, and M6PR) and others are more specific to recycling endosomes (*e.g.,* RAB11, RAB4). Thus, these proteins or suitable fragments thereof may be fused to the bioluminescent donor molecule (*e.g., Renilla* Luc) or fluorescent acceptor molecule (*e.g., Renilla* GFP) to link/target them to an endosomal localization. According to claim 9, the endosomal targeting moiety is an endosomal protein or a fragment thereof that localizes to the endosomes.

According to the embodiment of claim 10, the endosomal targeting moiety comprises a FYVE domain. The FYVE domain is defined by the three conserved elements: the N-terminal WxxD, the central RR/KHHCR, and the C-terminal RVC motifs. Examples of human proteins containing a FYVE domain include ANKFY1, EEA1 FGD1, FGD2, FGD3, FGD4, FGD5, FGD6, FYCO1, HGS MTMR3, MTMR4, PIKFYVE, PLEKHF1, PLEKHF2, RUFY1, RUFY2, WDF3, WDFY1, WDFY2, WDFY3, ZFYVE1, ZFYVE16, ZFYVE19, ZFYVE20, ZFYVE21, ZFYVE26, ZFYVE27, ZFYVE28 and ZFYVE9 (EMBL-EBI, family FYVE (PF01363)). Therefore, although not claimed as such but encompassed within the scope of claim 1 in an embodiment, the endosomal targeting moiety comprises the FYVE domain of human ZFYVE16/Endofin (UniProtKB - Q7Z3T8, SEQ ID NO: 39), for example about residues 747 to 805 or about residues 739 to 806 human Endofin.

According to the invention, the second component can comprise a Golgi targeting moiety. According to the present disclosure, the term "Golgi targeting moiety" as used herein refers to any moiety capable of recruiting or sequestering the bioluminescent donor or fluorescent acceptor molecule to the Golgi apparatus. According to claim 11, the Golgi targeting moiety is a Golgi protein or a fragment thereof that localizes to the Golgi. Several Golgi targeting moieties/markers are known in the art and include eNOS (*e.g.,* the N-terminal portion thereof, J. Liu et al., Biochemistry, 35 (1996), pp. 13277-13281), GM130, Golgin-97, the 58K protein, Trans-Golgi network membrane protein 2 (TGOLN2), TGN46, TGN38, Mannosidase 2, Syntaxin 6, GM130 (GOLGA2), Golgin-160, Membrin (GS27), GS28, Coatomer proteins, Rbet1 and RCAS1. Thus, these proteins or suitable fragments thereof may be fused to *Renilla* Luc or *Renilla* GFP to link/target them to a Golgi apparatus localization. The Golgi targeting moiety can be the N-terminal portion of a human eNOS protein (SEQ ID NO:46), for example residues 1 to 73 of human eNOS1. According to claim 12, the Golgi targeting moiety comprises residues 1 to 73 of human eNOS1 (SEQ ID NO:46).

According to the invention, there is no direct protein-protein interaction between (i) the PM-targeting moiety, endosomal-targeting moiety or Golgi-targeting moiety and (ii) the GASIP.

Although not specifically claimed but encompassed within the scope of claim 1, the bioluminescent donor or fluorescent acceptor molecule may be fused N-terminal, within or C-terminal relative to the targeting moiety. The PM targeting moiety can be fused to the C-terminal end of said bioluminescent donor or fluorescent acceptor molecule. The PM targeting moiety can be fused to the fluorescent acceptor molecule, preferably to the C-terminal end of the fluorescent acceptor molecule. The endosomal targeting moiety can be fused to the C-terminal end of said bioluminescent donor or fluorescent acceptor molecule, or to the C-terminal end of said fluorescent acceptor molecule.

Although not specifically claimed but encompassed within the scope of claim 1, the bioluminescent donor or fluorescent acceptor molecule may be fused N-terminal, within, or C-terminal relative to the GASIP. The bioluminescent donor or fluorescent acceptor molecule can be fused to the N-terminal end of the GASIP. The bioluminescent donor or fluorescent acceptor molecule can be fused to the C-terminal end of the GASIP. The bioluminescent donor molecule can be fused to the GASIP, especially to the C-terminal end of the GASIP.

In present description, the term "G protein-binding domain of Rap1GAP" refers to a polypeptide comprising the domain of the Rap1 GTPase-activating protein 1 (Rap1GAP) protein (UniProt accession No. P47736, SEQ ID NO:47), or a variant thereof, that has the ability to bind to Gα subunit protein(s) of the Gi family. The G protein-binding domain of Rap1GAP is located in the N-terminal portion of Rap1GAP, and for example comprises at least 50, 100, 150, 200, 250, 300, 350 or 400 residues from native Rap1GAP. The G protein-binding domain of Rap1GAP may comprise one or more mutations that do not abrogate the binding to the Gα subunit protein. According to claim 1, the GASIP can comprise the G protein-binding domain of Rap1GAP. According to the embodiment of claim 2, the GASIP is the G protein-binding domain of Rap1GAP that comprises residues 1 to 420 or 1 to 436 of native Rap1GAP. According to the disclosure and not specifically claimed the GASIP can be a variant of said residues 1 to 420 or 1 to 436 of Rap1GAP, to the proviso that the variant retains the ability to bind to Gα subunit protein(s) of the Gi family. Although not specifically claimed but encompassed within the scope of claim 1, the G protein-binding domain of Rap1GAP can comprises residues 1 to 442, or a variant thereof that retains the ability to bind to Gα subunit protein(s) of the Gi family. The G protein-binding domain of Rap1GAP can be fused to the N-terminal end of the bioluminescent donor molecule.

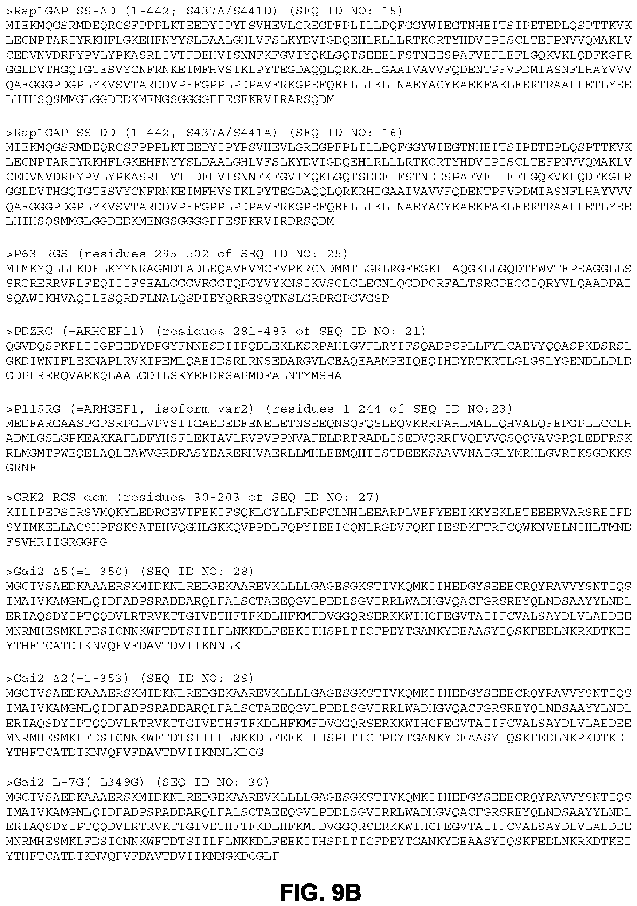

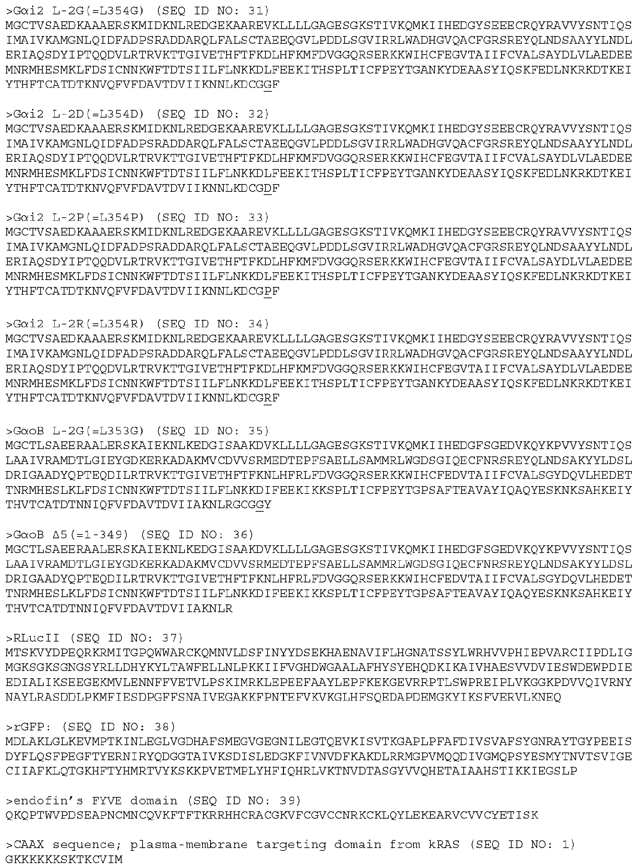

Although not specifically claimed but encompassed within the scope of claim 1, the G protein-binding domain of Rap1GAP can comprise a mutation that reduces its sensitivity to downstream signalling events such as kinase activation (*e.g.,* protein kinase A). Such reduction in sensitivity to downstream signalling events such as kinase activation may be achieved, for example, by introduction of mutation(s) (*e.g.,* deletion, substitution) of one or more of the putative phosphorylation sites, for example the serine residues at position 431, 437, 439 and/or 441 of native Rap1GAP. One or more of the serine residues at positions 437, 439 and/or 441 can be mutated in the G protein-binding domain of Rap1GAP, preferably at least two of the serine residues at positions 437, 439 and/or 441 are mutated in the G protein-binding domain of Rap1GAP, and more preferably all three serine residues at positions 437, 439 and 441 are mutated. The mutation can be a substitution, for example by an amino acid that cannot be phosphorylated, for example an alanine residue. All three serine residues at positions 437, 439 and 441 can be substituted by an alanine in the G protein-binding domain of Rap1GAP. The G protein-binding domain of Rap1GAP can comprise one of the sequences depicted in **FIG. 9A** and **FIG. 9B**.

The term "G protein-binding domain of a Regulator of G-protein signaling (RGS) protein" as used herein refers to a polypeptide comprising the domain of an RGS protein, or a variant thereof, that has the ability to bind to Gα subunit protein(s) of the Gi family. RGS proteins are a family of 22 proteins (RGS1-RGS22) comprising an RGS-box or RGS domain (PROSITE entry PS50132). The G protein-binding domain of the RGS protein may comprise at least 50, 100, 150, 200, 250, 300, 350 or 400 residues from an RGS protein, or a variant thereof retaining the ability to bind to Gα subunit protein(s) of the Gi family. The GASIP can comprise the G protein-binding domain of an RGS protein of the RZ/A subfamily, preferably RGS17 (RGSZ2, UniProt accession No. Q9UGC6, SEQ ID NO:17), RGS19 (GAIP, UniProt accession No. P49795, SEQ ID NO:18) or RGS20 (RGSZ1, UniProt accession No. 076081, SEQ ID NO:19), or a variant thereof that has the ability to bind to Gα subunit protein(s) of the Gi family. The G protein-binding domain of RGS17 can comprises residues 84-200 of native RGS17 or a variant thereof that has the ability to bind to Gα subunit protein(s) of the Gi family. The G protein-binding domain of RGS17 can comprise residues 64-210 of native RGS17 or a variant thereof that has the ability to bind to Gα subunit protein(s) of the Gi family. The G protein-binding domain of RGS19 can comprise residues 90-206 of native RGS19 or a variant thereof that has the ability to bind to Gα subunit protein(s) of the Gi family. The G protein-binding domain of RGS19 can comprise residues 70-217 of native RGS19 or a variant thereof that has the ability to bind to Gα subunit protein(s) of the Gi family. The G protein-binding domain of RGS20 can comprise residues 262-378 of native RGS20 or a variant thereof that has the ability to bind to Gα subunit protein(s) of the Gi family. The G protein-binding domain of RGS20 can comprise residues 242-388 of native RGS20 or a variant thereof that has the ability to bind to Gα subunit protein(s) of the Gi family. The G protein-binding domain of a RGS protein can be fused to the N-terminal end of the bioluminescent donor molecule. In the claims, the GASIP comprises the G protein-binding domain of RGS17, RGS19 or RGS20 according to claim 3 and said G protein-binding domain comprises residues 64 to 210 of RGS17 (SEQ ID NO:17), residues 70-217 of RGS19 (SEQ ID NO:18), or residues 242-388 of RGS20 (SEQ ID NO:19) according to claim 4.

Although not claimed and for illustration only, another protein comprising a domain that binds Gα subunit protein(s) of the Gi family is tumor necrosis factor-alpha (TNFα)-induced protein 8 (TNFAIP8, UniProtKB accession No. O95379, SEQ ID NO: 48).

The term "G protein-binding domain of P63RhoGEF" refers to a polypeptide comprising the domain of the P63RhoGEF (Rho guanine nucleotide exchange factor 25) protein (UniProt accession No. Q86VW2, SEQ ID NO:25), or a variant thereof, that has the ability to bind to Gα subunit protein(s) of the Gq family. The G protein-binding domain of P63RhoGEF is located in the C-terminal portion of P63RhoGEF, and for example comprises at least 50, 100, 150, 200, 250, 300, 350 or 400 residues from native P63RhoGEF, or a variant thereof that retains the ability to bind to Gα subunit protein(s) of the Gq family. The G protein-binding domain of P63RhoGEF may comprise one or more mutations that do not abrogate the binding to the Gα subunit protein. Although not specifically claimed but encompassed within claim 1, the G protein-binding domain of P63RhoGEF can comprise residues 348 to 466 of native P63RhoGEF, or a variant thereof that retains the ability to bind to Gα subunit protein(s) of the Gq family. In the embodiment of claim 5, the G protein-binding domain of P63RhoGEF comprises residues 295 to 502 of native P63RhoGEF. According to present disclosure, it can encompass a variant of the G protein-binding domain of P63RhoGEF which comprises residues 295 to 502 of native P63RhoGE, where the variant retains the ability to bind to Gα subunit protein(s) of the Gq family. The G protein-binding domain of P63RhoGEF can be fused to the N-terminal end of the bioluminescent donor molecule.

The term "G protein-binding domain of GRK2" refers to a polypeptide comprising the domain of the GRK2 (Beta-adrenergic receptor kinase 1) protein (UniProt accession No. P25098, SEQ ID NO: 27), or a variant thereof that has the ability to bind to Gα subunit protein(s) of the Gq family. The G protein-binding domain of GRK2 is located in the N-terminal portion of GRK2, and for example comprises at least 50, 100, 150, 200, 250, 300, 350 or 400 residues from native GRK2, or a variant thereof that retains the ability to bind to Gα subunit protein(s) of the Gq family. The G protein-binding domain of GRK2 may comprise one or more mutations that do not abrogate the binding to the Gα subunit protein. The G protein-binding domain of GRK2 may comprise residues 54 to 175 of native GRK2, or a variant thereof that retains the ability to bind to Gα subunit protein(s) of the Gq family. The G protein-binding domain of GRK2 may comprise residues 30 to 203 of native GRK2, or a variant thereof that retains the ability to bind to Gα subunit protein(s) of the Gq family. The G protein-binding domain of GRK2 can be fused to the C-terminal end of the bioluminescent donor molecule.

Other proteins comprising a domain that binds Gα subunit protein(s) of the Gq family that may be used according to present disclosure include GRK3 (*e.g.,* residues ~54-175), PLCβ proteins, RGS proteins (*e.g.,* RGS2, residues ~83-199), TRP1 and other RhoGEF proteins (*e.g.,* Triple functional domain protein, TRIO). Polypeptides comprising these domains or variants thereof that retains the ability to bind to Gα subunit protein(s) of the Gq family as defined above may be used in the systems/methods described herein.

The term "G protein-binding domain of PDZRhoGEF" refers to a polypeptide comprising the domain of the PDZRhoGEF (Rho guanine nucleotide exchange factor 11) protein (UniProt accession No. 015085, SEQ ID NO:21), or a variant thereof that has the ability to bind to Gα subunit protein(s) of the G12/13 family. The G protein-binding domain of PDZRhoGEF is located in the central portion of PDZRhoGEF, and for example comprises at least 50, 100, 150, 200, 250, 300, 350 or 400 residues from native PDZRhoGEF, or a variant thereof that retains the ability to bind to Gα subunit protein(s) of the G12/13 family. The G protein-binding domain of PDZRhoGEF may comprise one or more mutations that do not abrogate the binding to the Gα subunit protein. The G protein-binding domain of PDZRhoGEF can comprise residues 306 to 486 of native PDZRhoGEF, or a variant thereof that retains the ability to bind to Gα subunit protein(s) of the G12/13 family. In the embodiment of claim 6, the G protein-binding domain of PDZRhoGEF comprises residues 281 to 483 of native PDZRhoGEF. According to present disclosure, it can encompass a variant of the G protein-binding domain of PDZRhoGEF which comprises residues 281 to 483 to the proviso that the variant retains the ability to bind to Gα subunit protein(s) of the G12/13 family. The G protein-binding domain of PDZRhoGEF can be fused to the N-terminal end of the bioluminescent donor molecule.

The term "G protein-binding domain of P115RhoGEF" refers to a polypeptide comprising the domain of the P115RhoGEF (Rho guanine nucleotide exchange factor 1) protein (UniProt accession No. Q92888, SEQ ID NO:23), or a variant thereof that has the ability to bind to Gα subunit protein(s) of the G12/13 family. The G protein-binding domain of P115RhoGEF is located in the N-terminal portion of P115RhoGEF, and for example comprises at least 50, 100, 150, 200, 250, 300, 350 or 400 residues from native P115RhoGEF, or a variant thereof that retains the ability to bind to Gα subunit protein(s) of the G12/13 family. The G protein-binding domain of P115RhoGEF may comprise one or more mutations that do not abrogate the binding to the Gα subunit protein. In an embodiment, the G protein-binding domain of P115RhoGEF comprises residues 41 to 232 of native P115RhoGEF, or a variant thereof that retains the ability to bind to Gα subunit protein(s) of the G12/13 family. In the embodiment of claim 6, the G protein-binding domain of P115RhoGEF comprises residues 1 to 244 of native P115RhoGEF, or a variant thereof that retains the ability to bind to Gα subunit protein(s) of the G12/13 family. The G protein-binding domain of P115RhoGEF can be fused to the N-terminal end of the bioluminescent donor molecule.

Other proteins comprising a domain that binds Gα subunit protein(s) of the G12/13 family that may be used in the systems/methods described herein include RhoGEFs such as Rho guanine nucleotide exchange factor 12 (LARG, UniProtKB accession No. Q9NZN5, SEQ ID NO:49) (*e.g.,* residues -367-558), JNK-Interacting Leucine Zipper Protein (JLP, the C-terminal portion) cadherin proteins, AXIN1 (UniProtKB accession No. 015169, SEQ ID NO: 50, residues ~88-212, more selective for G12), PP2A (more selective for G12), Alpha-soluble NSF attachment protein (alphaSNAP, UniProtKB accession No. P54920, SEQ ID NO: 51, N-terminal portion, more selective for G12), polycistin-1 (UniProtKB accession No. P98161, SEQ ID NO: 52, more selective for G12), RGS16 (UniProtKB accession No. 015492, SEQ ID NO: 53, N-terminal portion, more selective for G13), AKAP110 (UniProtKB accession No. O75969, SEQ ID NO: 54, C-terminal portion, more selective for G13), HS1-associated protein X1 (HAX1, UniProtKB accession No. 000165, SEQ ID NO: 55, residues -176-247, more selective for G13). Polypeptides comprising these domains or variants thereof that retains the ability to bind to Gα subunit protein(s) of the G12/13 family as defined above may be used in the systems/methods described herein. Using domains more selective for one of the subunits (G12 or G13) may be useful for assessing the selective activation of the endogenous G12 or G13 proteins.

Domain that binds Gα subunit protein(s) of the Gs family includes SNX13 (RGS-PX1, UniProtKB accession No. Q9Y5W8, SEQ ID NO: 56, residues -373-496), AXIN1 (UniProtKB accession No. 015169, SEQ ID NO: 50, residues ~88-211) or TPR1 (tetratricopeptide repeat domain 1, UniProtKB accession No. Q99614, SEQ ID NO: 57, C-terminal portion). The Gα subunit protein of the Gs family can be the membrane-anchored XL(alpha)s subunit.

The length of the GASIP can be about 50, 75 or 100 amino acids to about 500, 600, 700 or 800 amino acids, for example about 100 or 150 amino acids to about 200, 250, 300, 350, 400 or 500 amino acids.

It is possible that the GASIP does not comprise the full-length sequences of Rap1GAP, the RGS protein (*e.g.,* RGS17, 19 or 20), P63RhoGEF, GRK2, PDZRhoGEF or P115RhoGEF, i.e., it comprises one or more mutations (substitutions, deletions, etc.) relative to the full-length proteins.

In the embodiment of claim 15, the system further comprises a fourth a recombinant Gα protein subunit. Gα protein subunit as defined herein includes, but is not limited to, the 17 different known isoforms, their splice variants, and any mutated Gα proteins, for example those leading to non-selective/promiscuous Gα. The herein described Gα proteins can be selected amongst any of the natural mammalian Gα proteins, which includes Gq, Gs, Gi1, Gi2, Gi3, Gt-cone, Gt-rod, Gt-gust, Gz, GoA, GoB, Golf, G11, G12, G13, G14, and G15/G16 (also designated GNA15). These isoforms have the splice variants thereof, as well as functional variants thereof. A recombinant Gα protein subunit can be of the Gi family, *e.g.,* Gi1, Gi2, Gi3, GoA, GoB, Gt-cone, Gt-rod, Ggus, and/or Gz. A Gα protein subunit can be of the G_{q} family, *e.g.,* Gq, G11, G14 and/or G15/16). A Gα protein subunit can be of the G12/13 family.

The recombinant Gα subunit polypeptide can comprise at least one mutation that decreases or abrogates the activation by GPCRs. Such mutated Gα subunit polypeptide may be useful for assessing non-receptor guanine nucleotide exchange factor (GEF)-mediated G protein activation, i.e. G protein activation not induced through GPCR engagement. According to claim 16 the recombinant Gα subunit polypeptide comprises at least one mutation in the carboxy (C)-terminal domain of said Gα subunit polypeptide, wherein said C-terminal domain corresponds to the last seven residues of said Gα subunit polypeptide. Accordingly the mutation may be in the carboxy (C)-terminal domain, and preferably is a mutation in one or more of the last seven residues at the C-terminal of said Gα subunit polypeptide. The mutation may be a truncation of the last one, 2, 3, 4, 5, 6 or 7 residues at the C-terminal. The mutation may be a substitution of at least one, 2, 3, 4, 5, 6 or all residues at the C-terminal. The mutation may be a deletion or substitution of at least one of the conserved leucine residues in said C-terminal domain, preferably a deletion or substitution of the last conserved leucine residue (penultimate residue of the native protein) in said C-terminal domain. The mutation may be a substitution of the last conserved leucine residue, preferably a substitution for an aspartic acid (D), a proline (P) or an arginine (R) residue. The mutated recombinant Gα subunit polypeptide may be of the Gi family, for example Gi2 or GoB. The mutated recombinant Gα subunit polypeptide may comprise one of the sequences depicted in **FIGs. 9B** and **9C**, *e.g.,* Gαi2 Δ5 (SEQ ID NO: 28); Gαi2 Δ2 (SEQ ID NO: 29); Gαi2 L-2G (SEQ ID NO: 31); Gαi2 L-2P (SEQ ID NO: 33); Gαi2 L-2R (SEQ ID NO: 34); Gαi2 L-2D (SEQ ID NO: 32); Gαi2 L-7G (SEQ ID NO: 30); GαoB Δ5 (SEQ ID NO: 36); or GαoB L-2G (SEQ ID NO: 35).

The term "recombinant" as used herein refers to a protein molecule which is expressed from a recombinant nucleic acid molecule, i.e. a nucleic acid prepared by means of molecular biology/genetic engineering techniques, for example a protein that is expressed following transfection/transduction of a cell (or its progeny) with a nucleic acid (*e.g.,* present in a vector) encoding the protein (as opposed to a protein that is naturally expressed by a cell).

According to the invention, the system further comprises a cell surface receptor as a third component. The cell of the biosensor may naturally express the cell surface receptor, or the cell surface receptor may be a recombinant cell surface receptor (*e.g.,* the cell has been transfected or transformed with a nucleic acid encoding the cell surface receptor). The term "cell surface receptor" as disclosed herein refers to a protein attached to or embedded with the plasma membrane and that induces G protein activation upon binding of a ligand. Examples of cell surface receptors that induces G protein activation include G protein-coupled receptors (GPCRs), receptor tyrosine kinases (RTKs), integrins. Whereas G protein activation typically occurs through GPCRs engagement by a ligand, G protein activation may also be achieved via non-receptor guanine nucleotide exchange factors (GEF) such as GIV (Gα-interacting vesicle-associated protein, also known as Girdin), NUCB1 (nucleobindin1, also known as calnuc), NUCB2 and DAPLE (Dishevelled-associating protein). For example, GIV activity is associated with RTKs (*e.g.,* EGFR) and integrin α-β complex modulation of Gi activity. According to claim 1, the cell surface receptor is a cell surface receptor that signals through said G protein, and is a GPCR, an RTK or an integrin receptor. In a particular embodiment where the GASIP comprises the G protein-binding domain of Rap1GAP, the cell surface receptor is a GPCR or a RTK. In another embodiment where the GASIP comprises the G protein-binding domain of a RGS protein, preferably RGS17, the cell surface receptor is a GPCR.

"GPCR" refers to full-length native GPCR molecules as well as mutant GPCR molecules. A list of GPCRs is given in Foord et al. (2005) Pharmacol Rev. 57, 279-288, and an updated list of GPCRs is available in the IUPHAR-DB database (Harmar AJ, et al. (2009) IUPHAR-DB: the IUPHAR database of G protein-coupled receptors and ion channels. Nucl. Acids Res. 37 (Database issue): D680-D685; Sharman JL, et al., (2013) IUPHAR-DB: updated database content and new features. Nucl. Acids Res. 41 (Database Issue): D1083-8). According to claim 14, the cell surface receptor is a GPCR.

"RTK" refers to full-length native RTK proteins as well as mutant RTK proteins. RTKs (EC 2.7.10.1 according to the IUBMB Enzyme Nomenclature) are cell surface receptors for many polypeptide growth factors, cytokines, and hormones, characterized by an intracellular region comprising catalytic domains responsible for the kinase activity of these receptors, which catalyses receptor autophosphorylation and tyrosine phosphorylation of RTK substrates. There are 58 known RTK in humans, distributed into 20 subfamilies (Robinson et al., Oncogene 2000, 19(49):5548-57).

"Integrin" refers to full-length native integrin proteins as well as mutant integrin proteins. Integrins are composed of two noncovalently associated transmembrane glycoprotein subunits called α and β. A variety of human integrin heterodimers are formed from 9 types of β subunits and 24 types of α subunits. Representative integrins found in vertebrates include α₁β₁, α₂β₁, α₃β₁, α₄β₁, α₅β₁, α₆β₁, α₇β₁, α_{L}β₂, α_{M}β₂, α_{IIb}β₃, α_{V}β₁, α_{V}β₃, α_{V}β₅, α_{V}β₆, αᵥβ₈, and α₆β₄.

The term "variant" (or "mutant") as used herein refers to a protein/polypeptide having has a sequence identity of at least 60% with a reference (*e.g.,* native) sequence and retains a desired activity thereof, for example the capacity to bind to Gα subunit or to act as a BRET donor or acceptor. The variant can have a similarity or identity of at least 65, 70, 75, 80, 85, 90, 91, 92, 93, 94, 95, 96, 97, 98 or 99% with a reference (*e.g.,* native) sequence and retains a desired activity thereof. "Similarity" and "identity" refers to sequence similarity/identity between two polypeptide molecules. The similarity or identity can be determined by comparing each position in the aligned sequences. A degree of similarity or identity between amino acid sequences is a function of the number of matching or identical amino acids at positions shared by the sequences. Optimal alignment of sequences for comparisons of similarity or identity may be conducted using a variety of algorithms, such as the local homology algorithm of Smith and Waterman, 1981, Adv. Appl. Math 2: 482, the homology alignment algorithm of Needleman and Wunsch, 1970, J. Mol. Biol. 48: 443, the search for similarity method of Pearson and Lipman, 1988, Proc. Natl. Acad. Sci. USA 85: 2444, and the computerized implementations of these algorithms (such as GAP, BESTFIT, FASTA and TFASTA in the Wisconsin Genetics Software Package, Genetics Computer Group, Madison, Wis., U.S.A.). Sequence similarity or identity may also be determined using the BLAST algorithm, described in Altschul et al., 1990, J. Mol. Biol. 215: 403-10 (using the published default settings). Software for performing BLAST analysis may be available through the National Center for Biotechnology Information web site.

The domains of the components (fusion molecules) described herein may be covalently linked either directly (*e.g.,* through a peptide bond) or "indirectly" via a suitable linker moiety, *e.g.,* a linker of one or more amino acids or another type of chemical linker (*e.g*., a carbohydrate linker, a lipid linker, a fatty acid linker, a polyether linker, PEG, etc. One or more additional domain(s) may be inserted before (N-terminal), between or after (C-terminal) the domains defined above. The domains of the fusion molecules may be covalently linked through a peptide bond. One or more of the components of the fusion molecules may be linked through a peptide linker. The linkers can be peptide linkers, typically ranging from 2 to 30 amino acids in length, for example about 5 to about 20-25 amino acids or about 10 to about 15-20 amino acids. The composition and length of each of the linkers may be chosen depending on various properties desired such as flexibility and aqueous solubility. For instance, the peptide linker may comprise relatively small amino acid residues, including, but not limited to, glycine; small amino acid residues may reduce the steric bulk and increase the flexibility of the peptide linker. The peptide linker may also comprise polar amino acids, including, but not limited to, serine. Polar amino acid residues may increase the aqueous solubility of the peptide linker. Furthermore, programs such as Globplot 2.3 (binding *et al.,* GlobPlot: exploring protein sequences for globularity and disorder, Nucleic Acid Res 2003 - Vol. 31, No.13, 3701-8), may be used to help determine the degree of disorder and globularity, thus also their degree of flexibility. The peptide linker may comprise one or more of the amino acid sequences disclosed in the Examples below and/or the figures (SEQ ID NOs:6, 7, 20, 22, 24 and 26).

According to the invention, the system comprises a cell expressing the various components defined in the systems as claimed, e.g. the first, second and third (cell surface receptor) components, and optionally the recombinant Gα protein subunit.

Although not claimed, the present disclosure provides for a nucleic acid or a plurality of nucleic acids encoding the above-defined first and/or second component(s) defined herein. Such nucleic acid(s) may be present in a vector/plasmid (or a plurality of vectors/plasmids), in particular expression vector(s)/plasmid(s). Such vectors comprise nucleic acid(s) encoding the above-defined first and/or second component(s) operably linked to one or more transcriptional regulatory sequence(s), such as promoters, enhancers and/or other regulatory sequences. The nucleic acid can encode the first and second components (polycistronic construct).

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". A recombinant expression vector described herein can be constructed by standard techniques known to one of ordinary skill in the art and found, for example, in Sambrook *et al., supra.* A variety of strategies are available for ligating fragments of DNA, the choice of which depends on the nature of the termini of the DNA fragments and can be readily determined by persons skilled in the art. The vectors described herein may also contain other sequence elements to facilitate vector propagation and selection in bacteria and host cells. In addition, the vectors described herein may comprise a sequence of nucleotides for one or more restriction endonuclease sites. Coding sequences such as for selectable markers and reporter genes are well known to persons skilled in the art.

A recombinant expression vector comprising one or more of the nucleic acids defined herein may be introduced into a cell (a host cell), which may include a living cell capable of expressing the protein coding region from the defined recombinant expression vector. The living cell may include both a cultured cell and a cell within a living organism. Accordingly, the description also provides host cells containing the recombinant expression vectors described herein. Claim 19 refers to a host cell of the invention. The terms "cell", "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into cells via conventional transformation or transfection techniques. The terms "transformation" and "transfection" refer to techniques for introducing foreign nucleic acid into a host cell, including calcium phosphate or calcium chloride coprecipitation, DEAE-dextran-mediated transfection, lipofection, electroporation, microinjection and viral-mediated transfection. Suitable methods for transforming or transfecting host cells can for example be found in Sambrook et al. (Molecular Cloning: A Laboratory Manual, 2nd Edition, Cold Spring Harbor Laboratory press (1989)), and other laboratory manuals. "Transcriptional regulatory sequence/element" is a generic term that refers to DNA sequences, such as initiation and termination signals, enhancers, and promoters, splicing signals, polyadenylation signals which induce or control transcription of protein coding sequences with which they are operably linked. A first nucleic acid sequence is "operably-linked" with a second nucleic acid sequence when the first nucleic acid sequence is placed in a functional relationship with the second nucleic acid sequence. For instance, a promoter is operably-linked to a coding sequence if the promoter affects the transcription or expression of the coding sequences. Generally, operably-linked DNA sequences are contiguous and, where necessary to join two protein coding regions, in reading frame. However, since for example, enhancers generally function when separated from the promoters by several kilobases and intronic sequences may be of variable lengths, some polynucleotide elements may be operably-linked but not contiguous.

In another aspect, the present disclosure describes a kit (not claimed) comprising a first nucleic acid encoding the first component and a second nucleic acid encoding the second component, or a nucleic acid encoding the first and second components. Such a kit can further comprise a nucleic acid encoding a Gα protein subunit. Such a the kit can further comprise a nucleic acid encoding a cell surface receptor.

In another aspect, the present disclosure provides a cell comprising or expressing the above-defined first and/or second component(s). In an aspect the cell has been transfected or transformed with a nucleic acid encoding the above-defined first and/or second component(s). In an aspect, the cell further comprises or expresses a recombinant Gα protein subunit. In an aspect, the cell further comprises or expresses a recombinant cell surface receptor, *e.g.,* a GPCR, an RTK or an integrin.

The present disclosure further describes a recombinant expression system, vectors and cells, such as those described above, for the expression of the first and/or second component(s) described herein, using for example culture media and reagents well known in the art. The cell may be any cell capable of expressing the first and second component(s) defined above. Suitable host cells and methods for expression of proteins are well known in the art. Any cell capable of expressing the component(s) defined above may be used. For example, eukaryotic host cells such as mammalian cells may be used (*e.g.,* rodent cells such as mouse, rat and hamster cell lines, human cells/cell lines). The above-mentioned cell may be a human cell line, for example an embryonic kidney cell line (*e.g.,* HEK293 or HEK293T cells).

In another aspect, the present disclosure provides a method for determining whether an agent modulates the activation of a G protein of interest, said method comprising:
contacting the system defined herein with a substrate for the bioluminescent donor molecule;
measuring the BRET signal in the system in the presence and absence of said agent;
wherein a difference in said BRET signal in the presence of said agent relative to the absence thereof is indicative that said agent modulates the activation of said G protein of interest.

The invention relates to a method for determining whether an agent modulates the activation of a G protein of interest as defined in claim 20.

The agent can be an agonist and the difference in said BRET signal is an increase.

The G protein of interest can be of the Gi protein subunit family, and the GASIP comprises the G protein-binding domain of Rap1GAP or of an RGS protein as defined herein. The system used in the above-mentioned method can comprise a recombinant Gα subunit polypeptide of the Gi protein subunit family, i.e. a recombinant Gi1, Gi2, Gi3, GoA, GoB, or Gz subunit polypeptide. To identify the profile or signature of a given test agent (i.e. to identity the specific Gi protein subunit(s) activated by the test agent), the above-mentioned method may be performed using a plurality (i.e. two or more) of systems, each system comprising a different Gi protein subunit, *e.g.,* a first system comprising a recombinant Gi1, a second system comprising a recombinant Gi2, a third system comprising a recombinant Gi3, etc.

The G protein of interest can be of the Gq protein subunit family, and the GASIP comprises the G protein-binding domain of P63RhoGEF or GRK2 as defined herein. The system used in the above-mentioned method can comprise a recombinant Gα subunit polypeptide of the Gq protein subunit family, i.e. a recombinant Gq, G11, G14 or G15 subunit polypeptide. To identify the profile or signature of a given test agent (i.e. to identity the specific Gq protein subunit(s) activated by the test agent), the above-mentioned method may be performed using a plurality (i.e. two or more) of systems, each system comprising a different Gq protein subunit, *e.g.,* a first system comprising a recombinant Gq, a second system comprising a recombinant G11, a third system comprising a recombinant G14, etc.

The G protein of interest can be of the G12/13 protein subunit family, and the GASIP comprises the G protein-binding domain of PDZRhoGEF or P115RhoGEF as defined herein. The system used in the above-mentioned method can comprise a recombinant Gα subunit polypeptide of the G12/13 protein subunit family, i.e. a recombinant Gq, G12 or G13 subunit polypeptide. To identify the profile or signature of a given test agent (i.e. to identity the specific G12/13 protein subunit(s) activated by the test agent), the above-mentioned method may be performed using a plurality (i.e. two or more) of systems, each system comprising a different G12/13 protein subunit, *e.g.,* a first system comprising a recombinant G12 and a second system comprising a recombinant G13.

In another aspect, the present disclosure provides a method for determining whether an agent modulates non-receptor guanine nucleotide exchange factor (GEF)-mediated G protein activation, said method comprising:
contacting the system defined herein with a substrate for the bioluminescent donor molecule;
measuring the BRET signal in the system in the presence and absence of said agent, wherein said system comprises a recombinant Gα subunit polypeptide comprising at least one mutation that decreases or abrogates the activation by GPCRs;
wherein a difference in said BRET signal in the presence of said agent relative to the absence thereof is indicative that said agent modulates non-receptor GEF-mediated G protein activation.

The invention relates to a method for determining for determining whether an agent modulates non-receptor guanine nucleotide exchange factor (GEF)-mediated G protein activation as defined in claim 21.

The agent can be an agonist and the difference in said BRET signal is an increase (or higher BRET signal).

The agent can be an antagonist and the difference in said BRET signal is a decrease (or lower BRET signal). To identify whether an agent is an antagonist, the system can be contacted with a known agonist, and a decrease in the BRET signal induced by the known agonist in the presence of the agent is indicative that the agent is an antagonist.

In another aspect, the present disclosure provides a method for determining whether an agent induces GPCR-mediated Gprotein activation or RTK/GEF-mediated Gprotein activation, the method comprising:
measuring the BRET signal in the presence of the agent using a first biosensor in which the GASIP comprises the G protein-binding domain of Rap1GAP, preferably a G protein-binding domain of Rap1GAP that comprises a mutation that reduces its sensitivity to downstream signalling events (*e.g.,* kinase activation), more preferably comprising a mutation at one or more of the serine residues at positions 437, 439 and/or 441 (*e.g.,* Rap1GAP(SSS-AAA)), as defined above;
measuring the BRET signal in the presence of the agent using a second biosensor in which the GASIP comprises the G protein-binding domain of an RGS protein, preferably RGS17, as defined above;
wherein an increase (*e.g.,* dose-dependent increase) in the BRET signal in the presence of the agent in the first biosensor without an increase in the BRET signal in the presence of the agent in the second biosensor is indicative that the agent induces RTK/GEF-mediated Gprotein activation; and wherein an increase (*e.g.,* dose-dependent increase) in the BRET signal in the presence of the agent in the first and second biosensors is indicative that the agent induces GPCR-mediated Gprotein activation.

The term "compound", "agent", "test compound" or "test agent" refers to any molecule (*e.g.,* drug candidates) that may be screened by the system/assay described herein may be obtained from any number of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means.

The "increased signal" or "higher signal" as used herein refers to signal that is at least 10, 20, 30, 40, 45 or 50% higher relative to the reference signal measured in the absence of the test agent. The "higher signal" can be determined by showing a statistically significant difference (determined using a suitable statistical analysis) in the signal measured in the presence relative to the absence of the test agent, for example by combining the results obtained in a plurality of samples. Statistical analysis (ANOVA, Student *t*-test, Chi square, etc.) to determine significant differences between different sets of data are known in the art, and such analysis may be performed using suitable computer programs.

Positive controls and negative controls may be used in the methods/assays described herein. Control and test samples may be performed multiple times to obtain statistically significant results.

The above-mentioned methods can be high-throughput methods (high-throughput screening, HTS). The term "high-throughput screening" (HTS) as used herein refers to a method that allow screening rapidly and in parallel large numbers of compounds (hundreds, thousands) for binding activity or biological activity against target molecules. Such HTS methods are typically performed in microtiter plates having several wells, for example 384, 1536, or 3456 wells. For HTS, it is important that the readout signal be detected with high sensitivity, accuracy and reproducibility. One way to determine whether a method is suitable or compatible with HTS is by measurement of the Z-factor, as described in the examples below. Ideally, the Z-factor should be at least 0.5 (i.e. between 0.5 and 1), and preferably at least 0.55, 0.6, 0.65, 0.7, 0.75 or 0.8.

Methods and devices to measure the BRET signal are well known in the art. The BRET signal may be measured, for example, by determining the intensity of the BRET acceptor signal (light intensity), and/or by calculating the ratio of the signal or light intensity emitted by the BRET acceptor over the signal or light intensity emitted by the BRET donor (BRET ratio). The BRET signal may be measured using a microplate reader or microscope with a suitable filter set for detecting the BRET donor and/or BRET acceptor light emissions.

The systems and methods described herein may be used in HTS to identify, for example, compounds that are active in modulating G protein activation (through GPCRs or other receptors signaling through G proteins) and are thus potential drug candidates or for use in treating disorders in which G protein signaling is involved, *e.g.,* for which G protein inhibition or stimulation would be beneficial. The systems and methods described herein may also be used be used to perform ligand profiling, *e.g.,* to determine the pathways modulated by ligands of GPCRs or other receptors signaling through G proteins.

The systems and methods described herein may be used for the screening of compound libraries to identify potential candidates for drug development. The change in BRET signal ratio when the system described herein is contacted with a compound may identify drugs or pharmaceutically active compounds and may identify their effect on a cellular pathway. Also, the systems and methods described herein may also be used for the determination of the dosage dependence of drug candidates.

Electromechanical plate readers can be used to detect signal ratio changes. Such plate readers can be employed for HTS, drug candidate screening, and drug dosage dependence studies using the system of the present disclosure or claimed. Examples of plate readers that can be used include the Fusion^{™} family of plate readers offered by PerkinElmer (Boston, MA), including the PerkinElmer Fusion^{™} Universal Microplate Analyzer devices. The PerkinElmer EnVision^{™} HTS model can also be employed in practicing the methods described herein.

### MODE(S) FOR CARRYING OUT THE INVENTION

The present invention is illustrated in further details by the following examples.

### Example 1: Materials and Methods

*Materials.* Angiotensin II (Angll; [Asp-Arg-Val-Tyr-Ile-His-Pro-Phe], SEQ ID NO: 58), Forkolin, Dopamine and poly-ornithine were from Sigma-Aldrich. u46619, I-BOP, CTA2, U51605, I-SAP, SQ 29558, were from Cayman Chemical^{®} (Ann Arbor, MI). Human EGF was from Cedarlane laboratories. Kallidin ([Lys0]-Bradykinin) was from Eurogentec. WAY-100635, A 412997, L 741742, SNC-80 and AR-M100390 were from Tocris Bioscience (Bio-Techne). Platelet activating factor (PAF-C16) was from Enzo Life Sciences, Inc. Salmon sperm DNA, Dulbecco's modified Eagles medium (DMEM), fetal bovine serum, calf serum, OPTI-MEM^{®}, and other cell culture reagents were purchased from Life technologies (Thermo Fisher Scientific) and from Wisent Inc. Polyethylenimine (PEI) 25 kDa linear was from Polysciences Inc. Prolume Purple I was purchased from Nanolight^{®} Technology. Phusion DNA polymerase was from Thermo Fisher Scientific. Restriction enzymes, T4 DNA ligase and Gibson assembly mix were obtained from New England Biolabs Ltd. White 96-well Polystyrene Cell Culture Microplates, solid bottom (CellStar 655 083) were from Greiner.

*Plasmids and constructions.* The plasma-membrane marker rGPF-CAAX and the early endomose marker rGFP-FYVE constructs were already described (Namkung Y. et al. 2016; Nat Commun. 7:12178). Plasmids encoding TPα receptor construct, all Gα subunits, Gβ1 subunit and Gγ1 subunits are from cdna.org. The coding sequence (cds) of the human receptors D4R, At1AR and Mu opiod receptor (hMOR1) with the signal peptide: MDSKGSSQKGSRLLLLLVVSNLLLCQGVVS (SEQ ID NO: 59), was sequence-optimized, synthetized at GeneART (Thermo Fisher Scientific) and subcloned by Gibson assembly in pCDNA3.1 (+) from Invitrogen (Thermo Fisher Scientific); hMOR1 was subcloned in pLVX IRES puro (Clontech, CA). The construct encoding the human EGFR was provided by. Dr Yosef Yarden (Tzahar E. et al. 1996; Molecular and Cellular Biology 16(10): 5276-5287). The Bradykinin B2 receptor (BKB2R) cds and RAPGAP(1-442)-Rluc8 construct (see **FIG. 9A** and **9B**) were sequence-optimized, synthetized and subcloned at Topgenetech in pCDNA3.1 (+); a peptidic linker GSGGGSGGGA (SEQ ID NO: 6) is present between the RapGAP(1-442) and Rluc8 (see **FIG. 2A**). The following construct encoding Rlucll tagged version of RapGAP (1-442), Rap1GAP ΔCT(1-420), Rap1GAP ΔSSS(1-436), Rap1GAP SSS-AAA(1-442;S437A/S439A/S441A), Rap1GAP SSS-TTT(1-442;S437T/S439T/S441T), Rap1GAP SS-AA(1-442;S437A/S441A), Rap1GAP SS-DA(1-442;S437A/S441A), Rap1GAP SS-AD(1-442;S437A/S441D), Rap1GAP SS-DD(1-442;S437A/S441A) were done by PCR amplification from pCDNA3.1 (+) RapGAP(1-442)-Rluc8 and subcloned by Gibson assembly in pCDNA3.1 Hygro(+) GFP10-Rlucll, replacing GFP10 with the coding sequences of the different variants of Rap1GAP (See **FIGs. 2A****,** **9A** **and** **9B**). A peptidic linker: GSAGTGGRAIDIKLPAT (SEQ ID NO: 7) is present between RAPGAP and Rlucll (See **FIG. 2A**). The construct encoding the RGS binding domain of the human RGS17 (residues: 64-210) tagged with Rlucll was done by PCR amplification from pCDNA 3.1 HA-RGS17 (from cDNA.org) and subcloned by Gibson assembly in pCDNA3.1 Hygro(+) GFP10-Rlucll, replacing GFP10 with the cds of RGS(RGS17 64-210) (See **FIGs. 3A** **and** **9D**). The construct encoding the RGS binding domain of the human RGS19 (residues: 70-217) tagged with Rlucll was done by PCR amplification from pCDNA 3.1 HA-RGS19 (from cDNA.org) and subcloned by Gibson assembly in pCDNA3.1 Hygro(+) GFP10-Rlucll, replacing GFP10 with the coding sequence of RGS19(70-217) (See **FIGs. 3A** **and** **9D**). The construct encoding the RGS binding domain of the human RGS20 var1 (residues: 242-388) tagged with Rlucll was done by PCR amplification from pCDNA 3.1 HA-RGS20 var1 (from cDNA.org) and subcloned by Gibson assembly in pCDNA3.1 Hygro(+) GFP10-Rlucll, replacing GFP10 with the coding sequence of RGS(RGS20 242-388) (See **FIGs 3A** **and** **9D**). A peptidic linker: GSAGTGGRAIDIKLASAT (SEQ ID NO: 20) is present between Rap1GAP and Rlucll (See **FIG 3A**). The constructs encoding the G12/13 binding domain of the human PDZRhoGEF (residues: 281-483) and P115RhoGEF (residues: 1-244) tagged with Rlucll were done by PCR amplification from IMAGE clones (OpenBiosystems) and subcloned by Gibson assembly in pCDNA3.1 Hygro(+) GFP10-Rlucll, replacing GFP10 (See **FIGs. 4A****,** **5A** **and** **9D**). Peptidic linkers: GILREALKLPAT (SEQ ID NO: 22) and RLKLPAT (SEQ ID NO: 24) are present between Rlucll and the G12/13 binding domain of PDZRhoGEF and P115RhoGEF, respectively (See **FIGs. 4A** **and** **5A****).** The construct encoding the Gq binding domain of the human P63RhoGEF (residues: 295-502) tagged with Rlucll was done from IMAGE clones (OpenBiosystems) and subcloned by Gibson assembly in pCDNA3.1 Hygro(+) GFP10-Rlucll, replacing GFP10 (See **FIGs. 6A** **and** **9B**). A peptidic linker: ASGSAGTGGRAIDIKLPAT (SEQ ID NO: 26) is present between the Gq binding domain and Rlucll (See **FIG. 6A**). The constructs encoding the RGS domain of the human GRK2 (residues: 30-203) tagged with Rlucll were done by PCR amplification from pcDNA3.1Z-GRK2-GFP10 and subcloned by Gibson assembly in pCDNA3.1 Hygro(+) GFP10-Rlucll and pCDNA3.1 (+) Rlucll-GFP10st2, replacing GFP10 and creating RGS(GRK2)-Rlucll and Rlucll-RGS(GRK2), respectively (See **FIGs. 7A** **and** **9B**). A peptidic linker: GSAGTGGRAIDIKLASAT (SEQ ID NO: 20) is present between the RGS(GRK2) domain and Rlucll, in both constructs (See **FIG. 7A**). Constructs encoding Gαi2 and GαoB mutants: Gαi2 Δ5(=1-350), Gαi2 Δ2(=1-353), Gαi2 L-7G(=L354G), Gαi2 L-2G(=L354G), Gαi2 L-2D(=L354D), Gαi2 L-2P(=L354P), Gαi2 L-2R(=L354R), GαoB L-2G(=L353G), GαoB Δ5(=1-349), were created by PCR-amplification from pCDNA3.1 (+) GNAi2 and pCDNA3.1 (+) GNAoB (from cdna.org) and subcloning by Gibson assembly in pCDNA3.1 Zeo(+).

*Cell culture and Transient Transfection.* Human embryonic kidney 293 (HEK293) cells were maintained in Dulbecco's Modified Eagle's Medium (DMEM) supplemented with 10% fetal bovine serum, 100 unit/ml penicillin/streptomycin at 37°C in a humidified atmosphere with 5% CO₂. HEK293SL cells were cultured in DMEM supplemented with 5% fetal bovine serum and 20 µg/ml gentamycin. Cells were grown at 37 °C in 5% CO₂ and 90% humidity.

*Transfection using Poly(ethylenimine) (PEI):* Two days before the experiments, HEK293 cells were washed with PBS containing no calcium or magnesium, detached and transfected with the indicated plasmids using PEI as a transfecting agent (at a ratio of 3 to 1, PEI/DNA). Cells were then directly seeded in 96-well plates pre-treated or not with poly-L-ornithine hydrobromide at a density of 35 000 cells per well. All experiments read in the Spark 10M reader were performed using non-treated plates.

*BRET measurements.* 48-hours post transfection, cells were washed twice with prewarmed Tyrode's buffer (140 mM NaCl, 2.7 mM KCI, 1 mM CaCl₂, 12 mM NaHCO₃, 5.6 mM D-glucose, 0.5 mM MgCl₂, 0.37 mM NaH₂PO₄, 25 mM HEPES, pH 7.4) before being stimulated with various concentrations of ligands at either room temperature (RT) or 37°C, as indicated. The cell-permeable substrate, coelenterazine Purple I was added at a final concentration of 1 µM in Tyrode's buffer for at least 6 min (at 37°C) to 10min (at RT) before BRET measurements. Measurements were either taken on a Spark 10M reader (Tecan Life Sciences; donor: 360nm/380nm & acceptor: 505nm/570nm), a LB 941 multimode plate reader (Berthold Technologies; donor: 400/470-nm and acceptor: 515/520-nm filters) or a Synergy Neo (Biotek: (donor: 400/480 nm, acceptor: 515/530 nm). The BRET signal was determined by calculating the ratio of the light intensity emitted by the rGFP (acceptor) over the light intensity emitted by the Rlucll (donor). All the BRET measurements were performed at 37 °C in the Tristar reader (**FIGs. 1-2M** & **FIGs. 2R-7F**), at RT in a Spark 10M reader (**FIGs. 2N-2Q** & **FIGs 8B-8S**) or at RT in a Synergy Neo (**FIGs. 8T-8X**).

*Z'-factors determination.* Z'-factor values were calculated as described by Zhang et al. (J Biomol Screen. 1999;4(2):67-73). A Z'-factor over 0.4 is considered a robust assay.

*Data Analysis.* Estimation of the EC₅₀ values were calculated using the GraphPad^{®} Prism curve fitting program. The curves presented throughout this study, representing the best fits, and were also generated using this GraphPad^{®} Prism program.

### Example 2: Generation and specificity of systems and assays for monitoring G protein activation in a G protein family-selective manner

FIGs. 1A to 1E show the generation and specificity of systems and assays for monitoring G protein activation in a G protein family-selective manner, and at different cellular compartments, based on the translocation of Gα subunit interacting polypeptides (GASIP) from G protein effectors. FIG. 1A depicts the principle of an effector-based sensor to monitor, in **i)** GPCR-mediated direct Gprotein activation and, in **ii)** guanine-nucleotide exchange factor (GEF)-mediated Gprotein activation. Cells expressing a receptor, a subcellular localization domain (for example for the plasma-membrane (PM) or for early endosomes (EE)) tagged with rGFP, the Gα-interaction domain of a specific effector tagged with a BRET donor (*e.g.,* Rlucll) are exposed to an agonist to activate the co-expressed G protein. In **i),** the agonist-induced GPCR stimulation activates directly G proteins, which recruits a tagged-effector from the cytoplasm to the *r*GFP-labeled membrane. In ii), G protein activation is mediated from the recruitment of a GEF such as GIV/Girdin following the activation of an RTK (*e.g.,* EGFR) or an integrin α-β complex. The G protein subunits do not need to be modified to monitor their activation, and specificity is achieved by coexpressing the Gα protein subunits with the studied receptor and by using a GASIP specific to each family of G proteins such as the G protein-binding domain of Rap1GAP for the Gi family (Gi1, Gi2, Gi3, GoA, GoB, Gz) (**FIG. 1B****)**, of P63RhoGEF (P63RG) for the Gq family (Gq, G11, G14 & G15) (**FIG. 1C**) and of PDZRhoGEF (PDZRG) for the G12/13 family (**FIGs. 1D** and **1E**). Only members of the Gi family showed a response greater than Mock condition (activation of endogenously expressed Gi1, i2 & i3 proteins) when the G protein-binding domain of Rap1GAP was used (**FIG. 1B**), only members of the G12/13 family showed a response greater than Mock condition (activation of endogenously expressed G12 and G13 proteins) when PDZRG was used (**FIG. 1C**), and only members of the Gq family show a response greater than Mock condition activation of endogenously expressed Gq and G11 proteins) when P63RG was used (**FIGs. 1D** and **1E**), thus confirming the specificity of the assay.

### Example 3: Systems and assays for monitoring activation of the G proteins of the Gi family

**FIGs. 2A** to **2Z** show the optimization and use of a Rap1GAP-based BRET sensor for monitoring activation of the G proteins of the Gi family (Gi1, i2, i3, oA, oB, z). The various constructs tested are shown in **FIG. 2A****.** The results presented in **FIGs. 2B** to **2E** shows that a detectable BRET signal was obtained using the two BRET donors tested (Rluc8 and Rlucll), with Rlucll typically giving a better dynamic window relative to Rluc8. The results presented in **FIGs. 2F** and **2G** demonstrate that the Rap1GAP(1-442) construct is sensitive to phosphorylation, as evidenced by the lower responses measured when cells were pre-treated with forskolin (which promotes an increase cAMP production and activation of protein kinase A leading to phosphorylation of different proteins), which could affect the assay. C-terminal truncated variants (1-420 and 1-436) and different mutants comprising combinations of Ser to Ala and Ser to Asp substitutions were made at putative phosphorylation sites (residues Ser 437, 439 and 441) were generated and tested. C-terminal truncated variants (ΔCT) of RAP1GAP were insensitive to the effects of forskolin, but the window of this shorter fragment (Rap1GAP 1-420) is significantly lower than that of Rap1GAP (1-442) (**FIG. 2H**). Among the other mutants tested, the only mutants still influenced by forskolin were Rap1GAP (SS-AD), Rap1GAP (SSS-TTT) and Rap1GAP (SS-AA), with the latter showing a dynamic window comparable to Rap1GAP (1-442)-Rlucll (**FIGs. 2J-2Q**). Rap1GAP (SSS-AAA) was used for the other experiments described below.

G protein profiling of the Dopamine D4 receptor (D4R) upon dopamine-promoted stimulation was obtained with Rap1GAP (SSS-AAA)-Rlucll translocation to the plasma membrane, each G protein (Gi1, Gi2, Gi3, GoA & GoB) showing dose-response curves with distinct pharmacological characteristics (**FIG. 2R**). Gz activation was used to profile several dopamine receptor ligands (A412 997, Dopamine, L741 742 and Way-100635) on the 5 dopamine receptors, and the results are depicted in **FIGs 2S** to **2W****.** Dopamine was shown to activate all the receptors (EC₅₀: D1R = 380 nM, D2R = 2.6 nM, D3R = 0.50 nM, D4R = 0.11 nM, D5R = 51 nM), the known D4R antagonist L741,742 did not show agonist nor inverse properties on any of the dopamine receptors. WAY-100635 only showed agonist properties on D4R (EC₅₀ = 0.83), whereas A412,997 activated both D3R (EC₅₀ = 281 nM) and D4R (EC₅₀ = 0.04 nM), but not D1R, D2R or D5R. The results depicted in **FIGs. 2X-2Z** show that the assay is robust and compatible with high-throughout screening (HTS), with Z' factor evaluated to 0.812, 0.703 and 0.607 for Gi2, GoA and Gz activation, respectively.

**FIGs. 3A-3D** show that an RGS domain of members of the Regulator of G-protein signaling (RGS) proteins such as RGS17, 19 and 20 may be used as an alternative to Rap1GAP to monitor G protein activation. Rlucll-tagged constructs based on the RGS domain of the RGS17 (residues 64-210), RGS19 (residues 70-217) and RGS20 (residues 242-388) proteins are presented in **FIG. 3A****.** Using only the Gα-binding RGS domain of these proteins advantageously allows for a cytosolic localization and translocation to different compartments upon G protein activation, with no influence of other domains present in the full-length protein or palmitoylation sites present at the N-terminal part of these proteins. Dose-response curves obtained with RGS(RGS17)-Rlucll (**FIG. 3B**), RGS(RGS19)-Rlucll (**FIG. 3C**) and RGS(RGS20)-Rlucll (**FIG. 3D**) are presented for D4R/Dopamine-mediated activation of Gi1, Gi2, Gi3, GoA, GoB and Gz activation at the PM. Similar results (coupling and EC₅₀) were obtained with the 3 RGS constructs, confirming that these RGS-based constructs may be used to monitor Gi and Go activation. The results were similar to those obtained with the Rap1GAP-based constructs, except that coexpression of Gz did not lead to a signal distinguishable from the mock condition, and the dynamic window for Gi3 was smaller with the RGS-based constructs relative to the Rap1GAP-based constructs.

### Example 4: Systems and assays for monitoring activation of the G proteins of the G12/13 family.

The optimization and use of a PDZRhoGEF-based BRET system for monitoring activation of G proteins of the G12/13 family is described in FIGs. 4A-G. A fragment of PDZRhoGEF comprising the G12/13 binding domain (residues 281-483, PDZRG) was tagged in C-terminal with the BRET donor Rlucll (**FIG. 4A**). The dynamic window for measuring activation of G12 and 13 was shown to be directly dependent on the level of expression of the Gα subunit, but the level of expression did not affect the potency for I-BOP/TPαR-mediated activation of G12 and 13 as evidenced by the comparable LogEC50 values obtained with the different amounts of G12 and 13 (**FIGs**. **4B** and **4C**). PDZRG-Rlucll was used to profile TPαR ligands on G12 and G13 activation at the PM. Cells were stimulated with known full agonists (U46619, I-BOP, CTA2), with one partial agonist (U51605) and the antagonists I-SAP and SQ 29,558. The results depicted in **FIGs. 4D** and **4E** show that SQ 29,558 is not acting as a TPαR agonist. Consistent with their known activity/properties, U46619, I-BOP and CTA2 are full agonists on G12 and 13 activation, confirming the validity of the assay to monitor G12/13 activation. Interestingly, U51605 and I-SAP were shown to act as biased ligands. U51605 was demonstrated to be a partial agonist on G12 (40% of Max) activation, and almost a full agonist on G13 (93% of Max). In contrast, I-SAP stimulation fails to induce significant G12 activation in the assay (**FIG. 4D**), but a partial agonist activity (53% of Max) was detected on G13 (**FIG. 4E**). Finally, Z' factors evaluated to 0.645 for G12 activation and 0.812 for G13 activation were obtained (**FIGs. 4F** and **4G**), again indicating that the assay is robust and compatible with high-throughput screening applications based on G12/G13 modulation.

The optimization and use of a P115RhoGEF-based BRET system for monitoring activation of G proteins of the G12/13 family is described in FIGs. 5A-E. A fragment of P115RhoGEF comprising the G12/13 binding domain (residues 1-244, P115RG) was tagged in C-terminal with the BRET donor Rlucll (**FIG. 5A**). P115RG-Rlucll was used to profile TPαR ligands on G12 and G13 activation at the PM. Cells were stimulated with U46619, I-BOP, CTA2, U51605, I-SAP and SQ 29,558. The results depicted in **FIGs. 5B** and **5C** are consistent with those obtained with PDZRG-Rlucll and show that SQ 29,558 is not acting as a TPαR agonist, U46619, I-BOP and CTA2 are full agonists on G12 and 13 activation, U51605 and I-SAP were shown to act as biased ligands. U51605 was demonstrated to be a partial agonist on G12 (14% of Max) and G13 (60% of Max) activation, whereas I-SAP stimulation was a partial agonist on G13 (19% of Max) and neutral on G12 activation. Finally, Z' factors evaluated to 0.703 for G12 activation and 0.743 for G13 activation were obtained (**FIGs. 5D** and **5E**), again indicating that the assay is robust and compatible with high-throughput screening applications based on G12/G13 modulation.

### Example 5: Systems and assays for monitoring activation of the G proteins of the Gq family.

The optimization and use of a P63RhoGEF-based BRET system for monitoring activation of G proteins of the Gq family (Gq, G11, G14 and G15) is described in FIGs. 6A-S. A fragment of P63RhoGEF comprising the Gq binding domain (residues 295-502, P63RG) was tagged in C-terminal with the BRET donor Rlucll (**FIG. 6A**). The full length P63RhoGEF protein is associated with the PM by its N-terminus. As with PDZRG-Rlucll and P115RG-Rlucll, using a fragment comprising only the G protein binding domain of P63RhoGEF advantageously allows for a cytosolic localization and translocation to different compartments upon G protein activation. Optimization for monitoring G protein activity at the PM and at early endosomes (EE) using different membrane markers (rGFP-CAAX for PM and rGFP-FYVE for EE) are presented in **FIGs. 6B-6E** (for PM) **and** FIGs. 6F-I (for EE). Mock represents responses obtained from endogenous G proteins, in cells not transfected with a recombinant Gα. The dynamic window for measuring activation of members of the Gq family was shown to be dependent on the level of expression of the Gα subunit. As presented in **FIG. 6B** for Gq and **FIG. 6D** for G14, the dynamic window increases as more Gα is cotransfected. For G15 (**FIG. 6E**), the effect is already maximum at 5ng of co-transfected constructs encoding Gα15, and transfecting more had no significant effect. For G11 (in **FIG. 6C**), increasing the level of transfected DNA over 5 ng was shown to lead to a decrease of the dynamic window. Similar results were obtained for the monitoring of G protein activity at EE (FIGs. 6F-I).

Optimized conditions were used to profile the TPαR ligands U46619, I-BOP, CTA2, U51605, I-SAP and SQ 29,558 at the PM (**FIGs. 6J-6M**) and EE (**FIGs. 6N-6Q**). As shown in **FIGs. 6J-6M****,** U46619, I-BOP and CTA2 are full agonists on Gq (**FIG. 6J**), G14 (**FIG. 6K**), G11 (**FIG**. **6L**) and G15 (**FIG. 6M**); U51605 is a partial agonist (32% of I-BOP max response for Gq, 25% for G14, 24% for G11 and 10% for G15); I-SAP (only 7% on Gq) and SQ 29,558 fail to induce significant activation of all four G proteins at the PM. Similar results (potency and efficacy) were obtained when monitoring G protein activity at EE (**FIGs. 6N-6Q**). For U51605, a higher efficacy was observed at EE for Gq (71% of I-BOP max response), G11 (61%) and G14 (75%), but no response for G15. Finally, Z' factors evaluated to 0.840 and 0.879 for Gq and G11 activation at the PM respectively, were obtained (**FIGs. 6R** and **6S**), again indicating that the assay is robust and compatible with high-throughput screening applications based on Gq modulation.

The optimization and use of a GRK2-based BRET system for monitoring activation of G proteins of the Gq family (Gq, G11, G14 and G15) is described in **FIGs. 7A-7F****.** Two RGS(GRK2) construct are presented. A fragment of GRK2 comprising the Gq binding domain (RGS domain) (residues 30-203, RGS(GRK2)) was tagged at the N-terminal (Rlucll-RGS(GRK2)) or C-terminal (RGS(GRK2)-Rlucll) with the BRET donor, Rlucll. The full length GRK2 protein has a Pleckstrin (PH) domain and a Gβγ-interacting domain that modulate its recruitment to the PM. Using only the Gq-binding RGS domain of GRK2 advantageously allows for a cytosolic localization and translocation to different compartments upon Gq protein activation, with no influence of PIP₂ or free Gβγ subunits levels that could be modulated through activation of other non-Gq G proteins, for example. In **FIGs 7B** and **7C****,** dose-response curves obtained with Rlucll-RGS(GRK2) are presented for Gq, G11, G14 and G15 activation at the PM, by two Gq-coupled receptors, AT1AR stimulated with angiotensinll (**FIG. 7B**) and TPαR stimulated with U46619 (**FIG. 7C**). As depicted in **FIGs. 7D** and **7E****,** similar results (coupling and EC₅₀) were obtained with RGS(GRK2)-Rlucll, albeit with dynamic windows that were generally lower relative to Rlucll-RGS(GRK2). Z' factor evaluated to 0.785 was obtained when assessing recruitment of Rlucll-RGS(GRK2) to the PM following activation of TPαR with 100 nM U46619, confirming that the assay is robust and compatible with high-throughput screening applications based on Gq modulation.

### Example 6: Systems and assays for monitoring activation of G proteins by non-receptor guanine nucleotide exchange factors (GEF).

G protein activation can be achieved via non-receptor guanine nucleotide exchange factors (GEF) such as GIV (Gα-interacting vesicle-associated protein, also known as Girdin), NUCB1 (nucleobindin1, also known as calnuc), NUCB2 and DAPLE (Dishevelled-associating protein). GIV/Girdin activity is associated with RTKs (*e.g*., EGFR) and integrin modulation of Gi activity. DAPLE is associated with rac and Gi-activation through wnt/Frizzle receptors (GPCRs). GEF-mediated activation of WT Gi proteins can be monitored using the systems/assays for monitoring activation of the G proteins of the Gi family described herein, such as the systems/assays using Rap1Gap (SSS-AAA)-Rlucll. However, as GPCRs have been shown to transactivate RTKs such as EGFR and IGFR, it could be useful to be able to discriminate between GPCR and GEF-mediated activation of G proteins, for HTS applications and ligand profiling studies. To achieve this objective, a group of mutant Gαi2 proteins having C-terminal mutations (FIG. 8A) were designed and tested. EGFR-mediated activation of WT and mutant Gi2 was compared to GPCR (BKB2R) response in dose response curves. Deleting the last 2 residues of Gαi2 (**FIGs. 8B, 8C**) or the last 5 residues of Gαi2 (**FIGs. 8D, 8E**) and GαoB (**FIGs. 8F, 8G**) was sufficient to prevent GPCR-mediated G protein activation while maintaining EGFR-mediated activation of Gi2 and GoB. For Gi2, both deletions changed the basal activity as compared to the WT G proteins for cells co-expressing EGFR. Substitutions were made at 2 conserved leucine residues (positions -7 and -2) to identify a residue that would lead to a similar basal activity with EGFR but with a mutant still inactive upon GPCR activation. The L-7G mutant showed a different basal activity then the WT with EGFR (**FIGs. 8H**, **8I**). Results with L-2G mutant of Gαi2 showed a similar basal activity than WT with EGFR but a different one with BKB2R (FIGs. 87J, **8K**). For the GαoB L-2G mutant, the basal activity was similar to the WT protein for both receptors (**FIGs**. **8L, 8M**). Additional L-2 mutant Gαi2 proteins were tested (L-2D, L-2P and L-2R). While their dynamic window, as measured with agonist-induced translocation of Rap1GAP(SSS-AAA)-Rlucll to the PM, is smaller than with the WT proteins, differences in basal activity were much closer relative to the deletion or L-2G mutants of Gαi2 protein (**FIGs. 8N-8S**). Z' factor evaluated to 0.70 with WT Gαi2 (**FIG. 8T**) and to 0.73 with the L-2P Gαi2 mutant (**FIG. 8U**) were obtained, which indicates a robust assay for monitoring non-GPCR-mediated G protein activation using both mutant and WT G proteins. **FIGs 8V-8X** show that Rap1GAP(SSS-AAA)-Rlucll can be used to monitor GPCR (**FIGs 8V** with SNC80-mediated DOR activation and in **FIG. 8W**, Quinpirole-mediated activation of D2R) as well as GEF-mediated Gprotein activation (through EGFR stimulation; **FIG. 8X**) while RGS(RGS17)-Rlucll-based sensor only monitors GPCR-mediated activation as there is no significant increase in the BRET signal in the presence of escalating concentrations of the agonist EGF. As GPCR activation can lead to RTK transactivation, these results provide evidence that WT Gα/RGS(RGS17)-RlucII and mutant Gα/Rap1GAP(SSS-AAA)-Rlucll could be used to distinguish GPCR-mediated from RTK/GEF-mediated Gprotein activation.

In the claims, the word "comprising" is used as an open-ended term, substantially equivalent to the phrase "including, but not limited to". The singular forms "a", "an" and "the" include corresponding plural references unless the context clearly dictates otherwise.

## Claims

1. A system for measuring modulation of G protein activation in a Gα protein subunit family-selective manner, said system comprising a cell expressing:
(i) a first component comprising a Gα subunit interacting polypeptide (GASIP) tagged with a bioluminescent donor molecule or a fluorescent acceptor molecule;
wherein said GASIP comprises the G protein-binding domain of:
Rap1GAP, a Regulator of G-protein signaling (RGS) protein, P63RhoGEF, PDZRhoGEF, or P115RhoGEF;
(ii) a second component comprising a plasma membrane (PM)-targeting moiety comprising (a) a palmitoylation, myristoylation, and/or prenylation signal sequence and/or (b) a polybasic sequence, an endosomal-targeting moiety or a Golgi-targeting moiety tagged with a bioluminescent donor molecule or a fluorescent acceptor molecule;
wherein if said GASIP is tagged with said fluorescent acceptor molecule, said PM-targeting moiety, endosomal-targeting moiety or Golgi-targeting moiety is tagged with said bioluminescent donor molecule, and if said GASIP is tagged with said bioluminescent donor molecule, said PM-targeting moiety, endosomal-targeting moiety or Golgi-targeting moiety is tagged with said fluorescent acceptor molecule;
(iii) a third component that is a cell surface receptor that signals through said G protein, wherein said receptor is a GPCR, an RTK or an integrin receptor;
and wherein there is no direct protein-protein interaction between (i) the PM-targeting moiety, endosomal-targeting moiety or Golgi-targeting moiety and (ii) the GASIP.

2. The system of claim 1, wherein said GASIP comprises residues 1 to 420 or 1 to 436 of Rap1GAP.

3. The system of claim 1, wherein said GASIP comprises the G protein-binding domain of RGS17, RGS19 or RGS20.

4. The system of claim 3, wherein said G protein-binding domain comprises residues 64 to 210 of RGS17 (SEQ ID NO:17), residues 70-217 of RGS19 (SEQ ID NO:18), or residues 242-388 of RGS20 (SEQ ID NO:19).

5. The system of claim 1, wherein said G protein-binding domain of P63RhoGEF comprises residues 295 to 502 of P63RhoGEF (SEQ ID NO:25).

6. The system of claim 1, wherein said G protein-binding domain of PDZRhoGEF comprises residues 281 to 483 of PDZRhoGEF (SEQ ID NO:21), and said G protein-binding domain of P115RhoGEF comprises residues 1 to 244 of P115RhoGEF (SEQ ID NO:23).

7. The system of any one of claims 1 to 6, wherein said GASIP is tagged with said bioluminescent donor molecule and said PM-targeting moiety, endosomal-targeting moiety or Golgi-targeting moiety is tagged with said fluorescent acceptor molecule.

8. The system of any one of claims 1 to 7, wherein said PM targeting moiety comprises the amino acid sequence GCMSCKCVLS (SEQ ID NO:60), GCMGLPCVVM (SEQ ID NO:61), CVKIKKCIIM (SEQ ID NO:62), KKKKKKSKTKCVIM (SEQ ID NO:63), or KNGKKKRKSLAKRIRERCCIL (SEQ ID NO: 45), CMSCKCCIL (SEQ ID NO:4), or SPKKGLLQRLFKRQHQNNSKS (SEQ ID NO:5).

9. The system of any one of claims 1 to 7, wherein said endosomal targeting moiety is an endosomal protein or a fragment thereof that localizes to the endosomes.

10. The system of claim 9, wherein said endosomal protein or fragment thereof comprises a FYVE domain.

11. The system of any one of claims 1 to 7, wherein said Golgi targeting moiety is a Golgi protein or a fragment thereof that localizes to the Golgi.

12. The system of claim 11, wherein said Golgi targeting moiety comprises residues 1 to 73 of human eNOS1 (SEQ ID NO:46).

13. The system of any one of claims 1 to 12, wherein (a) said first component further comprises a linker between (i) said GASIP and (ii) said bioluminescent donor molecule or fluorescent acceptor molecule; and/or said second component further comprises a linker between (i) said PM-targeting moiety, endosomal-targeting moiety or Golgi-targeting moiety and (ii) said bioluminescent donor molecule or fluorescent acceptor molecule.

14. The system of any one of claims 1 to 13, wherein said cell surface receptor is a GPCR.

15. The system of any one of claims 1 to 14, further comprising a fourth component that is a recombinant Gα subunit polypeptide.

16. The system of claim 15, wherein said G protein activation is non-receptor guanine nucleotide exchange factor (GEF)-mediated G protein activation, wherein said recombinant Gα subunit polypeptide comprises at least one mutation in the carboxy (C)-terminal domain of said Gα subunit polypeptide, and wherein said C-terminal domain corresponds to the last seven residues of said Gα subunit polypeptide.

17. The system of claim 16, wherein said GEF is GIV/Girdin, and wherein said GASIP comprises the G protein-binding domain of Rap1GAP as defined in claim 1 or 2.

18. The system of any one of claims 1 to 17, wherein said bioluminescent donor molecule is *Renilla* luciferase protein (rLuc) and said fluorescent acceptor molecule is a *Renilla* green fluorescent protein (*r*GFP).

19. A host cell expressing the components of the system defined any one of claims 1 to 18.

20. A method for determining whether an agent modulates the activation of a G protein of interest, said method comprising:
(a) contacting the system of any one of claims 1 to 18 with a substrate for said bioluminescent donor molecule; and
(b) measuring the BRET signal in the system in the presence and absence of said agent;
wherein a difference in said BRET signal in the presence of said agent relative to the absence thereof is indicative that said agent modulates the activation of said G protein of interest.

21. A method for determining whether an agent modulates non-receptor guanine nucleotide exchange factor (GEF)-mediated G protein activation, said method comprising
(a) contacting the system of claim 16 or 17 with a substrate for said bioluminescent donor molecule; and
(b) measuring the BRET signal in the system in the presence and absence of said agent;
wherein a difference in said BRET signal in the presence of said agent relative to the absence thereof is indicative that said agent modulates non-receptor GEF-mediated G protein activation.

## Patentansprüche

1. System zum Messen der Modulation der G-Protein-Aktivierung in einer Gα-Protein-Untereinheiten auf familienselektive Weise, wobei das System eine Zelle umfasst, die exprimiert:
(i) eine erste Komponente, die ein mit der Gα-Untereinheit interagierendes Polypeptid (GASIP) umfasst, das mit einem biolumineszenten Spendermolekül oder einem fluoreszierenden Empfängermolekül markiert ist;
wobei das GASIP die G-Protein-bindende Domäne von umfasst:
Rap1GAP, ein RGS-Protein (Regulator of G-protein signaling), P63RhoGEF, PDZRhoGEF, oder P115RhoGEF;
(ii) eine zweite Komponente, die eine auf die Plasmamembran (PM) abzielende Einheit umfasst, die (a) eine Palmitoylierungs-, Myristoylierungs- und/oder Prenylierungssignalsequenz und/oder (b) eine mehrbasige Sequenz, eine Endosom-Zieleinheit oder eine Golgi-Zieleinheit umfasst, die mit einem biolumineszenten Spendermolekül oder einem fluoreszierenden Empfängermolekül markiert ist;
wobei, wenn das GASIP mit dem fluoreszierenden Empfängermolekül markiert ist, die PM-Zieleinheit, Endosom-Zieleinheit oder Golgi-Zieleinheit mit dem biolumineszierenden Spendermolekül markiert ist, und wenn das GASIP mit dem biolumineszierenden Spendermolekül markiert ist, die PM-Zieleinheit, Endosom-Zieleinheit oder Golgi-Zieleinheit mit dem fluoreszierenden Empfängermolekül markiert ist;
(iii) eine dritte Komponente, bei der es sich um einen Zelloberflächenrezeptor handelt, der über das G-Protein Signale aussendet, wobei der Rezeptor ein GPCR, ein RTK oder ein Integrinrezeptor ist;
und wobei es keine direkte Protein-Protein-Wechselwirkung zwischen (i) der PM-Zieleinheit, der Endosom-Zieleinheit oder der Golgi-Zieleinheit und (ii) dem GASIP gibt.

2. System nach Anspruch 1, wobei das GASIP die Reste 1 bis 420 oder 1 bis 436 von Rap1GAP umfasst.

3. System nach Anspruch 1, wobei das GASIP die G-Protein-bindende Domäne von RGS17, RGS19 oder RGS20 umfasst.

4. System nach Anspruch 3, wobei die G-Protein-bindende Domäne die Reste 64 bis 210 von RGS17 (SEQ ID NO:17), die Reste 70-217 von RGS19 (SEQ ID NO:18) oder die Reste 242-388 von RGS20 (SEQ ID NO:19) umfasst.

5. System nach Anspruch 1, wobei die G-Protein-bindende Domäne von P63RhoGEF die Reste 295 bis 502 von P63RhoGEF (SEQ ID NO:25) umfasst.

6. System nach Anspruch 1, wobei die G-Protein-bindende Domäne von PDZRhoGEF die Reste 281 bis 483 von PDZRhoGEF (SEQ ID NO:21) umfasst, und die G-Protein-bindende Domäne von P115RhoGEF die Reste 1 bis 244 von P115RhoGEF (SEQ ID NO:23) umfasst.

7. System nach irgendeinem der Ansprüche 1 bis 6, wobei das GASIP mit dem biolumineszenten Spendermolekül markiert ist und die PM-Zieleinheit, die Endosom-Zieleinheit oder der Golgi-Zieleinheit mit dem fluoreszierenden Emfpängermolekül markiert ist.

8. System nach irgendeinem der Ansprüche 1 bis 7, wobei die PM-Zieleinheit die Aminosäuresequenz GCMSCKCVLS (SEQ ID NO:60), GCMGLPCVVM (SEQ ID N0:61), CVKIKKCIIM (SEQ ID NO:62), KKKKKKSKTKCVIM (SEQ ID NO:63) oder KNGKKKRKSLAKRIRERCCIL (SEQ ID NO:63) oder KNGKKKRKSLAKRIRERCCIL (SEQ ID NO: 45), CMSCKCCIL (SEQ ID NO:4) oder SPKKGLLQRLFKRQHQNNSKS (SEQ ID NO:5) umfasst.

9. System nach irgendeinem der Ansprüche 1 bis 7, wobei die Endosom-Zieleinheit ein endosomales Protein oder ein Fragment davon ist, das in den Endosomen angeordnet ist.

10. System nach Anspruch 9, wobei das endosomale Protein oder Fragment davon eine FYVE-Domäne umfasst.

11. System nach irgendeinem der Ansprüche 1 bis 7, wobei die Golgi-Zieleinheit ein Golgi-Protein oder ein Fragment davon ist, das im Golgi angeordnet ist.

12. System nach Anspruch 11, wobei die Golgi-Zieleinheit die Reste 1 bis 73 von menschlichem eNOS1 (SEQ ID NO:46) umfasst.

13. System nach einem der Ansprüche 1 bis 12, wobei (a) die erste Komponente weiterhin einen Binder zwischen (i) dem GASIP und (ii) dem biolumineszenten Spendermolekül oder fluoreszenten Emfpängermolekül umfasst; und/oder die zweite Komponente weiterhin einen Binder zwischen (i) der PM-Zieleinheit, der Endosom-Zieleinheit oder der Golgi-Zieleinheit und (ii) dem biolumineszenten Spendermolekül oder dem fluoreszenten Empfängermolekül umfasst.

14. System nach irgendeinem der Ansprüche 1 bis 13, wobei der Zelloberflächenrezeptor ein GPCR ist.

15. System nach irgendeinem der Ansprüche 1 bis 14 weiterhin umfassend eine vierte Komponente, bei der es sich um ein rekombinantes Gα-Untereinheiten-Polypeptid handelt.

16. System nach Anspruch 15, wobei die G-Protein-Aktivierung eine nicht-Rezeptor-Guanin-Nukleotid-Austauschfaktor (GEF)-vermittelte G-Protein-Aktivierung ist, wobei das rekombinante Gα-Untereinheiten-Polypeptid wenigstens eine Mutation in der Carboxy (C)-terminalen Domäne des Gα-Untereinheiten-Polypeptids umfasst und wobei die C-terminale Domäne den letzten sieben Resten des Gα-Untereinheiten-Polypeptids entspricht.

17. System nach Anspruch 16, wobei der GEF GIV/Girdin ist, und wobei der GASIP die G-Protein-bindende Domäne von Rap1GAP, wie in Anspruch 1 oder 2 definiert, umfasst.

18. System nach einem der Ansprüche 1 bis 17, wobei das biolumineszente Spendermolekül ein *Renilla-Luciferase-Protein* (rLuc) ist und das fluoreszierende Empfängermolekül ein grün fluoreszierendes *Renilla-Protein* (rGFP) ist.

19. Wirtszelle, die die Komponenten des Systems nach irgendeinem der Ansprüche 1 bis 18 exprimiert.

20. Verfahren zum Bestimmen, ob ein Mittel die Aktivierung eines G-Proteins von Interesse moduliert, wobei das Verfahren umfasst:
(a) inkontaktbringen des Systems nach irgendeinem der Ansprüche 1 bis 18 mit einem Substrat für das biolumineszente Spendermolekül; und
(b) messen des BRET-Signals im System in An- und Abwesenheit des Mittels;
wobei ein Unterschied in dem BRET-Signal in Anwesenheit des Mittels im Vergleich zu dessen Abwesenheit anzeigt, dass das Mittel die Aktivierung des G-Proteins von Interesse moduliert.

21. Verfahren zum Bestimmen, ob ein Mittel die durch eine Nicht-Rezeptor-Guanin-Nukleotid-Austauschfaktor (GEF) vermittelte G-Protein-Aktivierung moduliert, wobei das Verfahren umfasst
(a) inkontaktbringen des Systems nach Anspruch 16 oder 17 mit einem Substrat für das biolumineszente Spendermolekül; und
(b) messen des BRET-Signals im System in An- und Abwesenheit des Mittels;
wobei ein Unterschied in dem BRET-Signal in Anwesenheit des Mittels im Vergleich zur Abwesenheit desselben anzeigt, dass das Mittel die nicht Rezeptor-GEFvermittelte G-Protein-Aktivierung moduliert.

## Revendications

1. Système permettant de mesurer la modulation de l'activation de la protéine G d'une manière qui soit sélective par rapport à la famille de la sous-unité alpha de la protéine G (Gα), ledit système comprenant une cellule exprimant :
(i) un premier composant comprenant un polypeptide interagissant avec la sous-unité Gα (GASIP) marqué par une molécule donneuse bioluminescente ou une molécule acceptrice fluorescente ;
dans lequel ledit GASIP comprend le domaine de liaison à la protéine G de :
Rap1GAP, une protéine régulatrice de la signalisation des protéines G (RGS), P63RhoGEF, PDZRhoGEF ou P115RhoGEF ;
(ii) un deuxième composant comprenant un groupement ciblant la membrane plasmique (PM) comprenant (a) une séquence de signal de palmitoylation, de myristoylation et/ou de prénylation et/ou (b) une séquence polybasique, un groupement ciblant l'endosome ou un groupement ciblant l'appareil de Golgi, qui est marqué par une molécule donneuse bioluminescente ou une molécule acceptrice fluorescente ;
dans lequel, si ledit GASIP est marqué par ladite molécule acceptrice fluorescente, ledit groupement ciblant la PM, l'endosome ou l'appareil de Golgi est marqué par ladite molécule donneuse bioluminescente, et si ledit GASIP est marqué par ladite molécule donneuse bioluminescente, ladite fraction ciblant la PM, l'endosome ou l'appareil de Golgi est marquée par ladite molécule acceptrice fluorescente ;
(iii) un troisième composant qui est un récepteur de surface cellulaire qui émet un signal par l'intermédiaire de ladite protéine G, dans lequel ledit récepteur est un GPCR, un RTK ou un récepteur d'intégrine ;
et dans lequel il n'y a pas d'interaction protéine-protéine directe entre (i) le groupement ciblant la PM, le groupement ciblant l'endosome ou le groupement ciblant l'appareil de Golgi et (ii) le GASIP.

2. Système selon la revendication 1, dans lequel ledit GASIP comprend les résidus 1 à 420 ou 1 à 436 de Rap1GAP.

3. Système selon la revendication 1, dans lequel ledit GASIP comprend le domaine de liaison à la protéine G de RGS17, RGS19 ou RGS20.

4. Système selon la revendication 3, dans lequel ledit domaine de liaison à la protéine G comprend les résidus 64 à 210 de RGS17 (SEQ ID NO:17), les résidus 70 à 217 de RGS19 (SEQ ID NO:18), ou les résidus 242 à 388 de RGS20 (SEQ ID NO:19).

5. Système selon la revendication 1, dans lequel ledit domaine de liaison à la protéine G de P63RhoGEF comprend les résidus 295 à 502 de P63RhoGEF (SEQ ID NO:25).

6. Système selon la revendication 1, dans lequel ledit domaine de liaison à la protéine G de PDZRhoGEF comprend les résidus 281 à 483 de PDZRhoGEF (SEQ ID NO:21), et ledit domaine de liaison à la protéine G de P115RhoGEF comprend les résidus 1 à 244 de P115RhoGEF (SEQ ID NO:23).

7. Système selon l'une quelconque des revendications 1 à 6, dans lequel ledit GASIP est marqué par ladite molécule donneuse bioluminescente et ledit groupement ciblant la PM, l'endosome ou l'appareil de Golgi est marque par la molécule acceptrice fluorescente.

8. Système selon l'une quelconque des revendications 1 à 7, dans lequel ladite fraction ciblant la PM comprend la séquence d'acides aminés GCMSCKCVLS (SEQ ID NO:60), GCMGLPCVVM (SEQ ID NO:61), CVKIKKCIIM (SEQ ID NO:62), KKKKKKSKTKCVIM (SEQ ID NO:63), ou KNGKKKRKSLAKRIRERCCIL (SEQ ID NO: 45), CMSCKCCIL (SEQ ID NO:4) ou SPKKGLLQRLFKRQHQNNSKS (SEQ ID NO:5).

9. Système selon l'une quelconque des revendications 1 à 7, dans lequel le groupement ciblant l'endosome est une protéine endosomale ou un fragment de celle-ci qui se localise dans les endosomes.

10. Système selon la revendication 9, dans lequel ladite protéine endosomale ou un fragment de celle-ci comprend un domaine FYVE.

11. Système selon l'une quelconque des revendications 1 à 7, dans lequel le groupement ciblant l'appareil de Golgi est une protéine de Golgi ou un fragment de celle-ci qui se localise dans l'appareil de Golgi.

12. Système selon la revendication 11, dans lequel ledit groupement ciblant l'appareil de Golgi comprend les résidus 1 à 73 de l'eNOS1 humain (SEQ ID NO:46).

13. Système selon l'une quelconque des revendications 1 à 12, dans lequel (a) ledit premier composant comprend, en outre, un segment de liaison entre (i) ledit GASIP et (ii) ladite molécule donneuse bioluminescente ou molécule acceptrice fluorescente ; et / ou ledit deuxième composant comprend, en outre, un segment de liaison entre (i) ledit groupement ciblant la PM, ledit groupement ciblant l'endosome ou ledit groupement ciblant l'appareil de Golgi et (ii) ladite molécule donneuse bioluminescente ou molécule acceptrice fluorescente.

14. Procédé selon l'une quelconque des revendications 1 à 13, dans lequel ledit récepteur de surface cellulaire est un GPCR.

15. Système selon l'une quelconque des revendications 1 à 14, comprenant, en outre, un quatrième composant qui est un polypeptide recombinant de la sous-unité Gα.

16. Système selon la revendication 15, dans lequel ladite activation de la protéine G est une activation de la protéine G médiée par un facteur d'échange nucléotide guanine (GEF) non récepteur, dans lequel ledit polypeptide recombinant de la sous-unité Gα comprend au moins une mutation dans le domaine carboxy (C)-terminal dudit polypeptide de la sous-unité Gα, et dans lequel ledit domaine C-terminal correspond aux sept derniers résidus dudit polypeptide de la sous-unité Gα.

17. Système selon la revendication 16, dans lequel ledit GEF est GIV/Girdin, et dans lequel ledit GASIP comprend le domaine de liaison à la protéine G de Rap1GAP tel que défini dans la revendication 1 ou 2.

18. Système selon l'une quelconque des revendications 1 à 17, dans lequel ladite molécule donneuse bioluminescente est la protéine *Renilla* luciferase (rLuc) et ladite molécule acceptrice fluorescente est une protéine fluorescente verte *Renilla* (rGFP).

19. Cellule hôte exprimant les composants du système défini selon l'une quelconque des revendications 1 à 18.

20. Procédé permettant de déterminer si un agent module ou non l'activation d'une protéine G d'intérêt, ledit procédé comprenant :
(a) la mise en contact du système selon l'une quelconque des revendications 1 à 18 avec un substrat pour ladite molécule donneuse bioluminescente ; et
(b) la mesure du signal BRET dans le système en présence et en absence dudit agent ;
dans lequel une différence dans ledit signal BRET en présence dudit agent par rapport à son absence indique que ledit agent module l'activation de ladite protéine G d'intérêt.

21. Procédé permettant de déterminer si un agent module ou non l'activation de la protéine G médiée par le facteur d'échange nucléotide guanine (GEF) non récepteur, ledit procédé consistant à :
(a) mettre en contact le système selon la revendication 16 ou 17 avec un substrat pour ladite molécule donneuse bioluminescente ; et
(b) la mesure du signal BRET dans le système en présence et en absence dudit agent ;
dans lequel une différence dans ledit signal BRET en présence dudit agent par rapport à son absence indique que ledit agent module l'activation de la protéine G médiée par le GEF non récepteur.
